# EUROPEAN PATENT SPECIFICATION

(11) **EP 2 438 176 B1**
(45) Date of publication and mention of the grant of the patent: **17.12.2014**
(21) Application number: 10723281.1
(22) Date of filing: 01.06.2010
(51) Int. Cl.: C12N 15/82, A01H 5/00

(54) **Methods and compositions for stress tolerance in plants**
Verfahren und Zusammensetzungen für Stresstoleranz bei Pflanzen
Procédés et compositions pour la tolérance au stress chez les végétaux

(30) Priority: 01.06.2009 GB 0909318
(43) Date of publication of application: 11.04.2012
(73) Proprietor: Universidad Nacional Del Litoral, Santa Fe (AR); Consejo Nacional De Investigaciones Cientificas Y Tecnicas, Cdad. de Buenos Aires (AR)
(72) Inventor: CABELLO, Julieta Virginia, Santa Fe 3000 (AR); ARCE, Agustin Lucas, Santa Fe 3000 (AR); CHAN, Raquel Lia, Santa Fe 3000 (AR)
(74) Representative: Fleck, Barbara
(86) International application number: PCT/GB2010/050920
(87) International publication number: WO 2010/139993

(56) References cited:
- WO-A1-2008/021397
- WO-A2-03/006651
- WO-A2-2004/099365
- WO-A2-2009/073069
- US-A1- 2009 070 899
- CHAN R: "Phenotypic and molecular characteristics of transgenic plants over-expressing transcription factors from the HD-Zip family." WORKSHOP PROCEEDINGS. TALLER DE FENOTIPOS COMPLEJOS, 2007, XP002597801 Retrieved from the Internet: URL:http://www.conicet.gov.ar/scp/vista_re sumen.php?produccion=256296&id=10687&keywo rds=> [retrieved on 2010-08-24]
- RUEDA E C & CHAN R L: "HAHB-1, A SUNFLOWER HD-ZIP TRANSCRIPTIONFACTOR, NEGATIVELY REGULATES DEVELOPMENTALRATE IN RESPONSE TO OSMOTIC STRESS" BIOCELL, vol. 29 (Suppl.), PL-P37, December 2005 (2005-12), XP002597802 Mendoza, Argentina ISSN: 0327-9545
- RUEDA E ET AL.: "Los genes Hahb10 y Hahb1 de girasol, pertenecientes a la familia Hd-Zip, están invo-lucrados en la respuesta adaptativa de las plantas a las condiciones ambientales." REVISTA DEL CONGRESO. REUNION DE LA SAFV, [Online] 2004, XP002597803 Santa Rosa - La Pampa, Argentina Retrieved from the Internet: URL:http://www.conicet.gov.ar/scp/vista_re sumen.php?produccion=38025&id=10687&keywor ds=> [retrieved on 2010-08-24]
- CARLOS ALBERTO DEZAR ET AL: "Hahb-4, a sunflower homeobox-leucine zipper gene, is a developmental regulator and confers drought tolerance to Arabidopsis thaliana plants" TRANSGENIC RESEARCH, KLUWER ACADEMIC PUBLISHERS-PLENUM PUBLISHERS, NE, vol. 14, no. 4, 1 August 2005 (2005-08-01), pages 429-440, XP019269455 ISSN: 1573-9368
- GROVER ANITA ET AL: "Strategies for development of fungus-resistant transgenic plants." CURRENT SCIENCE (BANGALORE), vol. 84, no. 3, 10 February 2003 (2003-02-10), pages 330-340, XP002597804 ISSN: 0011-3891
- VIERHEILIG H ET AL: "Colonization of transgenic tobacco constitutively expressing pathogenesis- related proteins by the vesicular-arbuscular mycorrhizal fungus Glomus mosseae" APPLIED AND ENVIRONMENTAL MICROBIOLOGY 1995 US, vol. 61, no. 8, 1995, pages 3031-3034, XP002597805 ISSN: 0099-2240
- HANSON JOHANNES ET AL: "Sugar-dependent alterations in cotyledon and leaf development in transgenic plants expressing the HDZhdip gene ATHB13" PLANT MOLECULAR BIOLOGY, vol. 45, no. 3, February 2001 (2001-02), pages 247-262, XP002597972 ISSN: 0167-4412
- CHAN R L ET AL: "A cDNA encoding an HD-zip protein from sunflower." PLANT PHYSIOLOGY DEC 1994 LNKD- PUBMED:7846169, vol. 106, no. 4, December 1994 (1994-12), pages 1687-1688, XP002597806 ISSN: 0032-0889 cited in the application -& DATABASE EMBL [Online] 12 August 1993 (1993-08-12), "Helianthus annuus homeodomain containing protein (HAHB-1) mRNA, partial cds." XP002598004 retrieved from EBI accession no. EMBL:L22847 Database accession no. L22847
- KUWABARA CHIKAKO ET AL: "Molecular Basis of Disease Resistance Acquired through Cold Acclimation in Overwintering Plants" JOURNAL OF PLANT BIOLOGY, vol. 52, no. 1, February 2009 (2009-02), pages 19-26, XP002597807 ISSN: 1226-9239
- NI YONGXIANG ET AL: "Novel cotton homeobox gene and its expression profiling in root development and in response to stresses and phytohormones" ACTA BIOCHIMICA ET BIOPHYSICA SINICA, vol. 40, no. 1, January 2008 (2008-01), pages 78-84, XP002597808 ISSN: 1672-9145
- ARIEL ET AL: "The true story of the HD-Zip family" TRENDS IN PLANT SCIENCE, ELSEVIER SCIENCE, OXFORD, GB LNKD- DOI:10.1016/J.TPLANTS.2007.08.003, vol. 12, no. 9, 1 September 2007 (2007-09-01), pages 419-426, XP022233970 ISSN: 1360-1385 cited in the application
- Fuminori Kobayashi ET AL: "Positive role of a wheat HvABI5 ortholog in abiotic stress response of seedlings", Physiologia Plantarum, vol. 134, no. 1, 1 September 2008 (2008-09-01), pages 74-86, XP55084381, ISSN: 0031-9317, DOI: 10.1111/j.1399-3054.2008.01107.x
- ZHANG JI-YI ET AL: "Heterologous expression of two Medicago truncatula putative ERF transcription factor genes, WXP1 and WXP2, in Arabidopsis led to increased leaf wax accumulation and improved drought tolerance, but differential response in freezing tolerance", PLANT MOLECULAR BIOLOGY, vol. 64, no. 3, June 2007 (2007-06), pages 265-278, ISSN: 0167-4412
- ZHOU QI-YUN ET AL: "Soybean WRKY-type transcription factor genes, GmWRKY13, GmWRKY21, and GmWRKY54, confer differential tolerance to abiotic stresses in transgenic Arabidopsis plants.", PLANT BIOTECHNOLOGY JOURNAL JUN 2008, vol. 6, no. 5, June 2008 (2008-06), pages 486-503, ISSN: 1467-7652
- ZHANG GAIYUN ET AL: "Overexpression of the soybean GmERF3 gene, an AP2/ERF type transcription factor for increased tolerances to salt, drought, and diseases in transgenic tobacco.", JOURNAL OF EXPERIMENTAL BOTANY 2009, vol. 60, no. 13, 2009, pages 3781-3796, ISSN: 1460-2431
- HAAKE VOLKER ET AL: "Transcription factor CBF4 is a regulator of drought adaptation in Arabidopsis.", PLANT PHYSIOLOGY OCT 2002, vol. 130, no. 2, October 2002 (2002-10), pages 639-648, ISSN: 0032-0889

### SEQUENCE LISTING

<110> Universidad Nacional del Litoral Consejo Nacional De Investigaciones Cientificas Y Tecnicas Plant Bioscience Limited
<120> Methods and compositions for stress tolerance in plants
<130> PC925013WO
<150> GB 0909318.8
   <151> 2009-06-01
<160> 81
<170> PatentIn version 3.3
<210> 1
   <211> 1011
   <212> DNA
   <213> Helianthus annuus
<400> 1
   gtcgagctcg tctcgtaaaa tgttcgagtc agctccaatt aaatcatgtc ggcttgttat      60
   atttttttta atttattttt gatatttttt acatatattt ataacataaa aaacaaaata     120
   aaaataaaaa ttacacatat atctatatgt attatttttc taaaatttta aatagcgaaa     180
   gacatattaa aagtattata tgtataattt tgtttagctt cccatatttt tatatgttat     240
   taattaatta aacttaaaat gttaacactt taacacctct tacatacttt ttagttcaat     300
   acatttaaaa ttaaaattaa tctatatgca ataaaataat tcaagcaggc ttgcaagctc     360
   acgagtcgag ccatgcctgg ctcgagctcg actcatttac aaatcgagcc accaagccga     420
   ctcgtttata accgagtttt ttttagccga gtttttttca agcgaacttc aaacaagtca     480
   cgcgctttta attaacacat tctagtctaa agaagataat tgaaagagaa agtagatata     540
   agtaaaagga gtagccaaag atataaattt agggtctaaa caacctaata ttgtttaatt     600
   ttttttaaat aaactagttt ttttttaccg attatctgtg ttatatgtct tagtttgaca       660
   tgataagtta tcataattac ttgtagtatt tttatatcag aaatatacgt tggagaatta     720
   aattttatcc tgatcgtcaa ttgacaagaa caaaaatcaa catctcatgg ttttttacta     780
   atttatatga ttaaagatat atggttgtaa gaaaaagaac aatgtacatc aaatggtgaa     840
   atttgaatat ttgatagtaa cgtaatccat tgtgtatttc ttattatttt atcattttcc     900
   caaggtgtgt catatatagt gtctccattc tttctatagc acaatatcct tcacctccct     960
   ctctctctct ctctctctaa aaatgatgat gagacgacaa agatcgaatt c             1011
<210> 2
   <211> 1195
   <212> RNA
   <213> Helianthus annuus
<400> 2
   uugaaauucu gagaaaagcc acauaaucaa agcuaaagag gugguuuaaa cagcugauga      60
   cuugcacugg aauggcuuuc uucuccucca auuucauguu acaauccucc caagaagaug     120
   accaucaugc cccuacaucu cucucuccaa uccucccacc uugcaguacc accacucaag     180
   auuucagugg ugcugcuuuc uugggaaaaa gaucuauguc uucuuacuca gguuugaaca     240
   acaacaacau ggauggaugu gaucaagaag ggaacaugaa uggagaagau gaguuaucag     300
   augauggauc acagcuucuu gcaggagaga aaaagaggag auuaaacaug gaacaaguga     360
   agacacuuga gagaaacuuu gaguuaggaa auaagcuuga accugagagg aaaaugcaac     420
   uugcaagagc acuuggacua caaccaagac agauugcuau augguuucaa aacagaagag     480
   cuagauggaa aacuaaacag uuggaaaaag acuaugaugc ccucaagaga caguuugaag     540
   cuguuaaagc ugagaaugau ucacuccaau cucaaaauca uaaacuucau gcugagauaa     600
   uggcacuaaa aaauagggag ccagcagaac uaaucaaccu caacauaaaa gaaacagaag     660
   gaucuugcag caaccgaagc gaaaacagcu cugaaaucaa acuagacauc ucaagaacac     720
   cggcuaccga uagcccuuua ucaucacacc aucaacacca acaccagcca auaccuaauc     780
   uuuuuccauc gucgaauauc gauaggccua auucgaauaa cauuguggcg caucaacuuu     840
   uccacaauuc gucaucaagg ccggcagauc aucaacuuca uugccacaaa cucgaucaau     900
   cgaaugccau uaaagaagaa uguuuuagca caauguuugu ugguauggau gaucaaucag     960
   gguuuuggcc augguuggaa caaccacaau ucaauugaug gaaucaagaa gcaaaaaagc    1020
   aaaagaaaac gguacccgau ucgccuucuu ggcuuugguu ugauuauauu aaagauggag    1080
   aucaucaauc uguuuguucu cuaagcuuua aauucuuguu uuuugguacu uaaauuaaua    1140
   gaguaaaaau uagaagaaaa aacguauuau uauuuuaaau ucaagauuag uguuu         1195
<210> 3
   <211> 315
   <212> DNA
   <213> Helianthus annuus
<400> 3
   aattaactca ccttaactaa gttacttatg acaacatctc tctcatagat cttgatgcag 60
   cttgcattca tgagttgtga tgtacaactc attcatgcat tagggtttca gttttttcaa 120
   agtttttttt ttattttttc ttctgtttca agatcatgat gatgagttgt gctgaacact 180
   tgaacagctc attgatgcat tagggtttgt tttagtttca agttctttct tttctttcat 240
   tttcatgcac taaatccata tgggcttgaa gaaagtttga atctttatat gttagttgat 300
   gatcttgatg caggt 315
<210> 4
   <211> 778
   <212> DNA
   <213> Helianthus annuus
<400> 4
   gtaatattag tttgattgtt tattgcatct atcaatcatt agattctact ctttacttga 60
   tcacacagaa agtaactaaa ccttttttcc taatgataac aatatttgtt ttgcaaatct 120
   aatggcaatc aaataaaagt ttctggtaag cagccatgat ctatttattt ttcactattt 180
   gagtaagttt aaaagttgca tttatcctca ctaattatat acaacactaa aataatcatt 240
   aaactgactg ttataattac tttccgtaaa cggtatgcca aaacttaaaa tgattaacaa 300
   ttttataaga atggaaagta aaatcattac actatttccc atattagtca tgaccaaagt 360
   ttgtttcttt ctgaagggca aaagggtcaa tatgcttata tgcagcatgg gcaaaagaag 420
   tagagtgtat atcaaaattc atatctttat tttcttttca aagtttaggt aacaaaaaga 480
   agaaattata aacgagtttg ttacaattcc acaagtacat gaagaaacaa aatttgttag 540
   tatttttatt ttccatgttt ttagtaactt ccatatcaat ttagcactag aagataactt 600
   tttttaggac tcggtaaacc atacaagtag ggtcatactt tatcgtttat ccattaatgt 660
   atatccataa attcactgat tatgcggtat ttcctttgtt acactgtctt gaacaagtat 720
   tagtacatgt agtttcttaa agattgttta atcaaccaaa aagattgaaa ctttgcag 778
<210> 5
   <211> 313
   <212> PRT
   <213> Helianthus annuus
<400> 5
   Met Thr Cys Thr Gly Met Ala Phe Phe Ser Ser Asn Phe Met Leu Gln
   1               5                   10                  15
   Ser Ser Gln Glu Asp Asp His His Ala Pro Thr Ser Leu Ser Pro Ile
               20                  25                  30
   Leu Pro Pro Cys Ser Thr Thr Thr Gln Asp Phe Ser Gly Ala Ala Phe
           35                  40                  45
   Leu Gly Lys Arg Ser Met Ser Ser Tyr Ser Gly Leu Asn Asn Asn Asn
       50                  55                  60
   Met Asp Gly Cys Asp Gln Glu Gly Asn Met Asn Gly Glu Asp Glu Leu
   65                  70                  75                  80
   Ser Asp Asp Gly Ser Gln Leu Leu Ala Gly Glu Lys Lys Arg Arg Leu
                   85                  90                  95
   Asn Met Glu Gln Val Lys Thr Leu Glu Arg Asn Phe Glu Leu Gly Asn
               100                 105                 110
   Lys Leu Glu Pro Glu Arg Lys Met Gln Leu Ala Arg Ala Leu Gly Leu
           115                 120                 125
   Gln Pro Arg Gln Ile Ala Ile Trp Phe Gln Asn Arg Arg Ala Arg Trp
       130                 135                 140
   Lys Thr Lys Gln Leu Glu Lys Asp Tyr Asp Ala Leu Lys Arg Gln Phe
   145                 150                 155                 160
   Glu Ala Val Lys Ala Glu Asn Asp Ser Leu Gln Ser Gln Asn His Lys
                   165                 170                 175
   Leu His Ala Glu Ile Met Ala Leu Lys Asn Arg Glu Pro Ala Glu Leu
               180                 185                 190
   Ile Asn Leu Asn Ile Lys Glu Thr Glu Gly Ser Cys Ser Asn Arg Ser
           195                 200                 205
   Glu Asn Ser Ser Glu Ile Lys Leu Asp Ile Ser Arg Thr Pro Ala Thr
       210                 215                 220
   Asp Ser Pro Leu Ser Ser His His Gln His Gln His Gln Pro Ile Pro
   225                 230                 235                 240
   Asn Leu Phe Pro Ser Ser Asn Ile Asp Arg Pro Asn Ser Asn Asn Ile
                   245                 250                 255
   Val Ala His Gln Leu Phe His Asn Ser Ser Ser Arg Pro Ala Asp His
               260                 265                 270
   Gln Leu His Cys His Lys Leu Asp Gln Ser Asn Ala Ile Lys Glu Glu
           275                 280                 285
   Cys Phe Ser Thr Met Phe Val Gly Met Asp Asp Gln Ser Gly Phe Trp
       290                 295                 300
   Pro Trp Leu Glu Gln Pro Gln Phe Asn
   305                 310
<210> 6
   <211> 1195
   <212> DNA
   <213> Helianthus annuus
<400> 6
   ttgaaattct gagaaaagcc acataatcaa agctaaagag gtggtttaaa cagctgatga      60
   cttgcactgg aatggctttc ttctcctcca atttcatgtt acaatcctcc caagaagatg     120
   accatcatgc ccctacatct ctctctccaa tcctcccacc ttgcagtacc accactcaag     180
   atttcagtgg tgctgctttc ttgggaaaaa gatctatgtc ttcttactca ggtttgaaca     240
   acaacaacat ggatggatgt gatcaagaag ggaacatgaa tggagaagat gagttatcag     300
   atgatggatc acagcttctt gcaggagaga aaaagaggag attaaacatg gaacaagtga     360
   agacacttga gagaaacttt gagttaggaa ataagcttga acctgagagg aaaatgcaac     420
   ttgcaagagc acttggacta caaccaagac agattgctat atggtttcaa aacagaagag     480
   ctagatggaa aactaaacag ttggaaaaag actatgatgc cctcaagaga cagtttgaag     540
   ctgttaaagc tgagaatgat tcactccaat ctcaaaatca taaacttcat gctgagataa     600
   tggcactaaa aaatagggag ccagcagaac taatcaacct caacataaaa gaaacagaag     660
   gatcttgcag caaccgaagc gaaaacagct ctgaaatcaa actagacatc tcaagaacac     720
   cggctaccga tagcccttta tcatcacacc atcaacacca acaccagcca atacctaatc     780
   tttttccatc gtcgaatatc gataggccta attcgaataa cattgtggcg catcaacttt     840
   tccacaattc gtcatcaagg ccggcagatc atcaacttca ttgccacaaa ctcgatcaat     900
   cgaatgccat taaagaagaa tgttttagca caatgtttgt tggtatggat gatcaatcag     960
   ggttttggcc atggttggaa caaccacaat tcaattgatg gaatcaagaa gcaaaaaagc    1020
   aaaagaaaac ggtacccgat tcgccttctt ggctttggtt tgattatatt aaagatggag    1080
   atcatcaatc tgtttgttct ctaagcttta aattcttgtt ttttggtact taaattaata    1140
   gagtaaaaat tagaagaaaa aacgtattat tattttaaat tcaagattag tgttt          1195
<210> 7
   <211> 3299
   <212> DNA
   <213> Helianthus annuus
<400> 7
   gtcgagctcg tctcgtaaaa tgttcgagtc agctccaatt aaatcatgtc ggcttgttat     60
   atttttttta atttattttt gatatttttt acatatattt ataacataaa aaacaaaata    120
   aaaataaaaa ttacacatat atctatatgt attatttttc taaaatttta aatagcgaaa    180
   gacatattaa aagtattata tgtataattt tgtttagctt cccatatttt tatatgttat    240
   taattaatta aacttaaaat gttaacactt taacacctct tacatacttt ttagttcaat    300
   acatttaaaa ttaaaattaa tctatatgca ataaaataat tcaagcaggc ttgcaagctc    360
   acgagtcgag ccatgcctgg ctcgagctcg actcatttac aaatcgagcc accaagccga    420
   ctcgtttata accgagtttt ttttagccga gtttttttca agcgaacttc aaacaagtca    480
   cgcgctttta attaacacat tctagtctaa agaagataat tgaaagagaa agtagatata    540
   agtaaaagga gtagccaaag atataaattt agggtctaaa caacctaata ttgtttaatt    600
   ttttttaaat aaactagttt ttttttaccg attatctgtg ttatatgtct tagtttgaca    660
   tgataagtta tcataattac ttgtagtatt tttatatcag aaatatacgt tggagaatta    720
   aattttatcc tgatcgtcaa ttgacaagaa caaaaatcaa catctcatgg ttttttacta    780
   atttatatga ttaaagatat atggttgtaa gaaaaagaac aatgtacatc aaatggtgaa    840
   atttgaatat ttgatagtaa cgtaatccat tgtgtatttc ttattatttt atcattttcc 900
   caaggtgtgt catatatagt gtctccattc tttctatagc acaatatcct tcacctccct 960
   ctctctctct ctctctctaa aaatgatgat gagacgacaa agatcgaatt cttgaaattc 1020
   tgagaaaagc cacataatca aagctaaaga ggtggtttaa acagctgatg acttgcactg 1080
   gaatggcttt cttctcctcc aatttcatgt tacaatcctc ccaagaagat gaccatcatg 1140
   cccctacatc tctctctcca atcctcccac cttgcagtac caccactcaa gatttcagtg 1200
   gtaattaact caccttaact aagttactta tgacaacatc tctctcatag atcttgatgc 1260
   agcttgcatt catgagttgt gatgtacaac tcattcatgc attagggttt cagttttttc 1320
   aaagtttttt ttttattttt tcttctgttt caagatcatg atgatgagtt gtgctgaaca 1380
   cttgaacagc tcattgatgc attagggttt gttttagttt caagttcttt cttttctttc 1440
   attttcatgc actaaatcca tatgggcttg aagaaagttt gaatctttat atgttagttg 1500
   atgatcttga tgcaggtgct gctttcttgg gaaaaagatc tatgtcttct tactcaggtt 1560
   tgaacaacaa caacatggat ggatgtgatc aagaagggaa catgaatgga gaagatgagt 1620
   tatcagatga tggatcacag cttcttgcag gagagaaaaa gaggagatta aacatggaac 1680
   aagtgaagac acttgagaga aactttgagt taggaaataa gcttgaacct gagaggaaaa 1740
   tgcaacttgc aagagcactt ggactacaac caagacagat tgctatatgg tttcaaaaca 1800
   gaagagctag atggaaaact aaacagttgg aaaaagacta tgatgccctc aagagacagt 1860
   ttgaagctgt taaagctgag aatgattcac tccaatctca aaatcataaa cttcatgctg 1920
   gtaatattag tttgattgtt tattgcatct atcaatcatt agattctact ctttacttga 1980
   tcacacagaa agtaactaaa ccttttttcc taatgataac aatatttgtt ttgcaaatct 2040
   aatggcaatc aaataaaagt ttctggtaag cagccatgat ctatttattt ttcactattt 2100
   gagtaagttt aaaagttgca tttatcctca ctaattatat acaacactaa aataatcatt 2160
   aaactgactg ttataattac tttccgtaaa cggtatgcca aaacttaaaa tgattaacaa 2220
   ttttataaga atggaaagta aaatcattac actatttccc atattagtca tgaccaaagt 2280
   ttgtttcttt ctgaagggca aaagggtcaa tatgcttata tgcagcatgg gcaaaagaag 2340
   tagagtgtat atcaaaattc atatctttat tttcttttca aagtttaggt aacaaaaaga 2400
   agaaattata aacgagtttg ttacaattcc acaagtacat gaagaaacaa aatttgttag 2460
   tatttttatt ttccatgttt ttagtaactt ccatatcaat ttagcactag aagataactt 2520
   tttttaggac tcggtaaacc atacaagtag ggtcatactt tatcgtttat ccattaatgt 2580
   atatccataa attcactgat tatgcggtat ttcctttgtt acactgtctt gaacaagtat 2640
   tagtacatgt agtttcttaa agattgttta atcaaccaaa aagattgaaa ctttgcagag 2700
   ataatggcac taaaaaatag ggagccagca gaactaatca acctcaacat aaaagaaaca 2760
   gaaggatctt gcagcaaccg aagcgaaaac agctctgaaa tcaaactaga catctcaaga 2820
   acaccggcta ccgatagccc tttatcatca caccatcaac accaacacca gccaatacct 2880
   aatctttttc catcgtcgaa tatcgatagg cctaattcga ataacattgt ggcgcatcaa 2940
   cttttccaca attcgtcatc aaggccggca gatcatcaac ttcattgcca caaactcgat 3000
   caatcgaatg ccattaaaga agaatgtttt agcacaatgt ttgttggtat ggatgatcaa 3060
   tcagggtttt ggccatggtt ggaacaacca caattcaatt gatggaatca agaagcaaaa 3120
   aagcaaaaga aaacggtacc cgattcgcct tcttggcttt ggtttgatta tattaaagat 3180
   ggagatcatc aatctgtttg ttctctaagc tttaaattct tgtttttggg tacttaaatt 3240
   aatagagtaa aaattagaag aaaaaacgta ttattatttt aaattcaaga ttagtgttt 3299
<210> 8
   <211> 122
   <212> PRT
   <213> Helianthus annuus
<400> 8
   Leu Ile Asn Leu Asn Ile Lys Glu Thr Glu Gly Ser Cys Ser Asn Arg
   1 5 10 15
   Ser Glu Asn Ser Ser Glu Ile Lys Leu Asp Ile Ser Arg Thr Pro Ala
   20 25 30
   Thr Asp Ser Pro Leu Ser Ser His His Gln His Gln His Gln Pro Ile
   35 40 45
   Pro Asn Leu Phe Pro Ser Ser Asn Ile Asp Arg Pro Asn Ser Asn Asn
   50 55 60
   Ile Val Ala His Gln Leu Phe His Asn Ser Ser Ser Arg Pro Ala Asp
   65 70 75 80
   His Gln Leu His Cys His Lys Leu Asp Gln Ser Asn Ala Ile Lys Glu
   85 90 95
   Glu Cys Phe Ser Thr Met Phe Val Gly Met Asp Asp Gln Ser Gly Phe
   100 105 110
   Trp Pro Trp Leu Glu Gln Pro Gln Phe Asn
   115 120
<210> 9
   <211> 35
   <212> PRT
   <213> Helianthus annuus
<400> 9
   Leu Ile Asn Leu Asn Ile Lys Glu Thr Glu Gly Ser Cys Ser Asn Arg
   1               5                   10                  15
   Ser Glu Asn Ser Ser Glu Ile Lys Leu Asp Ile Ser Arg Thr Pro Ala
               20                  25                  30
   Thr Asp Ser
           35
<210> 10
   <211> 29
   <212> PRT
   <213> Helianthus annuus
<400> 10
   Ile Lys Glu Glu Cys Phe Ser Thr Met Phe Val Gly Met Asp Asp Gln
   1               5                   10                  15
   Ser Gly Phe Trp Pro Trp Leu Glu Gln Pro Gln Phe Asn
               20                  25
<210> 11
   <211> 34
   <212> PRT
   <213> Helianthus annuus
<400> 11
   Ser Ile Asn Leu Asn Lys Glu Thr Glu Gly Ser Cys Ser Asn Arg Ser
   1               5                   10                  15
   Glu Asn Ser Ser Asp Ile Lys Leu Asp Ile Ser Arg Thr Pro Ala Ile
               20                  25                  30
   Asp Ser
<210> 12
   <211> 29
   <212> PRT
   <213> Helianthus annuus
<400> 12
   Val Lys Glu Glu Ser Leu Ser Asn Met Phe Cys Gly Ile Asp Asp Gln
   1 5 10 15
   Ser Gly Phe Trp Pro Trp Leu Glu Gln Gln His Phe Asn
   20 25
<210> 13
   <211> 91
   <212> PRT
   <213> Helianthus annuus
<400> 13
   Met Thr Cys Thr Gly Met Ala Phe Phe Ser Ser Asn Phe Met Leu Gln
   1 5 10 15
   Ser Ser Gln Glu Asp Asp His His Ala Pro Thr Ser Leu Ser Pro Ile
   20 25 30
   Leu Pro Pro Cys Ser Thr Thr Thr Gln Asp Phe Ser Gly Ala Ala Phe
   35 40 45
   Leu Gly Lys Arg Ser Met Ser Ser Tyr Ser Gly Leu Asn Asn Asn Asn
   50 55 60
   Met Asp Gly Cys Asp Gln Glu Gly Asn Met Asn Gly Glu Asp Glu Leu
   65 70 75 80
   Ser Asp Asp Gly Ser Gln Leu Leu Ala Gly Glu
   85 90
<210> 14
   <211> 57
   <212> PRT
   <213> Helianthus annuus
<400> 14
   Lys Lys Arg Arg Leu Thr Asp Glu Gln Val Lys Ala Leu Glu Lys Ser
   1 5 10 15
   Phe Glu Leu Glu Asn Lys Leu Glu Pro Glu Arg Lys Val Gln Leu Ala
   20 25 30
   Arg Glu Leu Gly Leu Gln Pro Arg Gln Val Ala Val Trp Phe Gln Asn
   35 40 45
   Arg Arg Ala Arg Trp Lys Thr Lys Gln
   50 55
<210> 15
   <211> 33
   <212> DNA
   <213> Artificial
<220>
   <223> Primer PrH1R
<400> 15
   cggggatccc ctctttagct ttgattatgt ggc 33
<210> 16
   <211> 16
   <212> DNA
   <213> Artificial
<220>
   <223> Primer PR-TOPO
<400> 16
   aacagctatg accatg 16
<210> 17
   <211> 21
   <212> DNA
   <213> Artificial
<220>
   <223> Primer H1qF
<400> 17
   ggccggcaga tcatcaactt c 21
<210> 18
   <211> 21
   <212> DNA
   <213> Artificial
<220>
   <223> Primer H1qR
<400> 18
   ccaaccatgg ccaaaaccct g 21
<210> 19
   <211> 32
   <212> DNA
   <213> Artificial
<220>
   <223> Primer PR4 CDS-F
<400> 19
   ggcggatccc caccaagaaa acaaagactt at 32
<210> 20
   <211> 27
   <212> DNA
   <213> Artificial
<220>
   <223> Primer PR4 CDS-R
<400> 20
   ggggagctcc cgatcgatat tgacctc 27
<210> 21
   <211> 30
   <212> DNA
   <213> Artificial
<220>
   <223> Primer PR2 CDS-F
<400> 21
   ggcggatcca agaaaatgtc tgaatcaagg 30
<210> 22
   <211> 27
   <212> DNA
   <213> Artificial
<220>
   <223> Primer PR2 CDS-R
<400> 22
   ggggagctcg cccacaagtc tctaagg 27
<210> 23
   <211> 31
   <212> DNA
   <213> Artificial
<220>
   <223> Primer Glucanase CDS-F
<400> 23
   cgcggatccc taaggagcta agaacaaacc c 31
<210> 24
   <211> 29
   <212> DNA
   <213> Artificial
<220>
   <223> Primer Glucanase CDS-R
<400> 24
   cccgagctca tcactcaacc gccgtaccg 29
<210> 25
   <211> 24
   <212> DNA
   <213> Artificial
<220>
   <223> Primer tc23839-F
<400> 25
   aaaagtggtt tatttggatg agga 24
<210> 26
   <211> 22
   <212> DNA
   <213> Artificial
<220>
   <223> Primer tc23839-R
<400> 26
   gacacgtcag aacaaaattc ca 22
<210> 27
   <211> 25
   <212> DNA
   <213> Artificial
<220>
   <223> Primer tc19654-F
<400> 27
   gatcttcgtg acctgcttct aaaac 25
<210> 28
   <211> 26
   <212> DNA
   <213> Artificial
<220>
   <223> Primer tc19654-R
<400> 28
   caaaccactt ctaaatcatc ccatag 26
<210> 29
   <211> 26
   <212> DNA
   <213> Artificial
<220>
   <223> Primer tc16546-F
<400> 29
   caaaacaact tcttccacca atagtc 26
<210> 30
   <211> 25
   <212> DNA
   <213> Artificial
<220>
   <223> Primer tc16546-R
<400> 30
   aataaaccgt tgacttttct tcacc 25
<210> 31
   <211> 24
   <212> DNA
   <213> Artificial
<220>
   <223> Primer tc18434-F
<400> 31
   acatcatcaa cggtggttta gaat 24
<210> 32
   <211> 25
   <212> DNA
   <213> Artificial
<220>
   <223> Primer 18434-R
<400> 32
   acatggtgca ataccttctg taaaa 25
<210> 33
   <211> 24
   <212> DNA
   <213> Artificial
<220>
   <223> Primer At3g12500-F
<400> 33
   gggttatgga gtgattacga acat 24
<210> 34
   <211> 24
   <212> DNA
   <213> Artificial
<220>
   <223> Primer At3g12500-R
<400> 34
   taccaccagg attaacacca aata 24
<210> 35
   <211> 24
   <212> DNA
   <213> Artificial
<220>
   <223> Primer At3g57260-R
<400> 35
   taagaaggaa ccaacgtatg agaa 24
<210> 36
   <211> 24
   <212> DNA
   <213> Artificial
<220>
   <223> Primer At3g57260-R
<400> 36
   cataaaaagc ccacaagtct ctaa 24
<210> 37
   <211> 24
   <212> DNA
   <213> Artificial
<220>
   <223> Primer At3g04720-F
<400> 37
   attgaacatt gctacatcca aatc 24
<210> 38
   <211> 24
   <212> DNA
   <213> Artificial
<220>
   <223> Primer At3g04720-R
<400> 38
   attgaacatt gctacatcca aatc 24
<210> 39
   <211> 20
   <212> DNA
   <213> Artificial
<220>
   <223> Primer At1g62440-F
<400> 39
   accaacacca ccttctctgc 20
<210> 40
   <211> 20
   <212> DNA
   <213> Artificial
<220>
   <223> Primer At1g62440-R
<400> 40
   ttgatggtgg aggaggagac 20
<210> 41
   <211> 22
   <212> DNA
   <213> Artificial
<220>
   <223> Primer At2g43050-F
<400> 41
   ggttaaatgg agtgggtgtc at 22
<210> 42
   <211> 22
   <212> DNA
   <213> Artificial
<220>
   <223> Primer At2g43050-R
<400> 42
   caagtcctgg gtcgaaacta ac 22
<210> 43
   <211> 24
   <212> DNA
   <213> Artificial
<220>
   <223> Primer At4g16260-R
<400> 43
   gagacctgga agaggagtgg aaac 24
<210> 44
   <211> 24
   <212> DNA
   <213> Artificial
<220>
   <223> Primer At4g16260-R
<400> 44
   aatgtgatcg gaaattttgg ttgt 24
<210> 45
   <211> 24
   <212> DNA
   <213> Artificial
<220>
   <223> Primer At4g25480-F
<400> 45
   gacgttggtg gaggctattt acac 24
<210> 46
   <211> 24
   <212> DNA
   <213> Artificial
<220>
   <223> Primer At4g25480-R
<400> 46
   tattagccaa caaactcggc atct 24
<210> 47
   <211> 24
   <212> DNA
   <213> Artificial
<220>
   <223> Primer At4g02380-F
<400> 47
   atgctatctt ccgacgtggt tatg 24
<210> 48
   <211> 24
   <212> DNA
   <213> Artificial
<220>
   <223> Primer At4g02380-R
<400> 48
   cttccactcc cttcttcttc atca 24
<210> 49
   <211> 22
   <212> DNA
   <213> Artificial
<220>
   <223> Primer At4g25490-R
<400> 49
   cgttggcttt tcaagatgag ac 22
<210> 50
   <211> 24
   <212> DNA
   <213> Artificial
<220>
   <223> Primer At4g25490-R
<400> 50
   cgctctgttc cggtgtataa atag 24
<210> 51
   <211> 23
   <212> DNA
   <213> Artificial
<220>
   <223> Primer At1g27730-F
<400> 51
   gtccactagc cacgttagca gta 23
<210> 52
   <211> 23
   <212> DNA
   <213> Artificial
<220>
   <223> Primer At1g27730-R
<400> 52
   agttgaagtt tgaccggaaa gtc 23
<210> 53
   <211> 24
   <212> DNA
   <213> Artificial
<220>
   <223> Primer At3g04720-F
<400> 53
   attgaacatt gctacatcca aatc 24
<210> 54
   <211> 24
   <212> DNA
   <213> Artificial
<220>
   <223> Primer At3g12500-F
<400> 54
   gggttatgga gtgattacga acat 24
<210> 55
   <211> 24
   <212> DNA
   <213> Artificial
<220>
   <223> Primer At3g12500-R
<400> 55
   taccaccagg attaacacca aata 24
<210> 56
   <211> 25
   <212> DNA
   <213> Artificial
<220>
   <223> Primer At3g52130-F
<400> 56
   tttctcttta ataaccttgc tgctt 25
<210> 57
   <211> 24
   <212> DNA
   <213> Artificial
<220>
   <223> Primer At3g52130-R
<400> 57
   gctaatgact gagattttga ttcg 24
<210> 58
   <211> 24
   <212> DNA
   <213> Artificial
<220>
   <223> Primer At3g07450-F
<400> 58
   gacacttggt caaccttgtt tatg 24
<210> 59
   <211> 24
   <212> DNA
   <213> Artificial
<220>
   <223> Primer At3g07450-R
<400> 59
   tacatggaag aaaattggca gaac 24
<210> 60
   <211> 25
   <212> DNA
   <213> Artificial
<220>
   <223> Primer At5g44420-F
<400> 60
   cttgttctct ttgctgcttt cgacg 25
<210> 61
   <211> 24
   <212> DNA
   <213> Artificial
<220>
   <223> Primer At5g44420-R
<400> 61
   cttcaaggtt aatgcactga ttct 24
<210> 62
   <211> 24
   <212> DNA
   <213> Artificial
<220>
   <223> Primer At3g57260-F
<400> 62
   taagaaggaa ccaacgtatg agaa 24
<210> 63
   <211> 24
   <212> DNA
   <213> Artificial
<220>
   <223> Primer At3g57260-R
<400> 63
   cataaaaagc ccacaagtct ctaa 24
<210> 64
   <211> 1813
   <212> DNA
   <213> Arabidopsis thaliana
<400> 64
   accagaagtg gtatagtcta ggccgataca tttcactatc tctctctctt ttgtttttcc 60
   tcttcttctt tttttccatt tgatttcaaa ctctcacaca aagagcttca gatttataag 120
   accatgataa tggctttaag acaaagattg gcaagaagaa aaaactaaag agaaacgacc 180
   aaaatctcaa gcaaacagta ctaacttctg ttgcaaaaca gaagaagatg tcttgtaata 240
   atggaatgtc ttttttccct tcaaatttca tgatccaaac ctcttacgaa gatgatcatc    300
   ctcatcaatc tccatctctt gctcctcttc ttccttcttg ctctctacct caagatctcc    360
   atggtatata tacataaact tccacacaca tctcctctgt tttctctcta tctctttcta    420
   atgctctgtt ctgttctgtt tcaggatttg cttcgtttct aggtaagaga tctccaatgg    480
   aagggtgttg tgatttagaa acagggaaca atatgaatgg agaagaggat tattcagatg    540
   atgggtcaca aatgggagag aagaagagga gattgaacat ggaacaagtg aagacactag    600
   agaagaactt tgagcttgga aacaaacttg aaccagagag gaaaatgcag ctagctcgtg    660
   ccttaggttt gcaaccaaga cagatcgcga tttggtttca aaatcgaaga gctcgttgga    720
   aaacaaagca gctagagaaa gattatgata ctcttaaacg acagtttgat acacttaaag    780
   ctgaaaatga tcttcttcaa actcataatc agaaactcca agctgaggta attaatctca    840
   taaattaaca aaaaaaatca atatgtgtta tttttttttg ggttaatgat caataattac    900
   agttattttc catctaaagg atgatttttt tctttttaaa aaaggttaaa aattatattt    960
   ctggtttata attatttgga tcaggagttg ctttcaggta gggttaaaaa actggacatg   1020
   attcatgact tttcagacat cattatctct ttttttcttc actcttgtct ggaaagagat   1080
   ctgaaaacaa tagtttcttt atgcttatca cattgtacag taactctgtt tatgtttaaa   1140
   attttgtctt taattacgca gataatggga ttaaaaaaca gagaacaaac agaatcaata   1200
   aatctaaaca aagaaactga aggatcttgc agtaacagaa gtgataacag ttcagataat   1260
   ctcagactag atatctcaac tgcgccgcca tcaaacgaca gtacattaac cggtggccac   1320
   ccaccgccac cacagacagt tggtcgacac ttcttcccac cgtcgccagc caccgcaacg   1380
   acaactacta caacaatgca gttctttcaa aactcatctt caggacagag tatggttaaa   1440
   gaagagaata gtatcagtaa catgttctgt gcaatggatg accattctgg tttttggcca   1500
   tggcttgatc agcaacagta caattgaaat tggtctacct gttttttttg tttttgtttt   1560
   taaaaaaatt tatatttttt ttttttgtat ttggaatttt gatcagaaga acccatgcat   1620
   gttttcaaaa actggaatct atatcattag ctcactttga aatctgcaac caaacaccac   1680
   tgaggttttt tgtttacttt ttgagtaaat gagatgtaaa aaaatgggta atatccatta   1740
   tattatataa aaaataatat cattatggcc caacattttt ctgtatggag aaaaataaaa   1800
   taaattgtat att                                                      1813
<210> 65
   <211> 1544
   <212> DNA
   <213> Arabidopsis thaliana
<400> 65
   ctactaaaaa aattgtaagt acatacatat caatgttaat ttgtatataa ggagctaaga      60
   acaaacccaa ttaggcaact agcaattgct aaaacacgta agatctcaaa tatgaccacg     120
   ttattcctcc ttattgctct attcatcaca accatcctca acccaacaag tctctctctc     180
   tctctcttta atctcacatg atttattctc attttcaatt tttatagata tataaactta     240
   atcataatta accttaatta tgttatatga taaactaggt ggagaatcag taggtgtatg     300
   ctatggaatg atggggaaca accttccttc tcaatcagac acaatcgctc tctttagaca     360
   aaacaacatc cgacgtgtta gactctacga tccaaaccaa gccgctttaa acgctcttag     420
   aaacacgggt atcgaagtca tcatcggcgt tccaaacacc gatcttcgtt cactcactaa     480
   cccttcttcc gctagatcat ggctccaaaa caacgtcctc aactattacc ccgccgttag     540
   cttcaagtac atcgccgtag gtaacgaagt atctccgtcg aacggcggtg atgttgtgct     600
   ccctgccatg cgtaacgttt acgatgctct aagaggtgca aatcttcaag atcgtattaa     660
   agtttctacc gccattgata tgactttgat tggaaactct ttccctcctt cctccggaga     720
   gtttcgtggt gacgttagat ggtatatcga tcccgtcatc gggtaatttt ccaaccaaac     780
   caaataacca aaattcatta gatttagtta tttcccaata tttttcattt ctggttactt     840
   gtggaatgat tatttaattt cttcctatgt ggctaattag gtttcttacg agtacgaact     900
   cagcgttact agccaacatc tatccttact tcagctacgt tgacaatcca cgtgacatat     960
   ctctctctta cgctctcttc acttctcctt ccgtcgtcgt atgggacggc tctcgtggct    1020
   accaaaacct ctttgacgct ttacttgacg ttgtttactc tgccgttgaa cgctcaggcg    1080
   gtggatctct cccagtggtt gtttccgaga gcggatggcc ttctaacggt ggaaacgccg    1140
   cgagtttcga taacgcgcga agctttttac acgaatcttg cgtcgcgtgt gagagagaac    1200
   agaggaacac cgaagagacc tggaagagga gtggaaacgt atttgttcgc tatgtttgat    1260
   gagaatcaaa agagtcctga gatcgagaag aattttggtt tgttttttcc taataaacaa    1320
   ccaaaatttc cgatcacatt ctctgccgcg agagacggta cggcggttga gtgatgattt    1380
   tatatgctga gatttatgtg aataattggg agattatccc ataaaaggtt ccaaataaag    1440
   acaaatttca aataaaacct gttagtccaa gttaaattaa atactcggct ttgttttggt    1500
   ccacgttaga cttggtaaag tcatgcaata tttttatttg atat                     1544
<210> 66
   <211> 1343
   <212> DNA
   <213> Arabidopsis thaliana
<400> 66
   atatcatttt tcacagaatc atagaaaaat caagaaaatg tctgaatcaa ggagcttagc      60
   ctcaccacca atgttgatga ttcttctcag ccttgtaata gcttccttct tcaaccacac     120
   aggttcagtc atcttttaag ctattgtaac atctattaat catctccatc ttcacaaatt     180
   tattcaattt aatgattctt attttggaaa atgaagctgg acaaatcgga gtatgctacg     240
   ggatgctagg cgataccttg ccaagtccat cggacgttgt ggctctttac aaacaacaaa     300
   acatccagcg aatgcggctc tacggccctg acccaggcgc tcttgccgct ctccgtggct     360
   ctgacatcga gctcatcctc gacgttccca gttcagatct tgaacgtctc gcctccagtc     420
   aaacggaggc cgacaagtgg gttcaagaaa acgttcagag ctacagagat ggtgtcagat     480
   tccggtacat caacgttgga aatgaggtga aaccctcagt tggggggttt ctcttacaag     540
   caatgcagaa catcgagaac gcggtttctg gagcagggct tgaagtcaag gtctcaacag     600
   ctatagccac tgacaccacc actgatacgt ctcctccgtc tcaaggaagg ttcagggatg     660
   agtataagag ctttctcgaa ccagtgatag gtttcttggc aagcaagcaa tctcccttgc     720
   tcgtgaatct ctacccttac ttcagctaca tgggagacac ggccaacatc catctagact     780
   acgctctgtt caccgcccag tccactgttg ataacgatcc agggtactca taccaaaacc     840
   tattcgacgc aaatctcgac tcggtttatg cagcattgga gaaatcaggg ggcggatcgt     900
   tggaaatcgt ggtgtcggag accggttggc ccacagaggg agcagtcggg acgagtgtgg     960
   aaaacgcaaa gacttatgtt aacaatttga tacaacatgt gaagaatgga tcaccgagaa    1020
   ggccagggaa agctatagag acttatatat tcgctatgtt cgatgagaat aagaaggaac    1080
   caacgtatga gaagttttgg ggactgtttc atccagatcg acagtctaag tatgaagtta    1140
   atttcaacta atccttagag acttgtgggc tttttatgta agcgtattta aaaattggga    1200
   acttgttgta gtaataagga ataattaatg cgctttcagc gtgtagtatg ttgttatttt    1260
   taaggttata aatgagctgc aagcataaat aaggaaaaaa aatagcatgg gcctataggc    1320
   ccaataataa aacaagcttg ctt                                            1343
<210> 67
   <211> 998
   <212> DNA
   <213> Arabidopsis thaliana
<400> 67
   agaccaccaa gaaaacaaag acttatcgat catgaagatc agacttagca taaccatcat 60
   acttttatca tacacagtgg ctacggtggc cggacaacaa tgcggtcgtc aaggcggtgg 120
   tcgaacttgt cccggtaaca tctgctgcag tcagtacggt tactgtggta ccaccgcgga 180
   ctactgttct ccgaccaaca actgtcagag caattgttgg ggaagtgggc ctagcggacc 240
   aggggagagc gcgtcgaacg tacgcgccac ctaccatttc tataatccgg cgcagaataa 300
   ttgggatttg agagccgtga gtgcttattg ctccacgtgg gatgctgata agccgtacgc 360
   atggcggagc aagtatggct ggaccgcctt ctgcgggccg gcaggacctc gtggtcaagc 420
   ttcttgcggc aagtgtttaa gggtaagtta attaattatc tttttctcaa atctttatat 480
   aagtatgttt gtgcaaaagg agatcatata gaaagtgttg gaattaagac gaatacaaga 540
   taaaatttgt taccatttac caacgtcaac gtgttagtga aatatttcaa aagatgtata 600
   gccggtaaaa attgtgatta accggtgggt ataaatggat tcaggtgaag aacacaagaa 660
   caaatgctgc agtaactgtg agaatagtgg accaatgcag caacggaggc ttggatttgg 720
   atgtagcaat gttcaatcaa atagacaccg atggttttgg ctatcaacaa ggccatctca 780
   ttgttgacta ccaatttgtc gactgtggca atgagctcat tgggcagcct gattccagaa 840
   acatgcttgt ttcggccatt gatcgcgttt gatattatgt aatgattttg aggtcaatat 900
   cgatcggtct acataaaaat aataaagacc gctatatatg tattgtcgag ggatatatgt 960
   ttcgtatcaa taaggaaatt ttaaatatta ttatcatt 998
<210> 68
   <211> 20
   <212> DNA
   <213> Artificial
<220>
   <223> GmPR2qF
<400> 68
   ccttcttctg gtggaactgc 20
<210> 69
   <211> 20
   <212> DNA
   <213> Artificial
<220>
   <223> GmPR2qR
<400> 69
   ataggagaaa agagccccca 20
<210> 70
   <211> 22
   <212> DNA
   <213> Artificial
<220>
   <223> NtPR2qF
<400> 70
   ctggtttggg aaacaacatc aa 22
<210> 71
   <211> 22
   <212> DNA
   <213> Artificial
<220>
   <223> NtPR2qR
<400> 71
   aatctggcct ggattaccag aa 22
<210> 72
   <211> 22
   <212> DNA
   <213> Artificial
<220>
   <223> GmPR4qF
<400> 72
   acaggaacag gagcaaacac aa 22
<210> 73
   <211> 22
   <212> DNA
   <213> Artificial
<220>
   <223> GmPR4qR
<400> 73
   cattcccaca atccacaaac tg 22
<210> 74
   <211> 22
   <212> DNA
   <213> Artificial
<220>
   <223> NtPR4qF
<400> 74
   tttggcatgg aggaggaagt at 22
<210> 75
   <211> 22
   <212> DNA
   <213> Artificial
<220>
   <223> NtPR4qR
<400> 75
   tccacgattc tcactgtggt ct 22
<210> 76
   <211> 22
   <212> DNA
   <213> Artificial
<220>
   <223> GmGlucqF
<400> 76
   gagaaagtag gggcaccaaa tg 22
<210> 77
   <211> 22
   <212> DNA
   <213> Artificial
<220>
   <223> GmGlucqR
<400> 77
   ttctggtttc catcaaacat gg 22
<210> 78
   <211> 24
   <212> DNA
   <213> Artificial
<220>
   <223> GmEFlaqF
<400> 78
   tgaaacagat gatttgctgc tgta 24
<210> 79
   <211> 24
   <212> DNA
   <213> Artificial
<220>
   <223> GmEFlaqR
<400> 79
   caatcatgtt gtctccctca aaac 24
<210> 80
   <211> 24
   <212> DNA
   <213> Artificial
<220>
   <223> NtActqF
<400> 80
   ctgatggaca ggttatcacc attg 24
<210> 81
   <211> 24
   <212> DNA
   <213> Artificial
<220>
   <223> NtActqR
<400> 81
   taatgcggta atttccttgc tcat 24

## Description

### FIELD OF THE INVENTION

The disclosure relates to transgenic plants with enhanced tolerance to biotic and abiotic stress and to methods for making such plants. Also disclosed are isolated nucleic acid and peptide sequences.

### BACKGROUND OF THE INVENTION

Adverse climate conditions and human activity as well as biological agents are stress effectors for plants and seriously affect their productivity and survival. Losses in productivity due to this kind of stress reach sometimes more than 50%. Plant breeders have been and are devoted to developing strategies in order to avoid or diminish the negative impact of these situations.

Among abiotic stress-causing factors, drought, salinity of the soil and extreme temperatures are some of the most harmful. Regarding extreme temperatures, stresses are classified into three types: freezing (caused by temperatures below 0°C), chilling (caused by low temperatures over 0°C) and heat stress (caused by high temperatures). Chilling temperatures cause damage to photosynthetic tissues, inhibit the whole photosynthetic process and carbohydrate transport as well as protein biosynthesis and respiration rates. Simultaneously, protein degradation is accelerated. All of these effects occur rather slowly and involve partial or total loss of membrane functionality. In contrast, freezing temperatures cause quick damage, killing the plants. It has been observed, however, that plants subjected to chilling during several days tolerate freezing temperatures better than plants subjected to freezing without first having been exposed to a period of chilling; this process is termed "acclimation".

Species such as winter cereals are adapted to cold or moderate-cold weather and can tolerate temperatures ranging from 0 °C to 15°C, as well as freezing temperatures, rather well if they have previously been acclimated to reduced temperatures (Levitt, 1980, Thomashow, 1999). By contrast, tropical and subtropical species, including important crops, such as maize, rice or tomato, are sensitive to low temperatures and appear to lack efficient acclimation mechanisms.

Chilling and freezing tolerance occurs via different mechanisms. The response to chilling involves the activation of unsaturases which are able to change the lipid composition of the membranes generating increased membrane fluidity at low temperatures. On the other hand, freezing tolerance requires a previous acclimation period. During this acclimation period, certain specific proteins are synthesized and accumulated.

"Antifreeze" proteins are found in a wide range of overwintering plants; they inhibit the growth and recrystallization of ice produced in intercellular spaces at freezing temperatures. These proteins exhibit a high level of homology with pathogenesis-related proteins (PRs) and, in some cases, also protect against psychrophilic pathogens (Griffith & Yaish, 2004; Chinnusamy *et al.,* 2007). Other species present freezing tolerance via a mechanism implying the increase in sucrose (Guy, 1992) or free proline concentrations (Nanjo et *al.,* 1999).

One of the strategies to reduce losses in plant productivity is to increase natural stress tolerance, by strengthening endogenous systems. Transcription factors (TFs) play a crucial role in the plant response to environmental factors as well as in the morphogenetic program. They are proteins acting in *trans,* able to recognize and bind specific DNA sequences (*cis*-acting elements) localized in the regulatory regions of their target genes. When these proteins bind their targets, they activate or repress whole transduction signals pathways.

About 1500 TFs have been identified in plants using bioinformatics, and TFs comprise numerous gene families. However, while they might be involved in the response, they may not necessarily confer a tolerance. This is illustrated, for example, by Arabidopsis TFs ATHB7 and ATHB12 (Lee and Chun, 1998). These exhibit a high homology with sunflower HAHB4, especially in the HD-Zip domain. Both genes are up- regulated by drought and ABA. However, it was also shown that transgenic plants overexpressing these genes are not more tolerant to drought stress than WT ones. HAHB4 is described in WO 2004/099365. Another example is DREB2, a gene that is induced by cold temperatures, but does not confer cold tolerance in its wild type form. Therefore, it continues to be necessary to undertake a series of functional genomic experiments in order to test and demonstrate the effects of TFs on stress tolerance as such effects cannot be predicted (Arce *et al*., 2008).

HD-Zip proteins characterized by the presence of a homeodomain associated with a leucine zipper constitute one family of plant transcription factors. The association of the DNA binding domain (HD) with an adjacent dimerization motif (leucine zipper abbreviated ZipLZ or LZ) is a combination found only in the plant kingdom, although the domains are found independently of each other in a large number of eukaryotic transcription factors (Schena & Davis, 1992). This large family of plant TFs has been divided into four subfamilies (I to IV) according to sequence similarity in and outside the conserved domains and by the intron/exon patterns of the corresponding genes (Schena & Davis, 1994, Sessa et *al.,* 1994, Chan *et al.,* 1998; Ariel et *al.,* 2007). Members of subfamily I interact with the pseudopalindromic sequence CAAT(A/T)ATTG; subfamily II proteins recognize a motif CAAT(C/G)ATTG (Sessa *et al.,* 1993; Palena *et al.,* 1999). In all cases, the formation of protein homo- or hetero-dimers is a prerequisite for DNA binding (Sessa et *al.,* 1993; Gonzalez *et al.,* 1997).

Several authors have reported that expression of members of the HD-Zip family of transcription factors is regulated by various external factors such as illumination, ABA, salt or water stresses (Schena & Davis, 1992; Carabelli *et al.,* 1993; Schena *et al.,* 1993; Söderman *et al.,* 1994; Söderman *et al.,* 1996, Chan et *al.,* 1998; Lee & Chun, 1998; Söderman *et al.,* 1999a and 1999b; Gago *et al.,* 2002; Henriksson et *al.,* 2005). Studies in which HD-Zip I and II genes were overexpressed in transgenic plants further support the proposed role of this protein family as developmental regulators that are responsive to environmental conditions (Schena *et al.,* 1993; Manavella *et al.,* 2006; Manavella *et al.*, 2008; Ariel *et al*., 2007; Cabello et *al.,* 2007, Dezar *et al.,* 2005a). There remains the need to identify and characterize such proteins in a way that beneficial traits can be conferred on plants utilizing specific members of this class of molecules.

HAHB1 cDNA was isolated in 1992 from a sunflower stem cDNA library and its sequence was deposited in the Genebank (accession number L22847, see SEQ. ID. NO:2: herein for the nucleic acid sequence and SEQ ID. NO:5: herein for the translated protein sequence) and the cloning of the cDNA was described (Chan RL, González DH, 1994). The protein encoded by this gene has been referenced in the literature as a gene homologous to HD-Zip proteins from other species, but this conclusion is based only on comparison of the sequence in phylogenetic ,trees (Gonzalez et al 1997, Chan et al, 1998 and Ariel et al, 2007).

The present invention surprisingly demonstrates the utility of HAHB1 *(Helianthus Annuus Homeobox* 1)*,* in the production of transgenic plants with enhanced tolerance to stress conditions.

### SUMMARY OF THE INVENTION

In the present patent disclosure, we describe the use of HAHB1, a transcription factor that is a member of the sunflower subfamily I of HD-Zip proteins and variants thereof, such as ATHB13, to modify plant responses to stress conditions, including freezing, drought, salinity and biotic stress. Thus, the disclosure describes plants and method which confer or increase such stress tolerance. The gene was isolated from a genomic library and its expression pattern characterized. We demonstrate that by making transgenic plants bearing the sunflower *HAHB1* cDNA under the control of either the constitutive 35S promoter or the native HAHB1 promoter, transgenic plants are produced which exhibit clear increases in tolerance to low temperature conditions in the vegetative and reproductive stages. In addition, the transgenic plants exhibit better tolerance than non-transformed plants in response to drought or salinity conditions. Similar effects are observed when using the HAHB1 homologue ATHB13. Microarray analyses were performed to assess expression patterns in transgenic Arabidopsis plants. The data indicates that the observed tolerance occurs via the increase of antifreeze proteins localized in the cellular apoplast which inhibit the growth of large extracellular ice crystals. Moreover, the inventors have also shown that the transient overexpression of the *HAHB1* gene in sunflower induces the expression of several genes related to stress tolerance.

*HAHB1* is a member of the HD-Zip subfamily I (HD-Zip I). All HD-Zip I family members show high sequence similarity in the N-terminal homeodomain (HD) and leucine zipper domain (LZ), but are notably much more diverse in the C-terminal region. For example, HAHB1 and HAHB4 are both members of HD-Zip I, but they share very little sequence similarity in their C-terminal regions. Although HD-Zip I are grouped into a single family, different HD-Zip I family members exhibit differential expression patterns and are involved in different developmental and physiological processes as detailed below. The inventors have characterised HAHB1 and compared its structure to homologous sequences. Using chimeric constructs, the inventors have also found that it is the C-terminus of the HAHB1 protein that is important in conferring HAHB1 function. Genes homologous to *HAHB1* that show high homology not only in the HD and LZ domains, but also in the C-terminal domains, are predicted to have a similar effect as *HAHB1* when expressed in transgenic plants, as shown herein for *ATHB13.*

From the information provided herein, those skilled in the art will appreciate that *HAHB1,* a part or a homolog thereof, confers enhanced tolerance, for example, freezing tolerance to transgenic plants if it is expressed, either under the control of its own promoter or under the control of a constitutive promoter such as the CaMV 35S promoter, or under the control of another type of promoter, such as for example a cold inducible promoter or an abiotic stress inducible promoter.

This freezing tolerance is accompanied by enhanced tolerance to drought and high salt conditions. We also show that not only can *HAHB1* be expressed under the control of the *HAHB1* promoter to achieve stress-responsive expression, but any gene sequence may be operatively associated with the *HAHB1* promoter in order to achieve expression under cold or freezing conditions, high salt or low water or pathogen invasion.

As stated above, without wishing to be bound by any particular theory, the tolerance to freezing conditions conferred by expression of *HAHB1* is apparently due to the synthesis and action of antifreeze proteins in the cellular apoplast inhibiting ice crystal formation with its subsequent dehydration effects. It also is possible that other proteins outside the apoplast (inside the cell) are also up translated.

In light of the general information provided herein, those skilled in the art will appreciate that this disclosure describes and enables those skilled in the art to obtain and isolate a gene sequence from sunflower which can be used to confer enhanced tolerance to stress conditions including enhanced chilling and freezing tolerance, enhanced tolerance to drought, enhanced tolerance to conditions of high salinity and/or biotic stress. In addition, using methods known in the art, the isolated gene sequence from sunflower disclosed herein or parts thereof or can be used to isolate related sequences from other plants which can be used to confer enhanced tolerance to abiotic and biotic stress conditions as described herein.

The invention relates to a method for increasing tolerance to all of drought, high salinity and freezing comprising transforming a plant with a nucleic acid comprising a sequence of SEQ ID No. 6 or 7 or a sequence with at least 90% or at least 95% homology to a sequence comprising SEQ ID No. 6 or 7. The invention also relates to an isolated chimeric nucleic acid construct comprising a nucleic acid sequence encoding for the N-terminal sequence of another HD Zip protein of subfamily I operatively associated with a nucleic acid sequence encoding for a sequence comprising the C-terminus of HAHB1 as defined in SEQ ID NO. 8. The invention also relates to a polypeptide encoded by a gene construct encoded by such a construct. The invention also relates to a method for conferring tolerance in a plant to all of drought, high salinity and freezing which comprises introducing and expressing in a plant a gene construct a polypeptide as defined above. The invention further relates to use of a nucleic acid sequence of SEQ ID No. 6 or 7, or a sequence with at least 90% or at least 95% homology to SEQ ID No. 6 or 7 or use of a vector comprising a nucleic acid sequence of SEQ ID No. 6 or 7 or a sequence with at least 90% or at least 95% homology to SEQ ID No. 6 or 7 in conferring increased tolerance to all of drought high salinity and freezing. Accordingly, we describe an isolated protein or gene sequence from sunflower, *HAHB1,* a part or variant thereof, which can be used to confer enhanced tolerance to abiotic and biotic stress conditions as described herein, even in plant species other than sunflower.

Also described is an isolated gene sequence from sunflower, *HAHB1,* a part or variant thereof, which can be used to isolate related sequences from other plants, including plant species not related to sunflower, which sequences can be used to confer enhanced tolerance to abiotic and biotic stress conditions as described herein.

Further described is an isolated promoter gene sequence which can be used to regulate expression of sequences operatively associated with the promoter sequence to confer on the thus associated sequences the property of expression in response to abiotic and biotic stress conditions as described herein.

We also describe expression constructs or vectors comprising nucleic acid sequences described herein which confer on plants into which such expression constructs or vectors are introduced enhanced tolerance to abiotic and biotic stress conditions as described herein.

The disclosure also relates to transgenic plants which have enhanced or increased tolerance to abiotic and biotic stress conditions as described herein.

The disclosure also relates to compositions and methods to induce production of antifreeze proteins (AFPs) in a plant to thereby avoid or minimize damage otherwise caused in plants on exposure to low temperature conditions as a result of ice crystal formation.

The disclosure further relates to novel methods for identifying and using novel compositions disclosed herein to confer enhanced tolerance to abiotic and biotic stress conditions as described herein.

Thus, based on the information provided herein, those skilled in the art will be able to prepare expression constructs or vectors comprising nucleic acid sequences which confer on plants into which such expression constructs or vectors are introduced enhanced tolerance to abiotic and biotic stress conditions as described herein. This will enable those skilled in the art to produce plants which have enhanced tolerance to abiotic and biotic stress conditions as described herein.

Without wishing to be bound by theory, those skilled in the art will appreciate that the compositions and methods provided herein will permit induction of production of antifreeze proteins (AFPs) in a plant to thereby avoid or minimize damage otherwise caused in plants on exposure to low temperature conditions as a result of ice crystal formation.

Other objects, advantages and benefits of this invention will be apparent to those skilled in the art from a review of the complete disclosure provided herein and the appended claims.

### BRIEF DESCRIPTION OF THE DRAWINGS

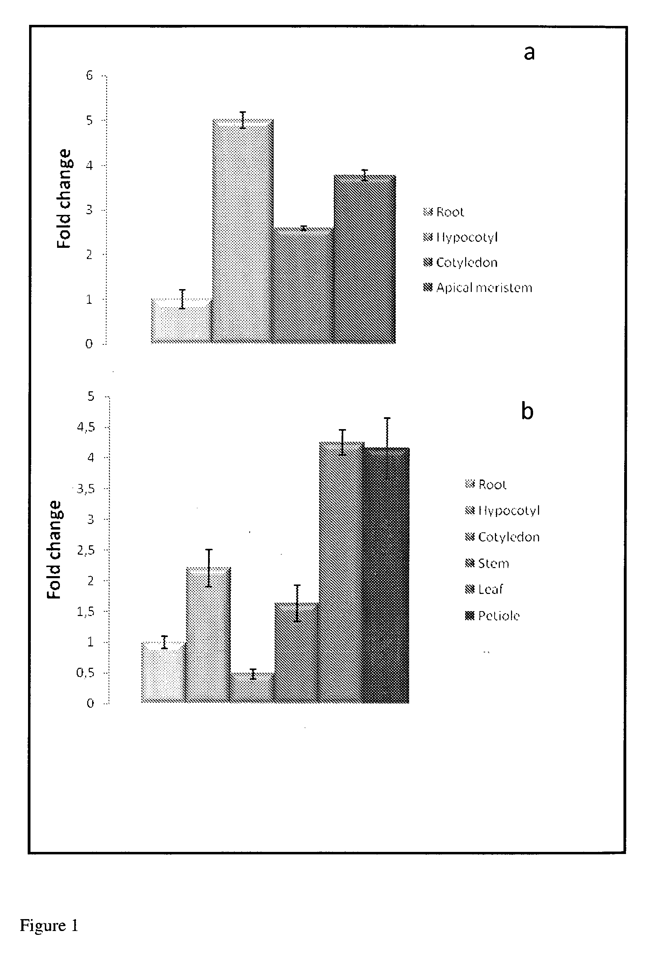

### Figure 1. HAHB1 expression pattern in seedlings and mature plants

a: Transcript levels of *HAHB1* in different tissues and organs of sunflower 7-day-old seedlings (from left to right: root, hypocotyl, cotelydon, apical meristem); b: Transcript levels of *HAHB1* quantified in different tissues and organs of sunflower 21-day-old plants (from left to right: root, hypocotyl, cotelydon, stem, leaf, petiole); transcript levels were determined by quantitative RT-PCR and standard deviations calculated from three independent samples in which actin transcripts *(ACTIN2* plus *ACTIN8)* were used as internal controls; all the values are normalized with respect to the value measured in roots, arbitrarily assigned a value of one. The y axis indicates the (x fold) change observed.

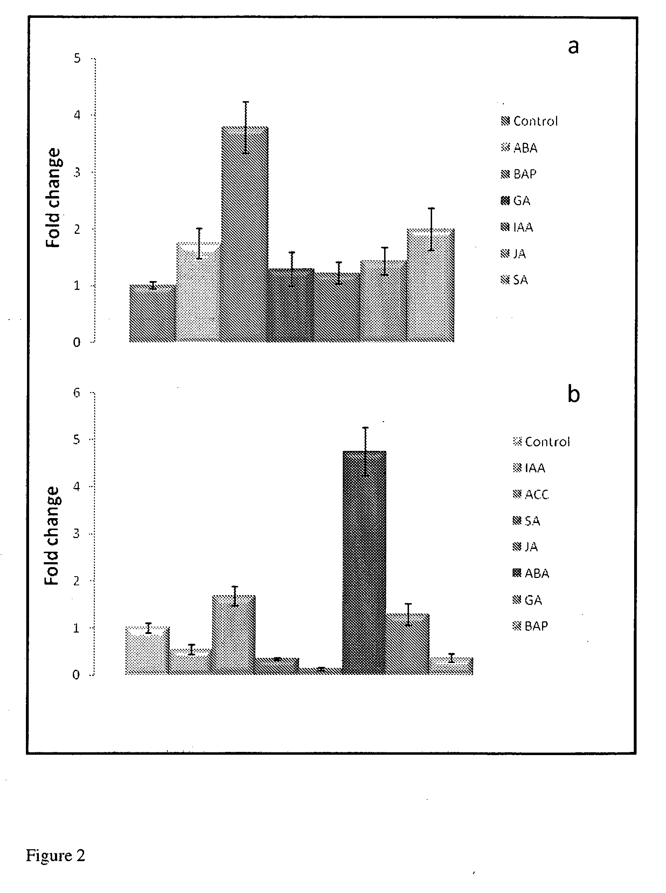

### Figure 2. Sunflower HAHB1 is up-regulated by cytokines in seedlings and by ABA in mature plants

a: Transcript levels of *HAHB1* in 7-day-old seedlings two hours after treatments with different hormones (from left to right:control, ABA, BAP, GA, IAA, JA, SA); b: Transcript levels of *HAHB1* in 21-day-old leaves disks two hours after treatments with different hormones (from left to right: control, IAA, ACC, SA, JA, ABA, GA, BAP); transcript levels were determined by quantitative RT-PCR and standard deviations calculated from three independent samples in which actin transcripts *(ACTIN2* plus *ACTIN8)* were used as internal controls; all the values are normalized with respect to the value measured in roots, arbitrarily assigned a value of one. The y axis indicates the (x fold) change observed.

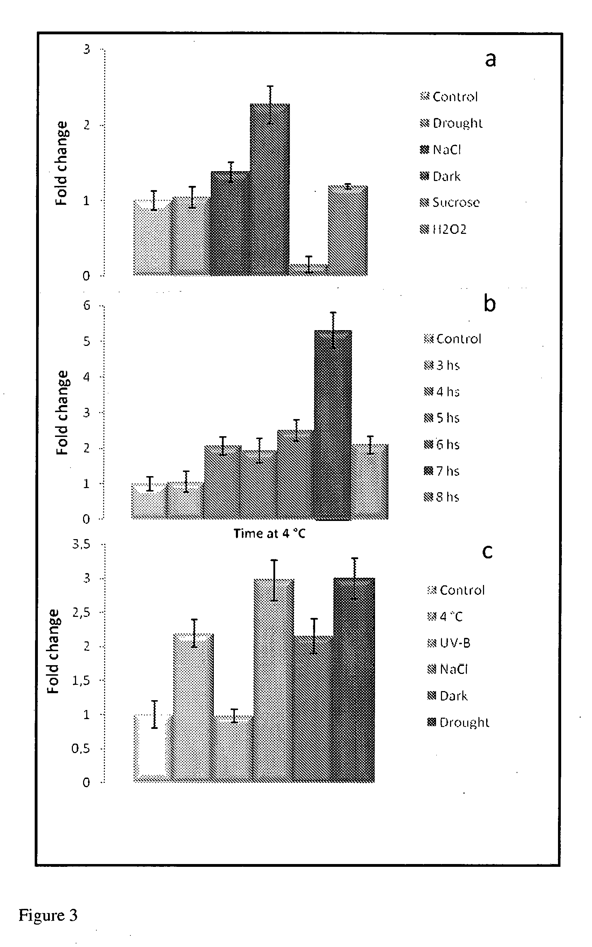

### Figure 3. Sunflower HAHB1 is up-regulated by abiotic stress factors

a: Transcript levels of *HAHB1* in 7-day-old seedlings two hours after treatment with abiotic stress factors as stated in the figure and detailed in the Experimental Procedures section (from left to right: control, drought, NaCl, dark, sucrose, H₂O₂) b: Kinetics of induction of *HAHB1* in 7-day-old seedlings placed at 4 °C (from left to right: control, 3, 4, 5, 6, 7, 8hs); c: Transcript levels of *HAHB1* in 21-day-old leaves disks two hours after treatments with abiotic stress factors as stated in the figure (from left to right: control, 4°C, UV-B, NaCl, dark, drought); transcript levels were determined by quantitative RT-PCR and standard deviations calculated from three independent samples in which actin transcripts *(ACTIN2* plus *ACTIN8)* were used as internal controls; all the values are normalized with respect to the value measured in roots, arbitrarily assigned a value of one. The y axis indicates the (x fold) change observed.

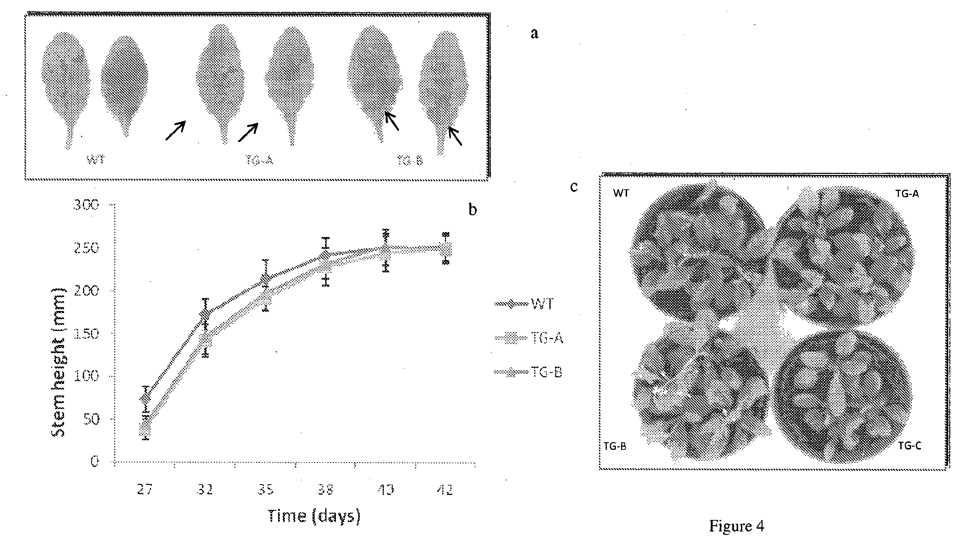

### Figure 4. Morphological and developmental characteristics of transgenic plants bearing the construct 35S:HAHH1

a: 21-old-day leaves of transgenic and WT plants. Transgenic plants exhibit serrated leaves while WT plants do not. The arrows designate the serrated borders of the transgenic plants. b: Stem height during the life cycle of transgenic and WT plants; c: 30-day-old plants cultured in normal growth conditions. Figure 4a shows the difference in the leaf morphology between transgenic and wild type plants. Transformed plants show serrated borders and a differentiated shape in comparison with their non-transformed counterparts (Figure 4a and 4c). In 4c, transgenic (35S:HAHB1; three independent lines A, B and C) and WT plants show similar developmental characteristics. The plants are healthy in normal growth conditions.

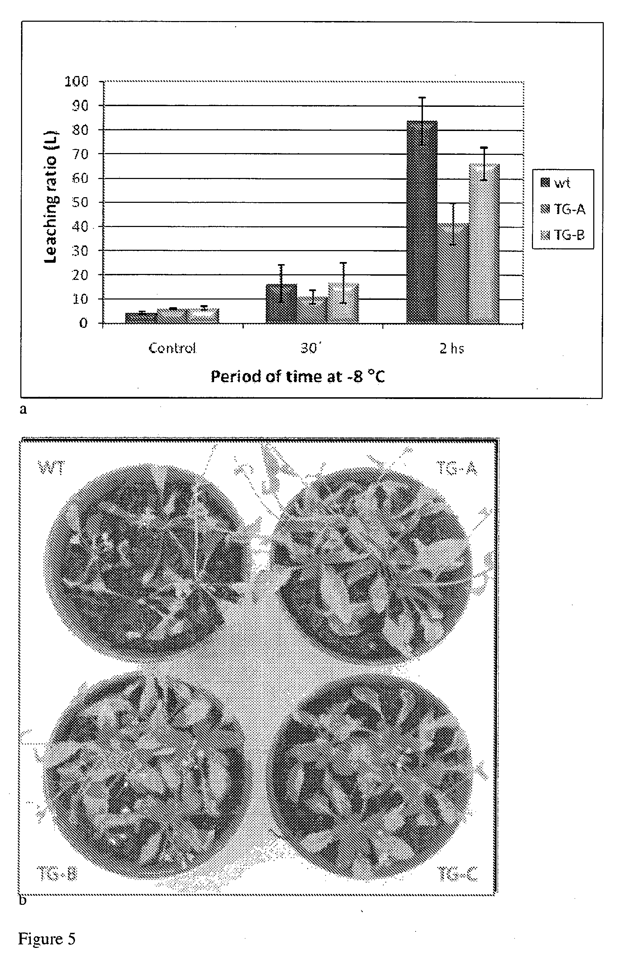

### Figure 5. Transgenic plants ectopically and constitutively expressing 35S:HAHB1 are more tolerant to freezing conditions

a: Membrane stability of transgenic and non-transformed plants measured as the conductivity of the supernatant solution (see Experimental Procedures, inverse to stability) after freezing treatments; b: Photograph taken 6 days after placing the plants to recover in normal growth conditions after a treatment of 8 hours in freezing conditions: WT plants are dead after a freezing treatment while transgenic plants (35S:HAHB1; three independent lines TG (transgenic) A, TG-B and TG-C) show a lesser extent of damage. Approximately, 25% of the leaves (TG genotypes) are senescent while the others are green and healthy. The x axis shows the period of time at -8°C and the y axis shows the leaching ratio (L). Percentage of survivors of all genotypes (transgenic and non-transformed) subjected to freezing conditions as in b:

| Genotype | Survivors % |
|---|---|
| Wt | 22+-3 |
| TG-A | 85+-2 |
| TG-B | 74+-4 |
| TG-C | 70+-3 |
| TG-D | 65+-2 |
| TG-E | 60+-5 |

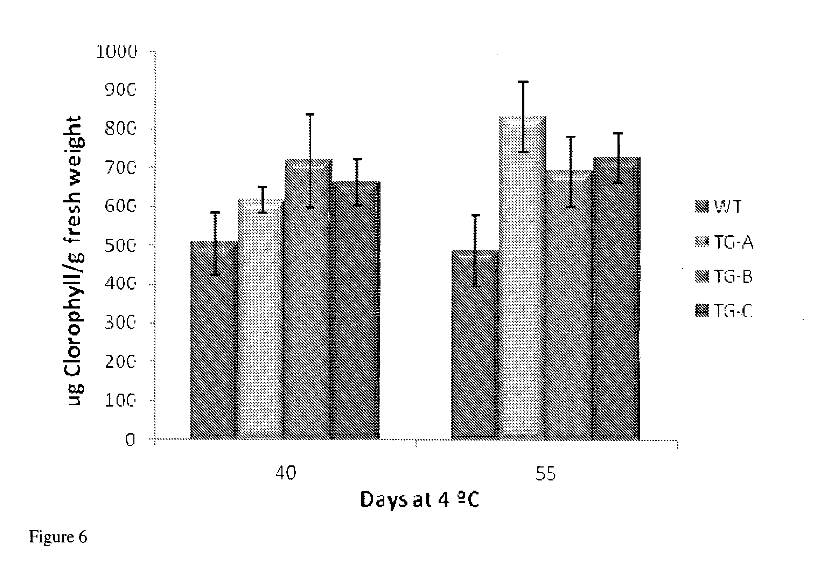

### Figure 6. Chlorophyll content in plants subjected to chilling conditions

Chlorophyll content was measured as described in Experimental Procedures in transgenic plants expressing *HARB1* and wild type plants subjected to chilling temperatures for the periods indicated. The values on the y axis are expressed as µg of chlorophyll per g of fresh leaves. The x axis shows the days at 4°C.

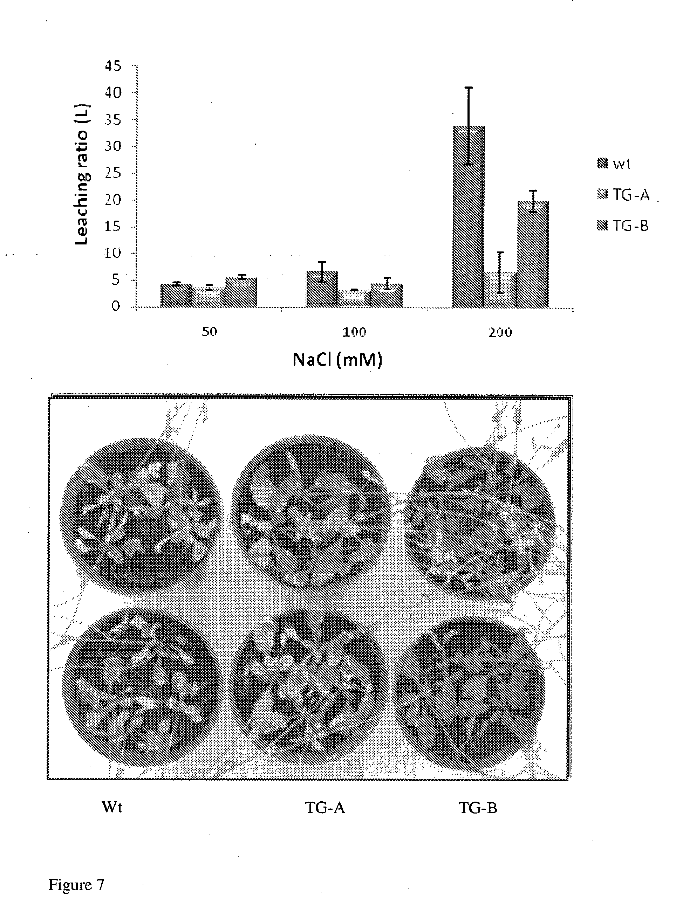

### Figure 7. Transgenic plants expressing HAHB1 are more tolerant to salt stress

a: Membrane stability of transgenic (TG-A and TG-B) plants expressing *HAHB1* and non transformed plants (wt) measured as the conductivity of the supernatant solution (see Experimental Procedures, inverse to stability) after salt stress treatments; concentrations of NaCl are expressed in mM on the x axis; b: Illustrative photograph taken 2 days after watering with 200 mM NaCl. WT plants show approximately 50% of dead leaves (lighter coloured leaves) while transgenic plants exhibit more than 80% and 100% healthy green leaves for line A and B respectively. Some (but not all) dead leaves are marked with an arrow.

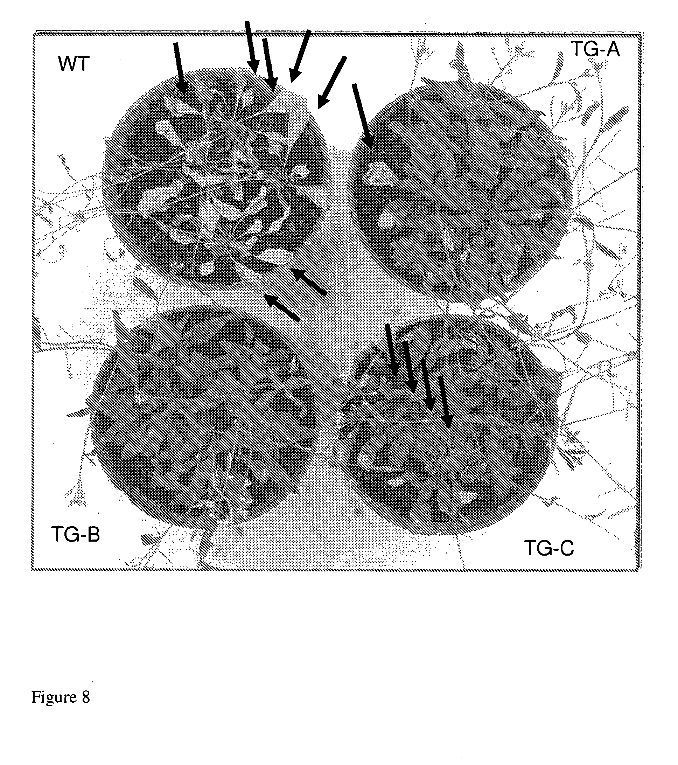

### Figure 8. Transgenic plants expressing HAHB1 are more tolerant to drought

Illustrative photograph taken 2 days after watering of transgenic and wild type plants subjected to drought as described in the Experimental Procedures section. Approximately 75% of the WT plants are dead as can be appreciated by the senescent leaves (lighter colour); while 25% are damaged, but present few green leaves. Transgenic plants present a different number of dead leaves (20% for line A, 50% for lines B and C) and younger leaves are green and healthy.

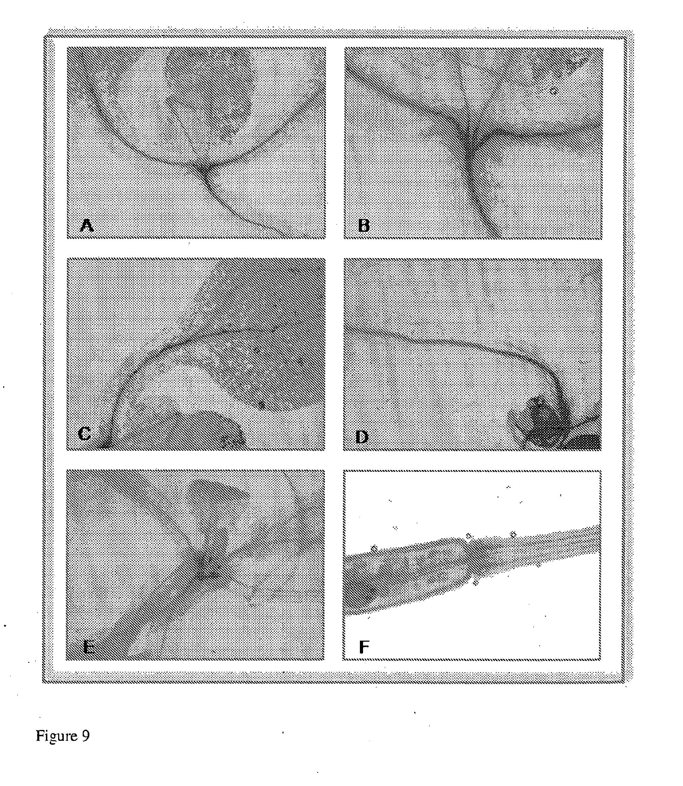

### Figure 9. Expression pattern of the GUS reporter gene directed by the HAHB1 promoter region.

Histochemical detection of GUS enzymatic activity in *Arabidopsis* transgenic plants transformed with *prHAHB1:GUS.* a, b, c and d: 14-day-old seedlings; e, 30 day-old plants and f, 45 day-old plant; view of meristems (a and b); cotyledons **(c),** hypocotyls **(d),** apical meristem **(e)** and siliques **(f).** GUS activity due to the expression of the gene directed by HAHB1 promoter (darker areas) is clearly visualized in the vascular system of hypocotyls, cotyledons and leaves (A-D) as well as in the meristematic region (E) and in the siliques base (F).

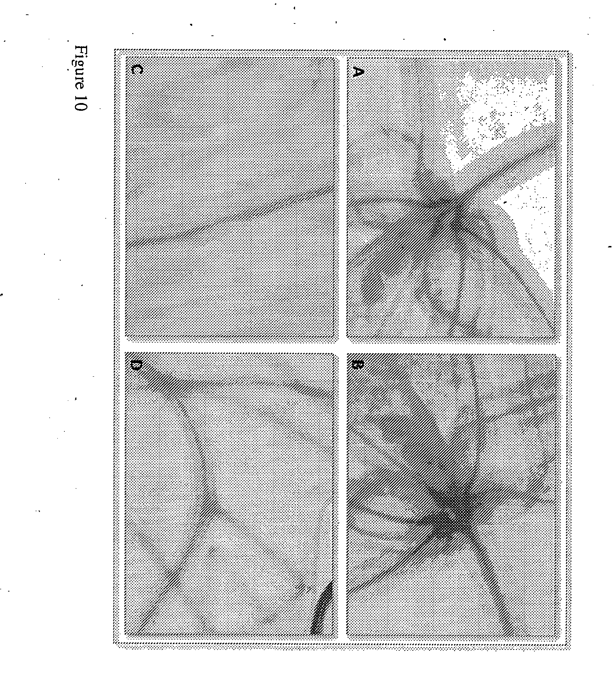

### Figure 10. Expression pattern of the GUS reporter gene directed by the HAHB1 promoter region in plants subjected to chilling conditions.

Histochemical detection of GUS enzymatic activity in *Arabidopsis* transgenic plants transformed with *prHAHB1:GUS* after chilling conditions; **a** and **b,** apical meristems; **c** and **d,** roots; the plants used are 30-days-old. GUS activity due to the expression of the gene directed by HAHB1 promoter (darker areas) is clearly visualized in the meristematic region (A and B) and the vascular system of roots (C and D).

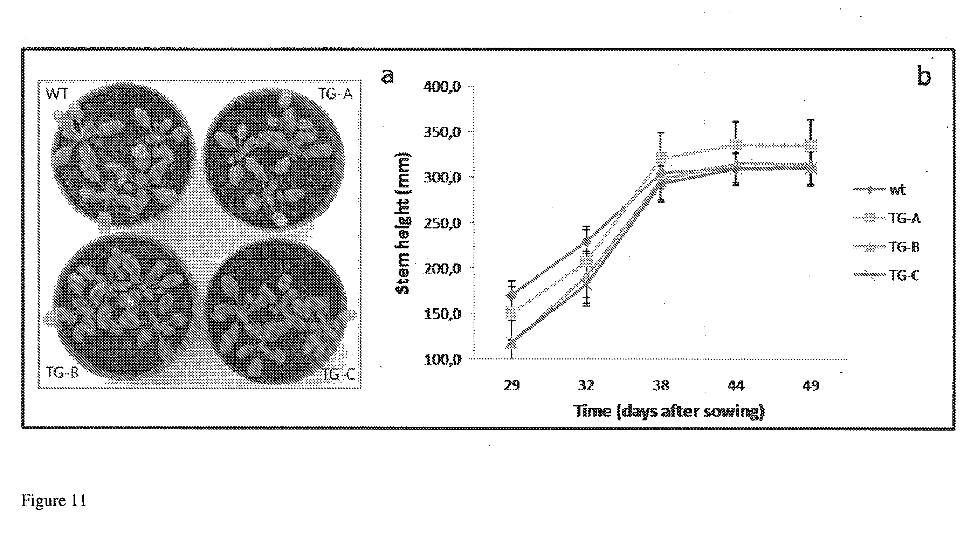

### Figure 11. Morphological and developmental characteristics of transgenic plants bearing the construct promXAHB1:XAHB1

a: 25-day-old plants (wt and transgenic plants expressing *HAHB1)* cultured in normal growth conditions. Plants (WT and three independent transgenic genotypes) exhibit indistinguishable phenotypes in normal growth conditions. b: Stem height (y axis) during the life cycle of transgenic and WT plants.

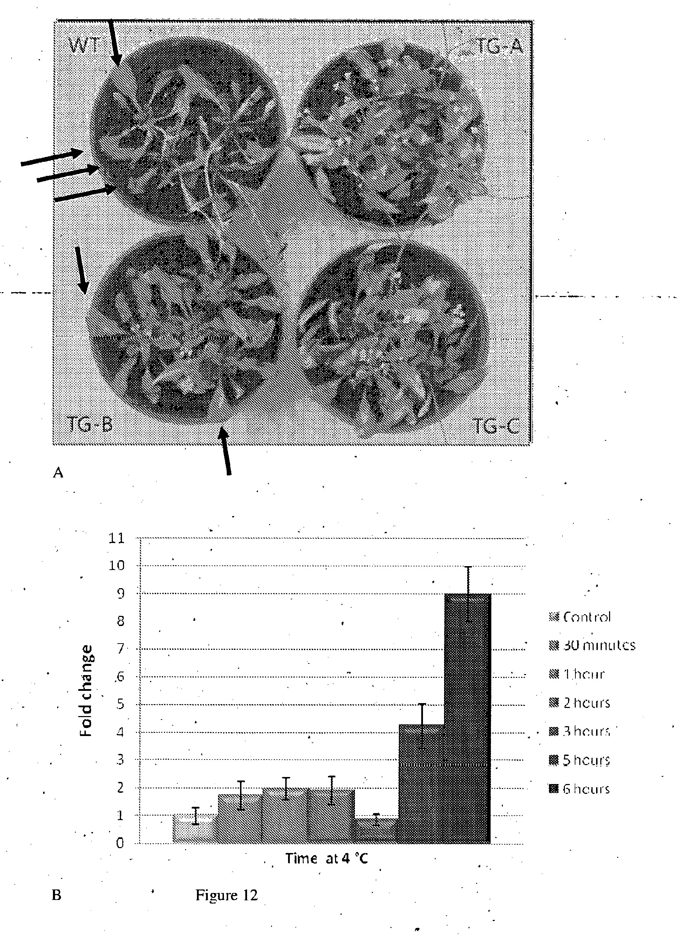

### Figure 12. Transgenic plants expressing HAHB1 under the control of its own promoter are more tolerant to freezing conditions

a: Photograph taken 6 days after placing plants (wt and transgenic plants expressing *HARB1,* previously grown during 8 hours at -8 °C) to recover in normal growth conditions; TG-A, TG-B and TG-C represent plants from three independent transgenic genotypes (construct *promHAHB1:HAHB1).* WT plants are senescent and almost dead (some, but not all, dead leaves are marked with an arrow) while transgenic plants exhibit certain damage in old leaves, but a healthy aspect in younger ones. b: *HAHB1* transcript levels in transgenic plants bearing the construct *promHAHB1:HAHB1* as a function of the incubation time at 4 °C with the y axis representing the x fold change; transcript levels were determined by quantitative RT-PCR and standard deviations calculated from three independent samples in which actin transcripts *(ACTIN2* plus *ACTIN8)* were used as internal controls; all the values are normalized with the value measured in untreated plants (time 0), arbitrarily assigned a value of one. Percentage of survivors from the four genotypes after the freezing/recovery treatment:

| Genotype | Survivors % |
|---|---|
| Wt | 38+-5 |
| TG-A | 90+-8 |
| TG-B | 88+-6 |
| TG-C | 75+-10 |

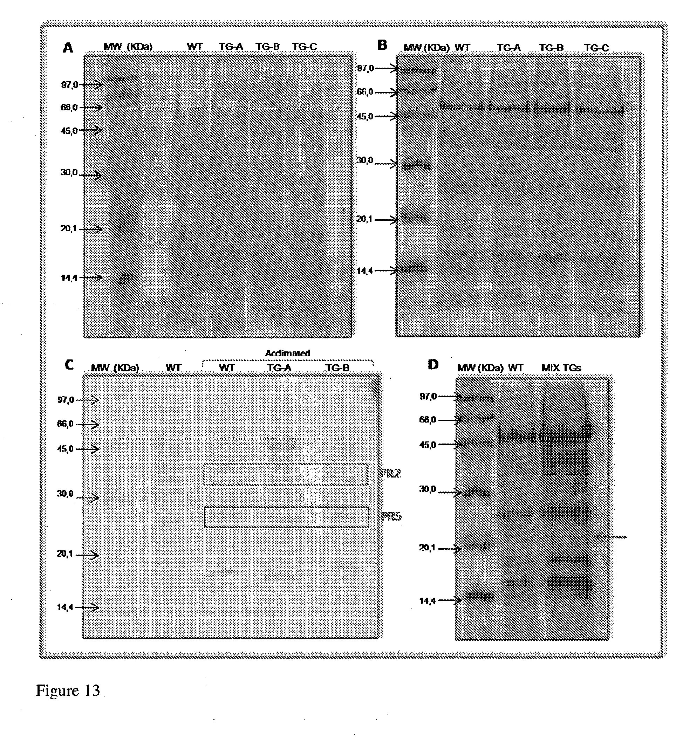

### Figure 13. Apoplastic proteins of transgenic (35S:HAHB1) and wild type plants

SDS-PAGE showing the proteins present in the cellular apoplast, obtained from non-acclimated plants (A), plants acclimated during 16 hours at 4°C (B), plants acclimated during 10 days at 4 °C (C) and plants placed during 3 hours at -8 °C (D, freezing treatment). All the samples were isolated from 25-day-old WT or transgenic plants (three independent lines of *35S:HAHB1* (A, B and C) or a mix of them (D). Samples loaded in A, B and C were obtained from 7 g leaf tissue and an equal volume extract was loaded in the gel. Samples loaded in D were obtained from 3 g leaf tissue and equal volumes loaded in the gel. The arrow indicates the band differentially expressed.

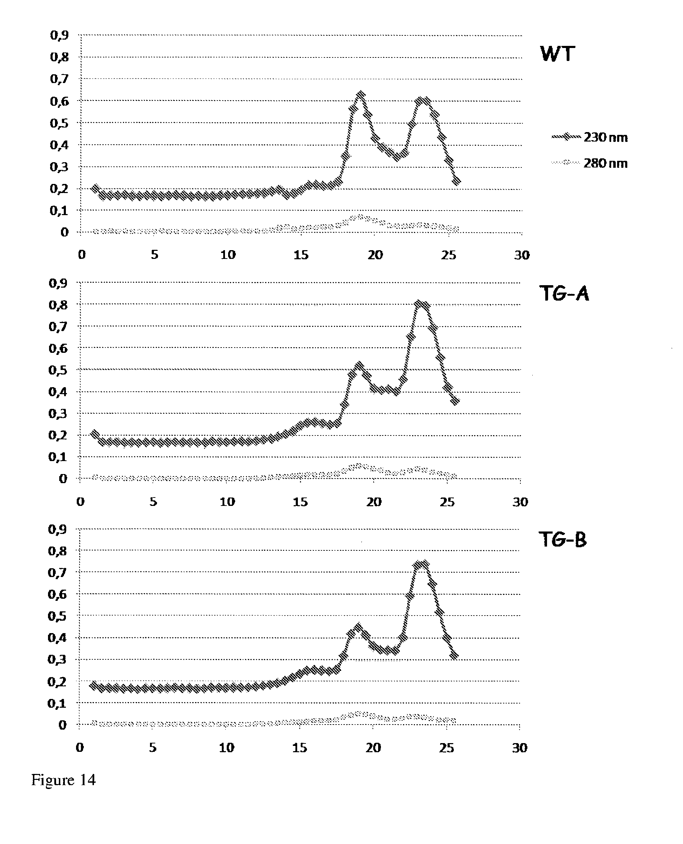

### Figure 14. Chromatogram of apoplastic proteins isolated from acclimated transgenic and wild type plants

Sephadex G-200 column elution chromatogram (see Experimental Procedures) of apoplastic proteins purified from both genotypes (transgenic, two independent lines expressing *HAHB1:* TG-A and TG-B, or wild type); the second peak in the transgenic apoplastic proteins increased to 0.8-0.9 OD compared with the WT extract in which this peak reaches 0.5-0.6 OD.

### Figure 15. Expression levels of antifreeze proteins in transgenic and wild type plants subjected to different stress treatments.

PR2 (15c and f), PR3 (15b and e) and PR4 (15a and d) expression levels in plants (control and two independent transgenic lines expressing *HAHB1:* TG-A and TG-B) subjected to drought (EH), 4°C or treated with ACC (ethylene precursor), salicylic acid (SA) or abscisic acid (ABA).

### Figure 16. HAHB1 prevents the formation of large ice crystals

Apoplastic proteins were extracted and mixed with 26% sucrose and the mixture was frozen at -80°C over a period of a few minutes; after that, the samples were gradually warmed to 0°C and then placed for one hour at -8°C; finally, the samples were observed and photographed with a optical microscope (x4); A, B, C, crystals formed in the presence of apoplastic transgenic proteins (three independent lines from the genotype *35S:HAHB1*)*;* D, crystals formed in the presence of apoplastic WT proteins; E, crystals formed in the presence of 26% sucrose without proteins.

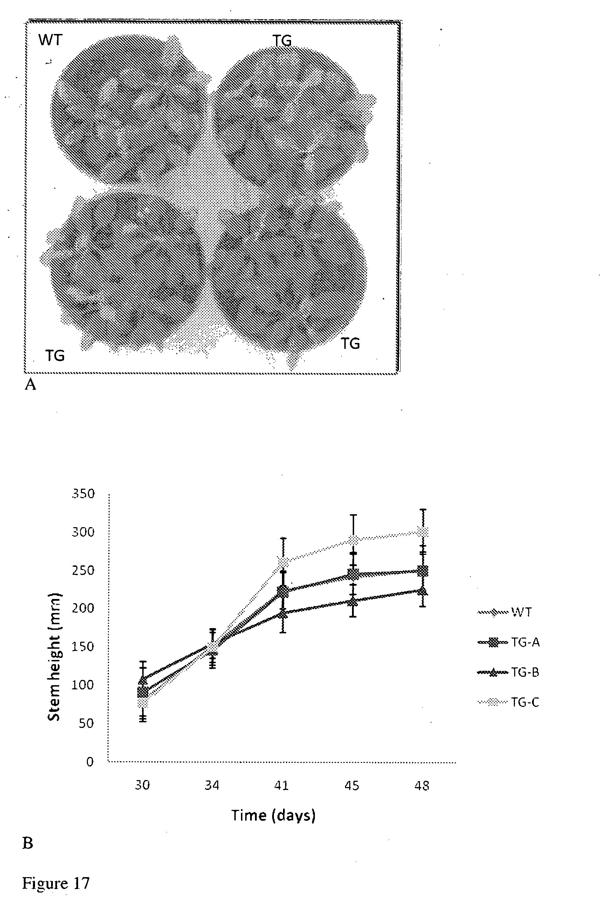

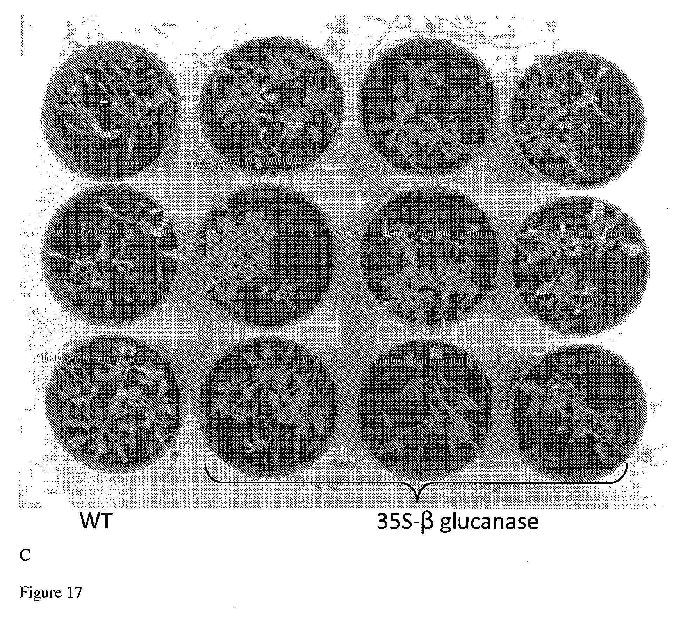

### Figure 17. Transgenic plants bearing the construct 35S:GLUC are more tolerant to freezing conditions.

a: morphological characteristics of transgenic plants expressing *35S:GLUC* and WT plants in normal growth conditions; non-significant differences were detected in the morphological characteristics of five independent F2 lines of transgenic plants (bearing the *GLUC* gene, AT4g16260) compared with control plants; b: stem height of transgenic and WT plants in later developmental stages; c: plants subjected to freezing conditions (7 hrs at -8 °C) and left in normal conditions (22-24 °C) over six days for recovery before taking the photograph; three independent lines were used in the experiment, four plants per pot of 100 g soil (7 x 8 cm). In 17a, WT and three independent transgenic genotypes exhibit indistinguishable phenotypes in normal growth conditions while when they were subjected to stress, all WT plants shown are wilted (17c, dead leaves are of a lighter colour) and the transgenic plants show much improved survival rates. Percentage of survivors of each genotype after the freezing treatment:

| Genotype | Survivors % |
|---|---|
| 101.3 | 8 |
| *35S:GLUC* | 50 |

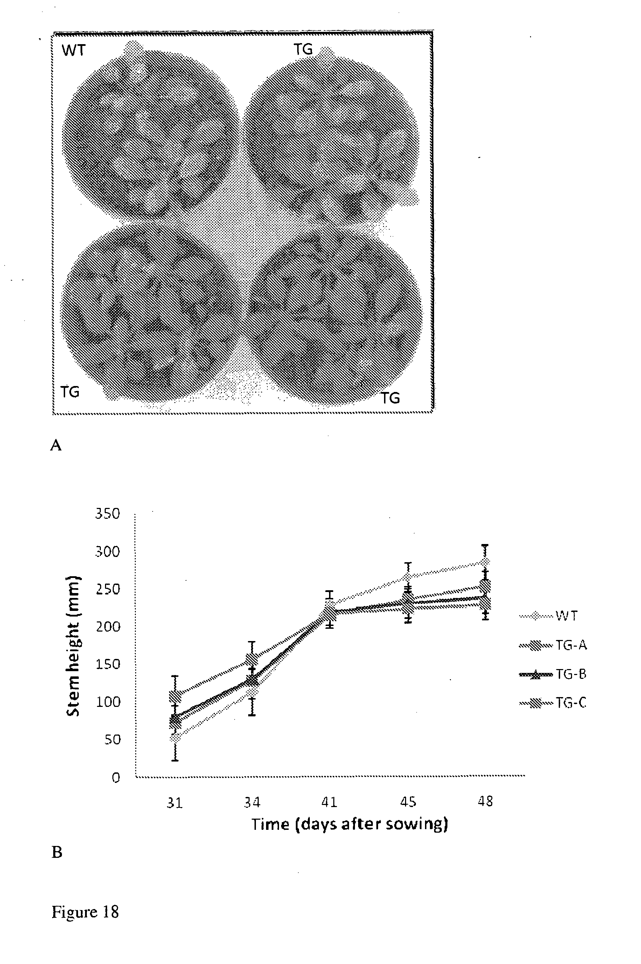

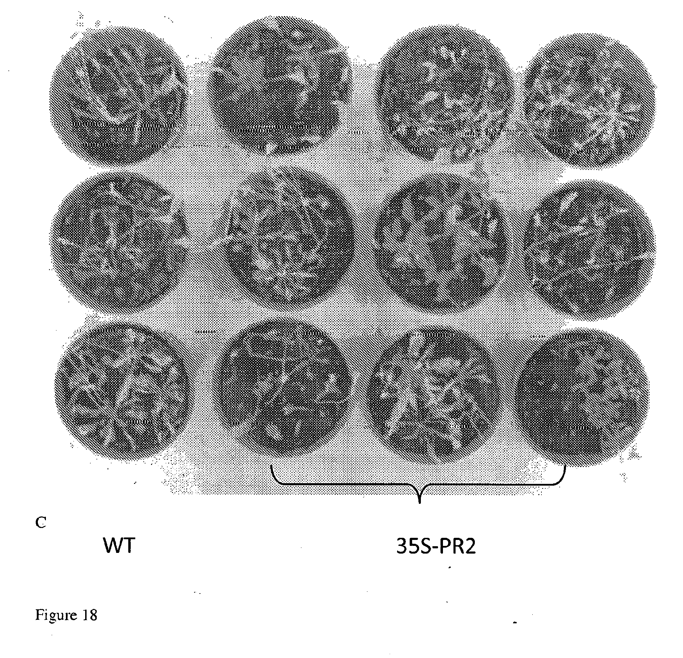

### Figure 18. Transgenic plants bearing the construct 35S:PR2 exhibit differential behaviour in freezing conditions as compared to their WT counterparts

a: morphological characteristics of transgenic and WT plants in normal growth conditions; non-significant differences were detected in the morphological characteristics of five independent F2 lines of transgenic plants (bearing the PR2/glucanase gene/BGL2, AT3g57260) compared with control plants; b: stem height of transgenic and WT plants in later developmental stages; c: plants subjected to freezing conditions (7 hours at -8 °C) and in normal conditions (22-24 °C) over six days of recovery before taking the photograph; three independent lines were used in the experiment, four plants per pot of 100 g soil (7 x 8 cm). In 18a, WT and three independent transgenic genotypes exhibit undistinguishable phenotypes in normal growth conditions while when they were subjected to stress WT plants have wilted (18c) and transgenic plants are also rather damaged as shown in c, but a few ones are still green and healthy.Percentage of survivors of each genotype after the freezing treatment:

| Genotype | Survivors % |
|---|---|
| 101.3 | 8 |
| 35S:PR2 | 17 |

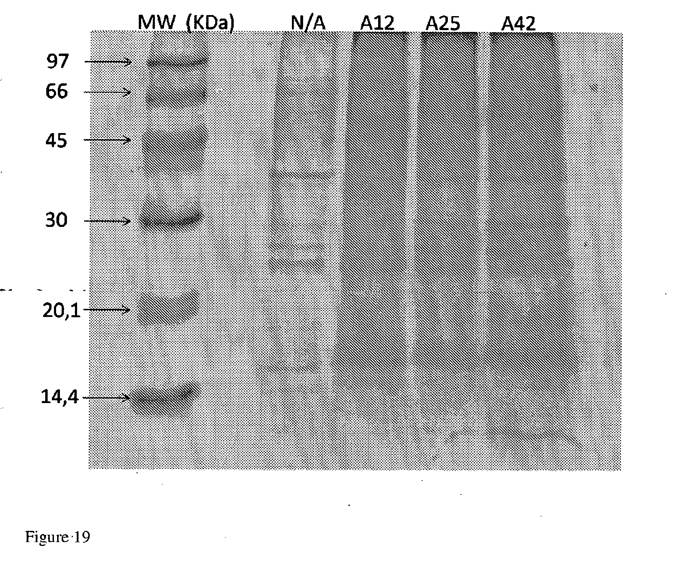

### Figure 19. Sunflower apoplastic proteins are differentially expressed during cold acclimation

SDS-PAGE showing the expression pattern of apoplastic proteins obtained from plants acclimated during at 4°C in 2 week-old sunflower plants; NA: non-acclimated plants; A12, A25 and A42 represent samples taken 12, 25 and 42 days after placing the plants at 4 °C.

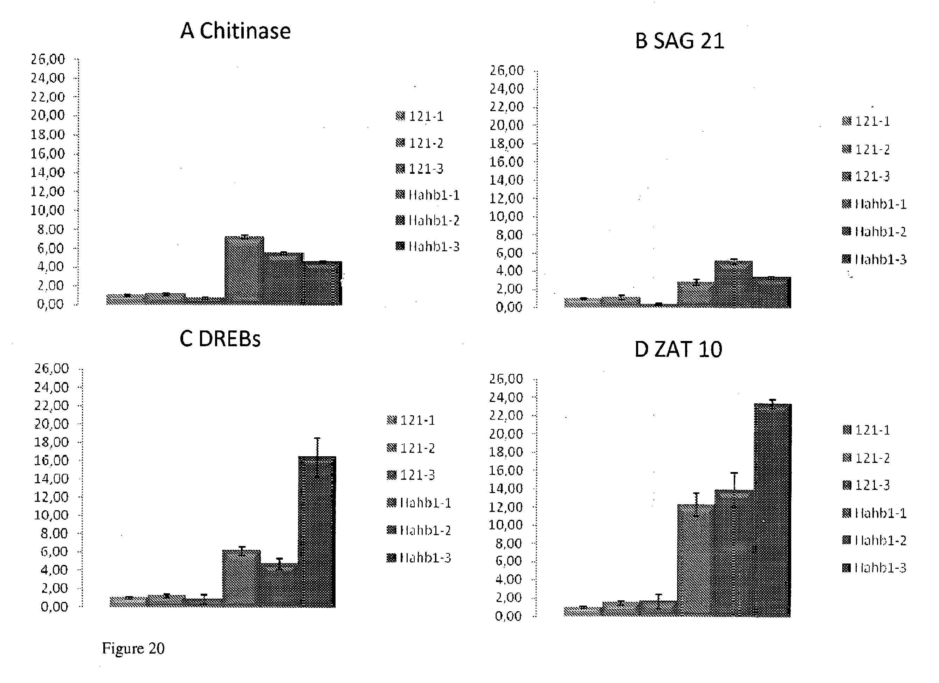

### Figure 20. Sunflower leaf discs transformed with 35S:HAHB1 over-express genes putatively related to the cold response

Sunflower leaves were transformed with an empty vector (121) or *35S:HAHB1* (HAHB1); transcript levels of different response genes were measured by qRT-PCR; *ACTIN* genes *(ACTIN2* plus *ACTIN8)* were used as internal controls and standard deviations were calculated from at least three independent experiments; the measured genes are chitinase (A), SAG21 (B), ZAT10 (D) and DREBs (C) the function of each is described in the Results section.

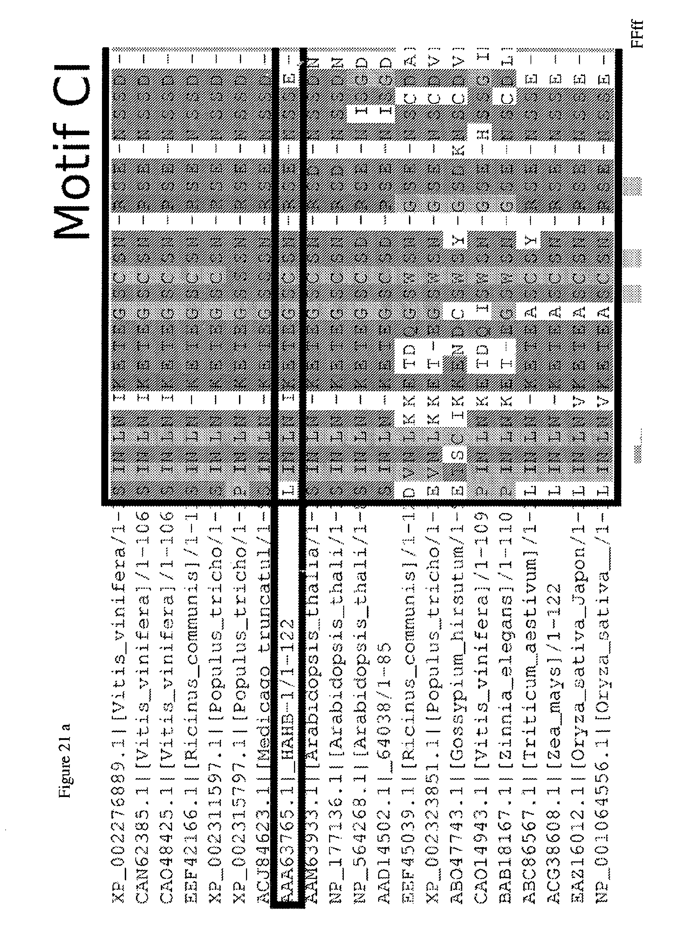

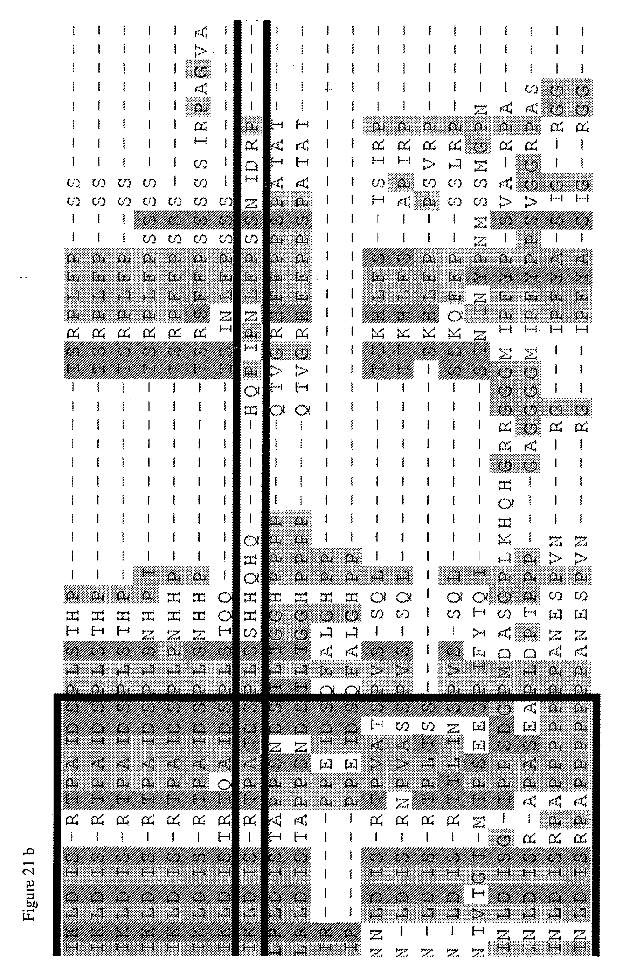

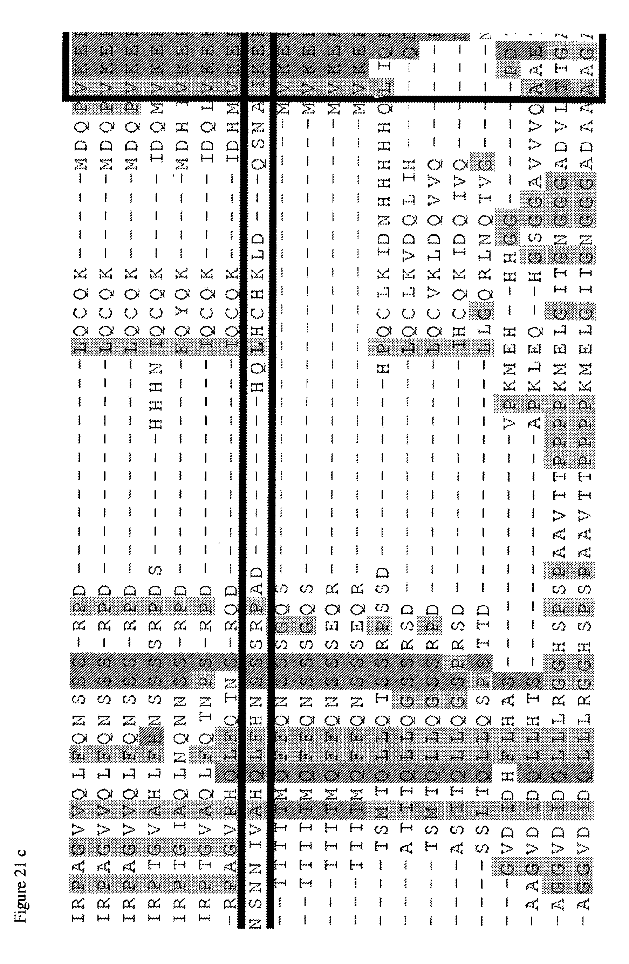

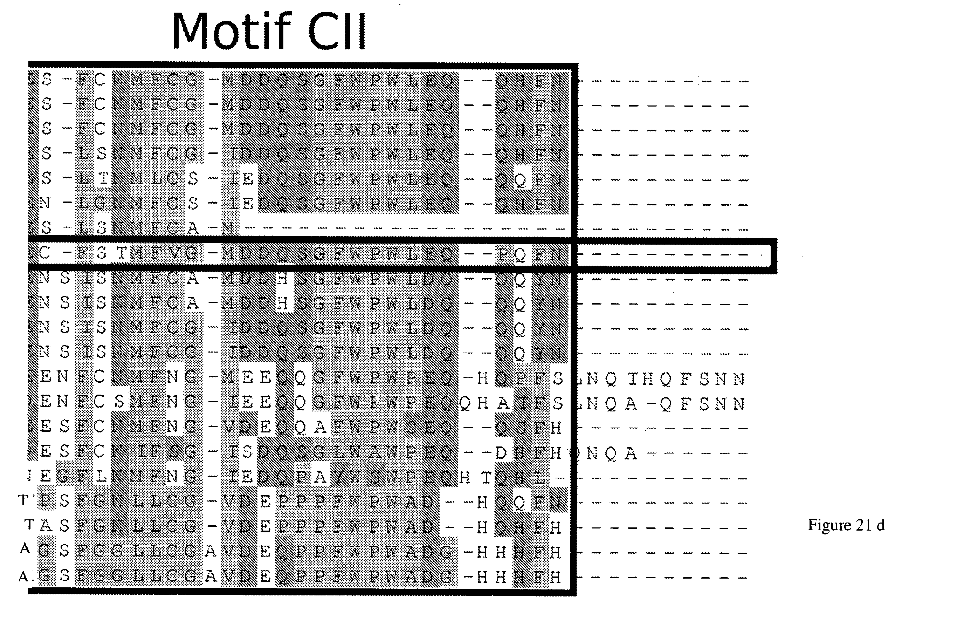

### Figure 21. Comparison of members of the HD-Zip class of proteins

The sequence of HAHB1 carboxy-terminus was compared with the carboxy-termini of the most homologous proteins from other plant species found using the blast algorithm. a) to d) show the C-terminus sequence from the N- to the C-terminal end of the C-terminus. a) shows the CI motif, d) the CII motif. The sequences were aligned using the clustal algorithm (Waterhouse et *al.,* 2009). For proteins which are not anticipated to have similar functions to HAHb1 or ATHB13, notwithstanding the high homology in the HD-Zip regions, there is significant divergence in the C-terminus. However, this comparison of each HD-Zip member also shows some degree of conservation in the carboxy-terminal motifs CI and CII. Most of the homologous proteins are not functionally characterized, but the high homology in the HD-Zip domain allows to classify them as HD-Zip proteins or genes encoding HD-Zip proteins. On the basis of the alignment, it was possible to deduce a consensus sequence as shown herein.

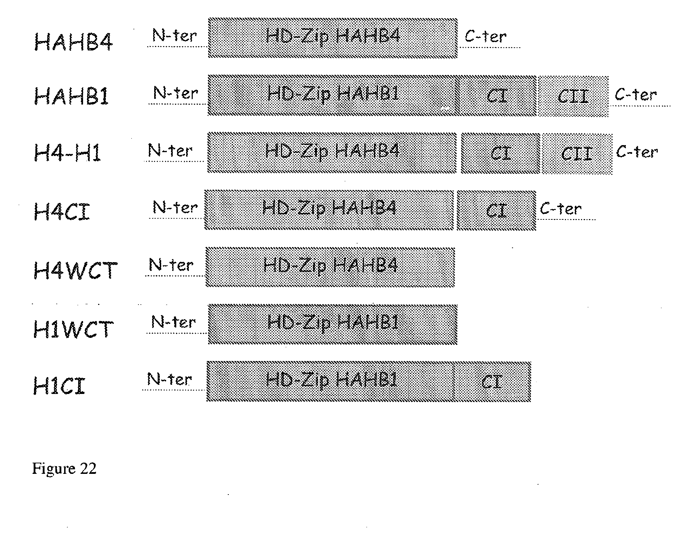

### Figure 22. Schematic of chimeric proteins fusing different HD-Zip domains

Chimerical proteins fusing the HD-Zip domain of one protein with the carboxy-terminal domain of another were used to transform Arabidopsis plants, obtain homozygous lines and analyze phenotypes, especially regarding known characteristics conferred by wt proteins HAHB1, as compared, for example, to the known characteristics and effects of HAHB4. Left side: names of the constructs. CI and CII represent the two carboxy-terminus conserved motifs of HAHB1. C-ter indicates the carboxy-terminus of HAHB4 which is different from *HAHB1* or its homologues.

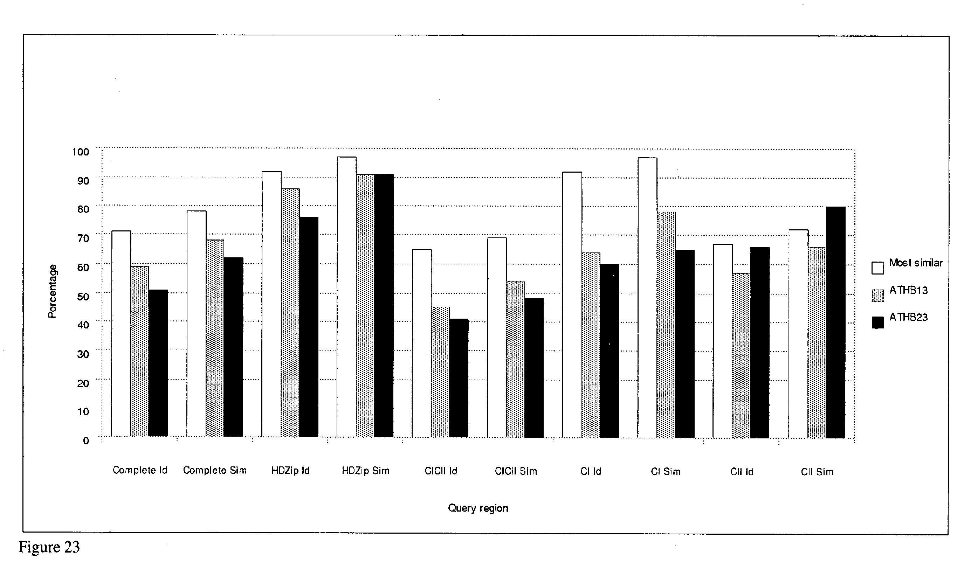

### Figure 23. Alignment of different HAHB1 regions vs non-redundant protein DB(Blastp algorithm)

The bars in the graph represent the % of identity (ID) or similarity (SIM) between certain regions of HAHB1 and the most similar protein in that region (white), ATHB13 (patterned) and ATHB23 (black) as it has been calculated by the Blatp algorithm. The different segments compared are: complete HAHB1 sequence, the HD-Zip domains, the whole COO terminal (CICII), the first motif isolated (CI) and the second motif isolated (CII). The Blastp algorithm calculates these percentages taking the region with highest homology. This is shown in the table below.

| Alignment Summary | | | | | | |
|---|---|---|---|---|---|---|
| | HAHB1 REGION | | | | | |
| | Complete | | HDZip | | CICII | |
| | 313 | | 100 | | 122 | |

| | Identity | Similarity | Identity | Similarity | Identity | Similarity |
|---|---|---|---|---|---|---|
| Most similar | 224/314 (71 %) | 47/314 (78%) | 92/100 (92%) | 97/100 (97%) | 79/121 (65%) | 84/121 (69%) |
| ATHB13 | 192/322 (59%) | 222/322 (68%) | 86/100 (86%) | 91/100 (91%) | 56/124 (45%) | 68/124 (54%) |
| ATHB23 | 162/316 (51 %) | 199/316 (62%) | 76/100 (76%) | 91/100 (91%) | 50/121 (41%) | 59/121 (48%) |

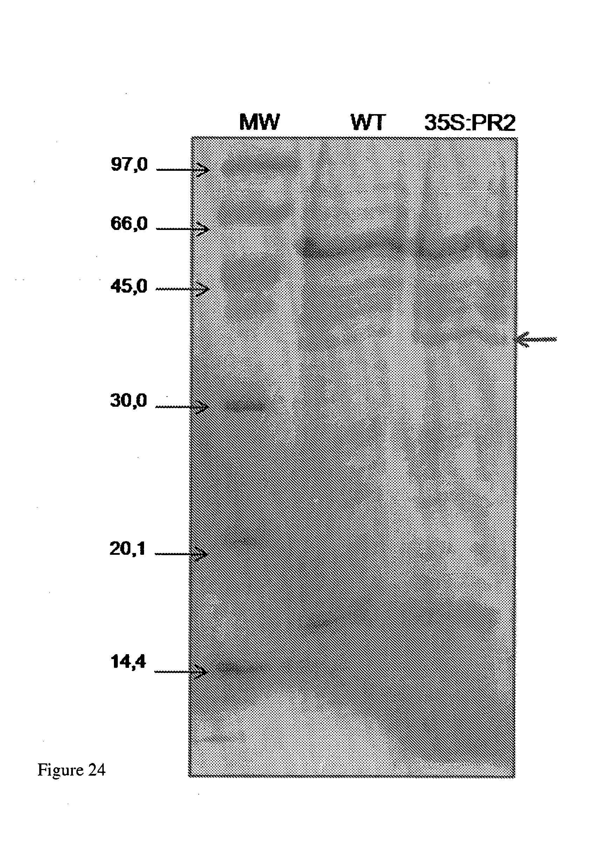

### Figure 24. Apoplastic proteins of transgenic (35S:PR2) and wild type plants

SDS-PAGE showing the apoplastic proteins obtained from non-acclimated WT and transgenic *(35S:PR2)* plants. The samples were isolated from 25-day-old plants grown in normal conditions. The arrow indicates the band differentially expressed. Apoplastic proteins from the WT and transgenic plants grown in normal conditions were isolated and analyzed by SDS-PAGE. As it can be observed, although the plants were not subjected to cold stress, the PR2 expressed is present in the cellular apoplast in the transgenic genotype. The molecular weight of this band is coincident with the expected for PR2, but sequence determination was not carried out yet.

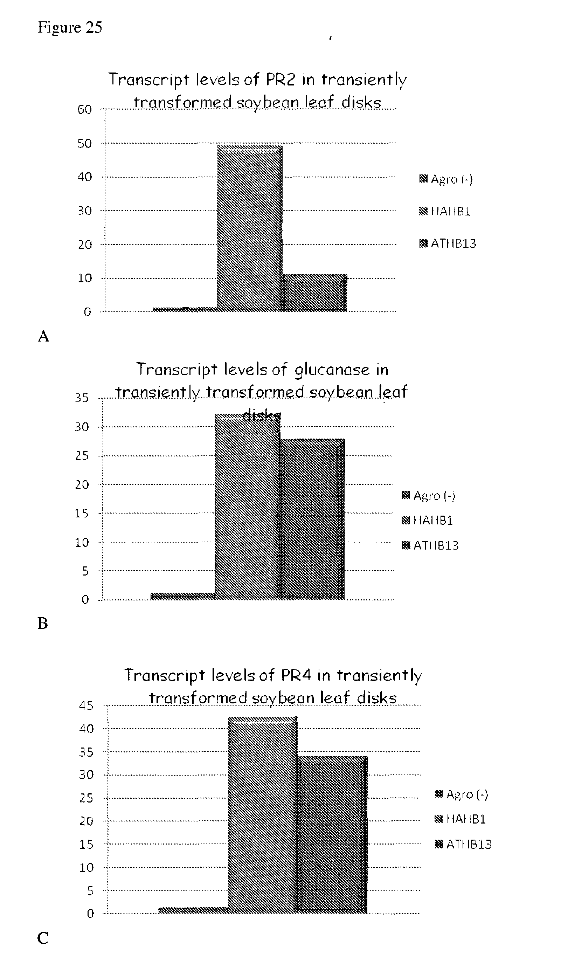

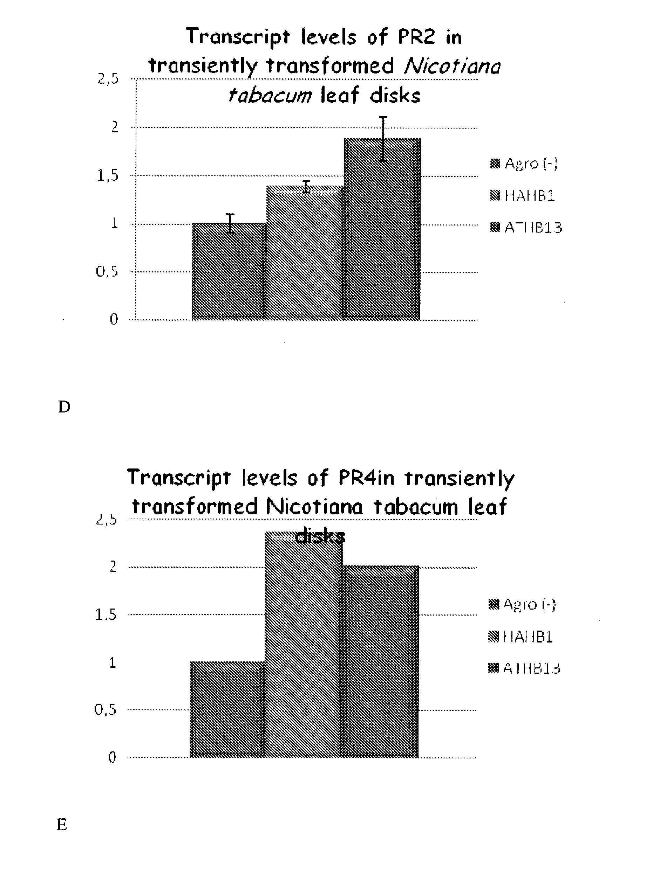

### Figure 25. Putative PR2, PR4 and glucanase are upregulated in transiently transformed soybean and Nicotiana tabacum leaf disks with the constructs 35S:HAHB1 and 35S:ATHB13.

Transcript levels of different response genes were measured by qRT-PCR; *ACTIN* genes *(ACTIN2* plus *ACTIN8)* were used as internal controls and standard deviations were calculated from at least three independent experiments; the measured genes for sunflower are chitinase (tc18434), HASAG21 (tc19654), HAZAT10 (tc16546) and HADREB (tc23839) while for soybean, they were GMPR2 (AK285952.1), GMPR4 (AK246040.1) and GM-glucanase (AY461847.1)and for tobacco, NTPR2 (EU867448.1) and NTPR4 (S72452.1)

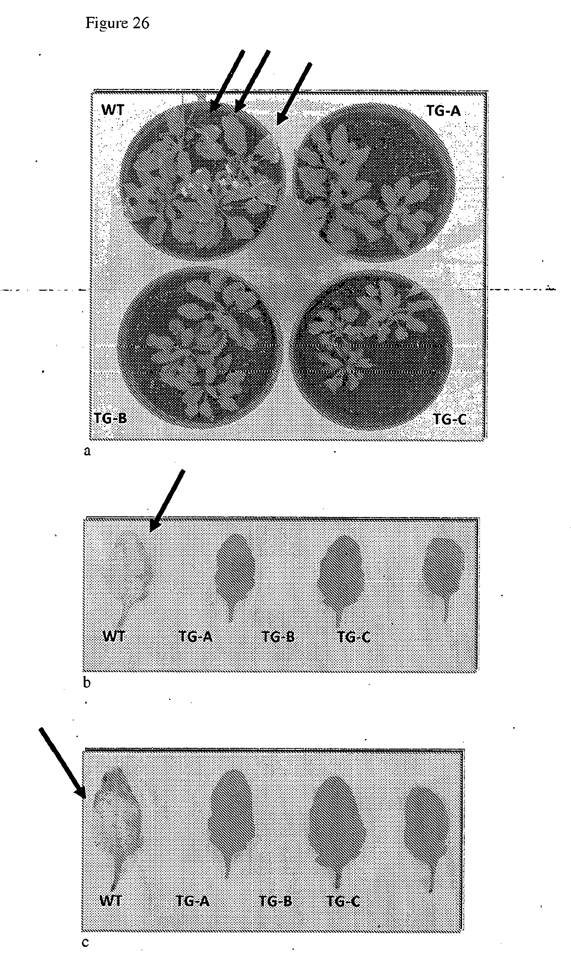

### Figure 26. Homozygous plants expressing the transcription factor HAHB1 are more tolerant to Pseudomonas infection.

26a: Plants infected with Pseudomonas are dying (wilted area is shown with an arrow) whilst transgenic plants are healthy and green. 26b is a photographs of the plants two days after the infection. In this photograph it can be seen that transgenic plants do not present necrosis, visualized by brighter coloured areas, while WT plants do. 26c is a photograph taken after staining with Evans blue (which colours the necrosis tissue). In this figure also it can be appreciated a difference between genotypes: WT plants are stained (speckled areas as marked by arrow) while transgenic are not.

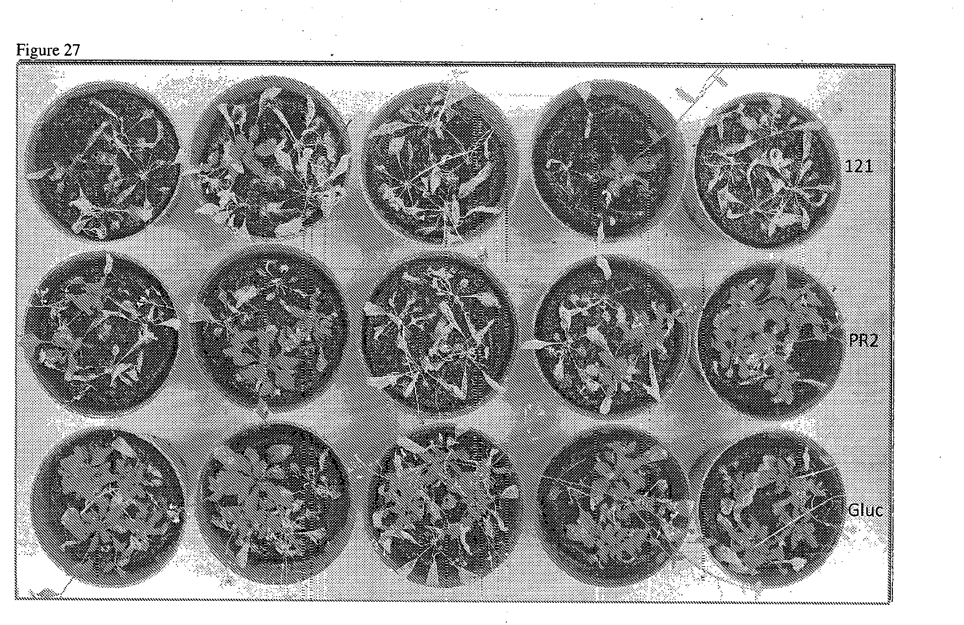

### Figure 27. Homozygous Arabidopsis plants expressing 35S-β glucanase and PR2.

35S-β glucanase (At4g16260) is one of the target genes of HAHB1 identified in the microarray experiments and plants expressing 35S-PR2 are more resistant to freezing conditions. 121 designates wild type (14% survivors in freezing conditions), 35S-β glucanase (62%), 35S-PR2 (42%). Control plants exhibit severe damage visualized as a marked wilting of the whole plant after a freezing treatment while transgenics present a greater percentage of healthy tissue, being higher in 35S:glucanase than in the 35S:PR2 genotype.

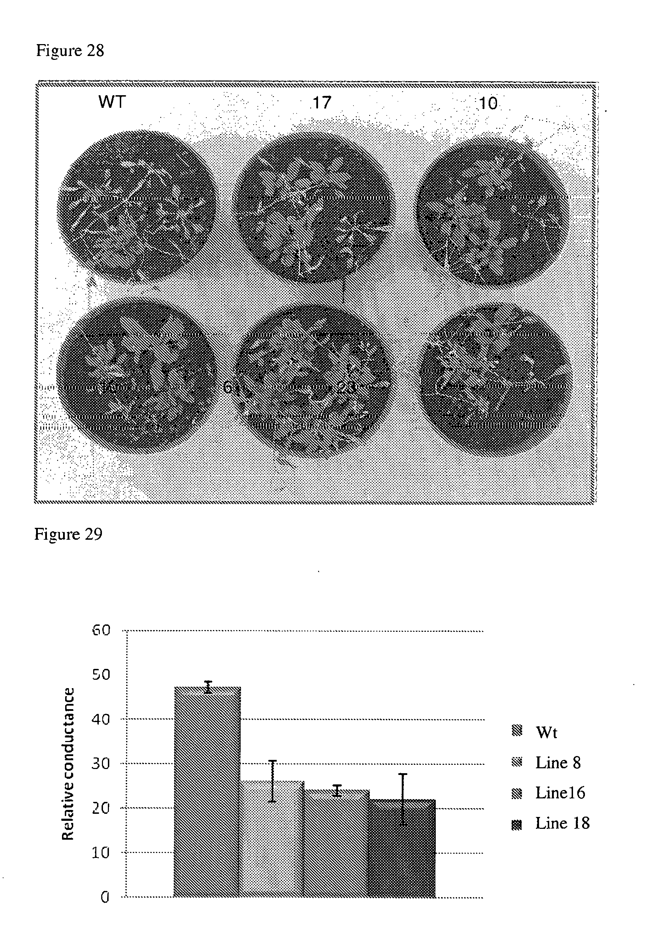

### Figure 28. PR4 (three of five independent lines shown) confers freezing tolerance when it is over and ectopically expressed in transgenic Arabidopsis plants.

Plants were grown in control conditions during 25 days and then treated 2 hours at -8°C in vermiculite/perlite. The plants were then placed in normal conditions for 6 days for recovery before being photographed. Control plants exhibit severe damage visualized as a marked wilting of the whole plant after a freezing treatment while transgenics (35S:PR4) present a greater percentage of healthy tissue. Transgenic plants were F2 (heterozygous). Percentage of survivors of each genotype after treatment:

| Genotype | Survivors % |
|---|---|
| WT | 31 |
| PR4-17 | 75 |
| PR4-19 | 75 |
| PR4-6 | 92 |
| PR4-10 | 75 |
| PR4-23 | 50 |

### Figure 29. Membrane stability in wt and transgenic plants expressing PR2 (line 8, 16 and 18) measured as conductivity of released saline.

The lower relative conductance indicate greater membrane stability in 35S:PR2 plants compared with WT plants after a freezing treatment. The x axis shows a treatment of "1 hour at -8°C".

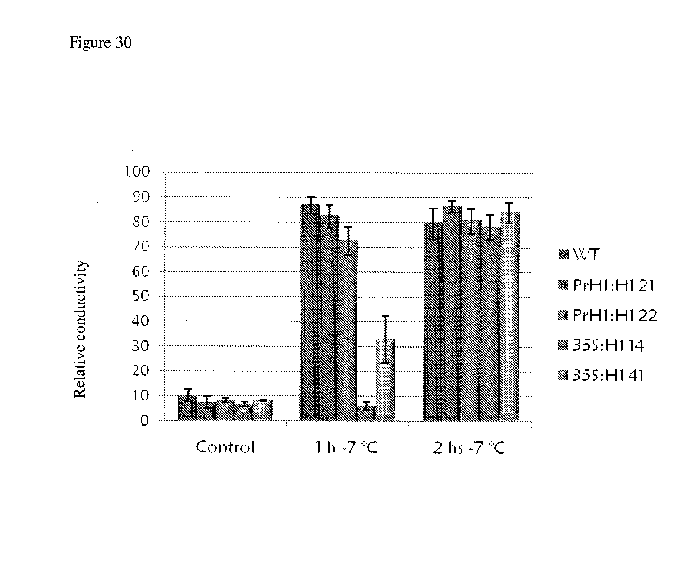

### Figure 30. Membrane stability in wt and transgenic plants expressing glucanase measured as conductivity of released saline.

The leaching technique was carried out essentially as described by Sukumaran and Waiser (1972). Plants were grown for 20 days in standard conditions and then irrigated with 50 mM NaCl (1 1 in a 45 x 45 cm tray). One week after this, the plants were irrigated with 150 NaCl and one more week later with 200 mM NaCl. One day after each saline irrigation, six leaves from each plant were excised and exhaustively washed with distilled water. After that the leaves were placed in 15 ml of double-distilled deionized water with continuous agitation in a water bath at 25 °C for 3 hr. After decantation the aqueous extract conductance (C1) was measured. Then, the leaves were placed in a 65 °C water bath for 16 hours with continuous agitation and one additional hour at 25°C prior to the measurement of the solution conductance (C2). The real conductance was calculated as the ratio between C2/C1 (L = C1/C) and used as index of injury. L values higher than 0.5 indicate a severe injury. The Y axis indicates "relative conductance". The lower relative conductance in 35S:H1 plants indicates greater membrane stability than in PrH1:H1 and WT plants after a one hour freezing treatment. In control conditions or after two hours of freezing treatment, there is no difference between genotypes.

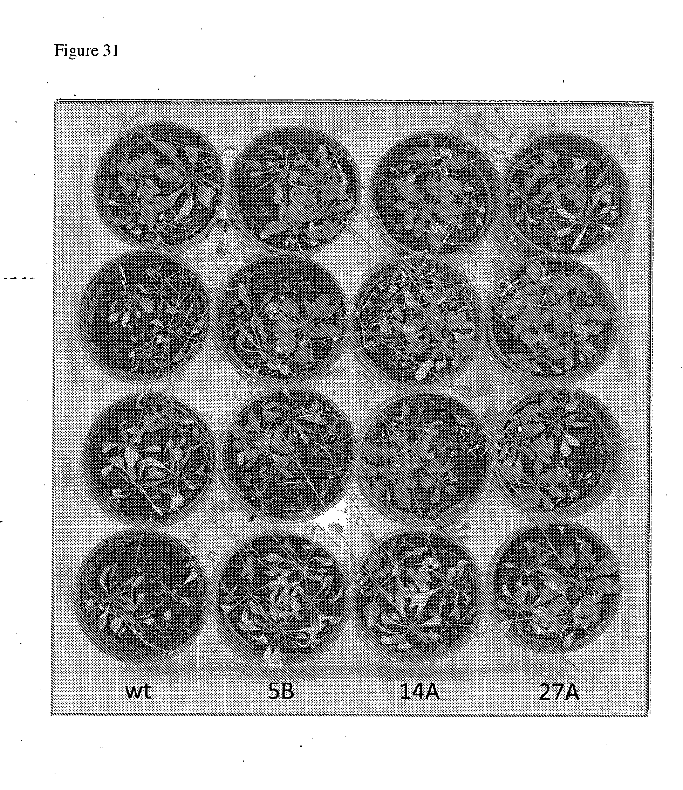

### Figure 31. Glucanase transgenic plants are more tolerant to drought stress

25 day old plants were subjected to drought stress by not watering the plants for 7 days. The photographs were taken five days after re-watering. 5b, 14a and 27a are lines carrying the glucanase transgene. WT plants (first column of plants left hand side) are more severely damaged than 35S:glucanase plants (second to third columns), as the wilting in their leaves demonstrates. Percentage of survivors of each genotype after treatment:

| Genotype | Survivors % |
|---|---|
| Wt | 20 |
| 5B | 46 |
| 14A | 62 |
| 27A | 69 |

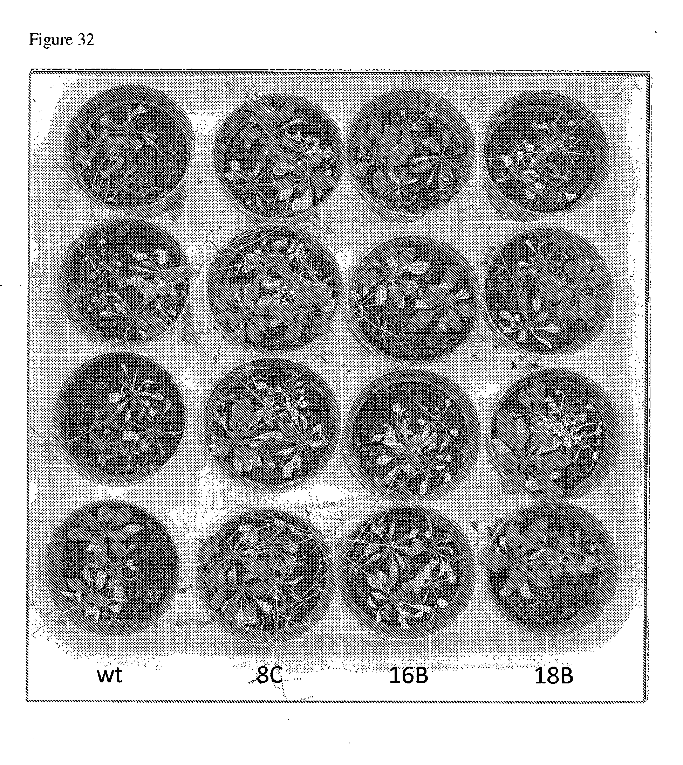

### Figure 32. Homozygous plants overexpressing PR2 are more tolerant to drought stress

This assay was performed essentially as the one described in figure 31, but with the genotype 35S:PR2 vs WT plants. The wilting in the aerial portion of WT plants (first column of plants on the left hand side) shows that they are significantly more affected by drought than 35S:PR2 plants (second to third columns: lines 8C, 16B, 18B), which display healthier tissues.

**Percentage of survivors of each genotype after treatment:**

| Genotype | Survivors % |
|---|---|
| Wt | 23 |
| 8C | 62 |
| 16B | 44 |
| 18B | 50 |

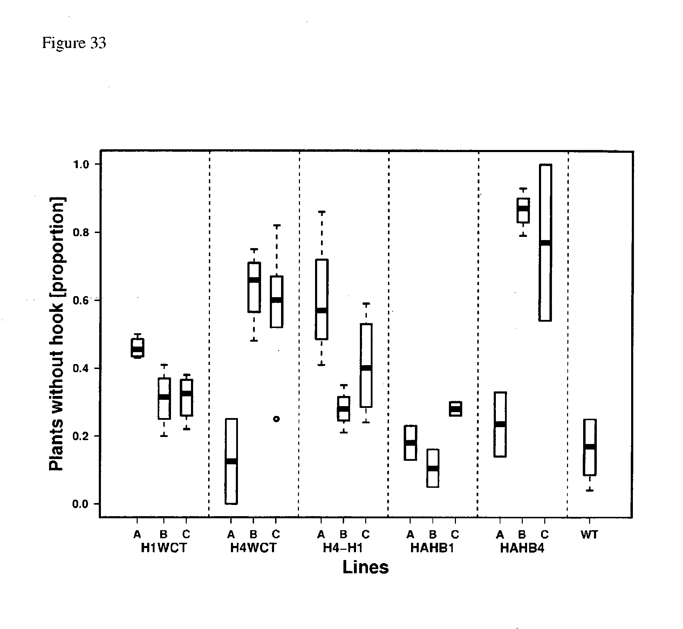

### Figure 33. Seedlings treated with ACC, ethylene precursor, were evaluated for the development of the triple response.

The presence or absence of the apical hook was quantified and the resulting proportions used to compare the degree of response in each line. 35SCaMV:HAHB4 Arabidopsis lines were almost unresponsive to ethylene. H4WCT, H1WCT and H4H1 showed an intermediate response. Finally, HAHB1 and WT were highly responsive. X axis: lines, Y axis: plants without an apical hook (proportion).

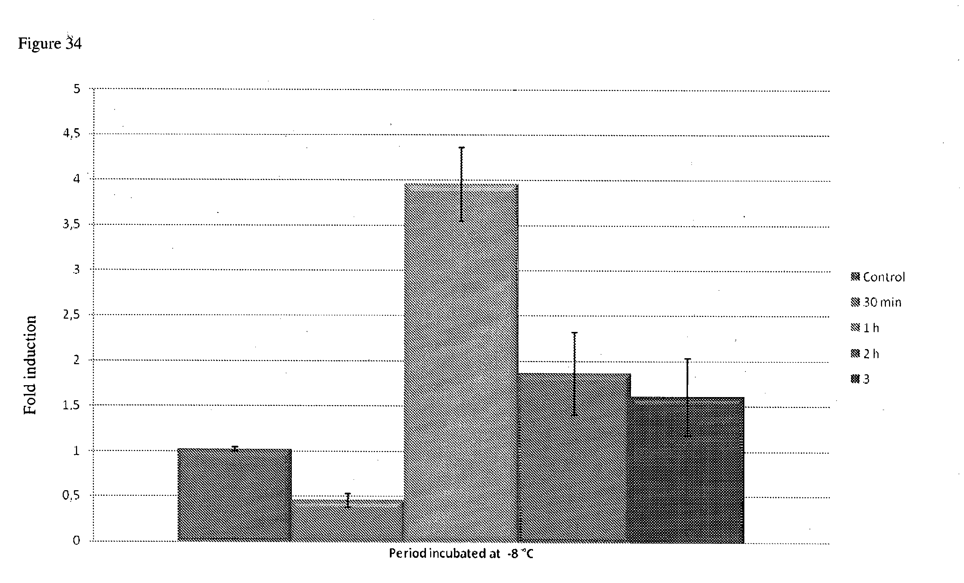

### Figure 34. ATHB13 expression kinetics in plants subjected to -8ºC

Kinetics of induction of *ATHB13* in 21-day-old seedlings placed at -8°C during 3 hours. Samples were taken at 0, 30, 60, 120 and 180 minutes. Transcript levels of *ATHB13* in 21-day-old leaves placed at -8°C were determined by quantitative RT-PCR and standard deviations calculated from three independent samples in which actin transcripts *(ACTIN2* plus *ACTIN8)* were used as internal controls; all the values are normalized with respect to the value measured at time 0, arbitrarily assigned a value of one. The Y axis shows fold induction.

### DETAILED DESCRIPTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The practice of the present invention will employ, unless otherwise indicated, conventional techniques of botany, microbiology, tissue culture, molecular biology, chemistry, biochemistry and recombinant DNA technology, which are within the skill of the art. Such techniques are explained fully in the literature.

The term stress/stress tolerance as used herein includes abiotic and biotic stress. Said stress/stress tolerance is preferably selected from freezing, low temperature, chilling, drought, high salinity and/or invasion of pathogens. As shown herein, the transgenic plants show increased/enhanced tolerance to these types of stresses. The tolerance can be measured as shown in the examples. Tolerance is increased compared to wild type (wt) plants. The increase can be at least two-fold up to 10 fold or more.

We describe an isolated nucleic acid sequence comprising a nucleic acid sequence of SEQ ID. No. 1, a functional fragment, part or a functional variant thereof. In one embodiment, the isolated nucleic acid sequence comprises or consists of a nucleic acid sequence of SEQ ID. No. 1.

The term "functional part or functional variant of *HAHB1"* as used herein refers to a variant gene sequence or part of the gene sequence which retains the biological function of the full non-variant sequence, i.e. confers stress tolerance when expressed in a transgenic plant. Specifically, the variant may be a chimeric sequence that encodes for the C-terminus of HAHB1 as described herein (or to CI and/or CII of HAHB1 or the consensus sequence), for example coupled to the N-terminus of another HD-Zip I family member. The term "variant" may also refer to a sequence that encodes a peptide/protein sequence that is homologous to HAHB1 and which shows homology in the HD and LZ domains and also in the C-terminal domains (in particular to CI and CII as explained below). A functional variant also comprises a variant of HAHB1 which is substantially identical, i.e. has only slight sequence variations, for example in non conserved residues, to the HAHB1 and HAHB1 sequences as shown herein and confers stress tolerance. A functional part may be the sequence encoding for the CI and/or CII motif.

The inventors have also shown that the *HARB1* promoter as defined in SEQ ID. No. 1 is effective in sensing stressful conditions and can be used to confer stress-induced gene expression on a transgene, for example under chilling, freezing, low salinity or drought conditions. Thus, we also disclose a stress inducible promoter comprising or consisting of a nucleic acid sequence of SEQ ID. No. 1, a functional fragment or a functional variant thereof. The promoter is useful for controlling transgene expression of transgenes in transgenic plants transformed with a gene under the control of said promoter. Accordingly, we further disclose a vector comprising a gene construct comprising a nucleic acid sequence of SEQ ID. No. 1, a functional fragment or a functional variant thereof and to the use of a sequence as defined in SEQ ID No. 1, a functional fragment or a functional variant thereof as a stress inducible promoter. Furthermore, we disclose a method for conferring stress induced gene expression in a plant wherein said method comprises transforming a plant with an expression cassette comprising a nucleic acid sequence of SEQ ID No. 1, a functional fragment or a functional variant thereof, operably linked to a gene sequence of interest for expression.

Any gene sequence of interest may be operatively associated in this manner to achieve such induced expression on exposure of plants bearing the construct to appropriate stress conditions selected from freezing, low-temperature, chilling drought and/or conditions of high salinity.

We further describe an isolated nucleic acid sequence comprising or consisting of a nucleic acid sequence of SEQ ID. No. 7. In another aspect, we also disclose an isolated nucleic acid sequence comprising or consisting of a nucleic acid sequence of SEQ ID. No. 2. Described herein is an isolated polypeptide sequence comprising or consisting of a sequence of SEQ ID. No. 5.

Described are also vectors which comprise gene constructs encoding for a protein that confers stress tolerance in plants. Specifically, Described is a vector comprising a gene construct encoding for the HAHB1 protein comprising or consisting of SEQ ID No. 5, a functional part or variant thereof. In one embodiment, the vector comprises a gene construct encoding for the HAHB1 protein comprising or consisting of SEQ ID No. 5.

Also described is a vector comprising a gene construct comprising a nucleic acid sequence of SEQ ID. No 2, 6 or 7, a functional part or functional variant thereof. In particular, the sequence comprises or consists of nucleic acid SEQ ID. No. 6 or 7. In one embodiment, a nucleic acid sequence of SEQ ID. No 2, 6 or 7, a functional part or functional variant thereof is operably linked to a promoter sequence. For example, the vector comprises an expression cassette wherein a nucleic acid sequence of SEQ ID. 6 is operably linked to a promoter. In another embodiment, a nucleic acid sequence of SEQ ID. 7 is operably linked to a promoter.

The promoter used in the gene constructs of the vectors described above may be the endogenous HAHB1 promoter comprising SEQ ID No. 1, a functional part or functional variant thereof. For example, the vector comprises an expression cassette wherein a nucleic acid sequence of SEQ ID. 6 or 7 is operably linked to the HAHB1 promoter comprising a nucleic acid sequence of SEQ ID No. 1. In another embodiment, the promoter may be a promoter that drives constitutive overexpression of a gene. Overexpression according to the invention means that the transgene is expressed at a level that is higher than expression driven by its endogenous promoter. For example, overexpression may be carried out using a strong promoter, such as the cauliflower mosaic virus promoter (CaMV35S), the rice actin promoter or the maize ubiquitin promoter or any promoter that gives enhanced expression. A stress-inducible promoter, such as the RubisCO small subunit promoter may also be used. This list is not considered limiting as the skilled person will be able to select a suitable promoter.

For example, the vector comprises an expression cassette wherein a nucleic acid sequence of SEQ ID. 6 or 7 is operably linked to the CaMV35S promoter. Alternatively, enhanced or increased expression can be achieved by using transcription or translation enhancers or activators and may incorporate enhancers into the gene to further increase expression. Furthermore, an inducible expression system may be used, such as a steroid or ethanol inducible expression system. Also envisaged is ectopic expression, i.e. gene expression in a tissue in which it is normally not expressed.

Also described is a host cell transformed with a vector or a gene sequence as described herein. Specifically described is a host cell expressing a protein of SEQ ID NO. 5, a functional part or functional variant thereof.In a preferred embodiment, the cell is a plant cell. The plant cell may be a cell of a monocot or dicot plant as further defined herein.

We demonstrate herein that *HAHB1* confers stress tolerance, for example freezing tolerance, to transgenic plants via the induction of antifreeze protein (AFP) biosynthesis, which inhibits ice crystal formation.

Therefore, we describe a transgenic plant transformed with a vector as described herein or transformed with a gene sequence as described herein. Thus, we describe a transgenic plant expressing or overexpressing a gene encoding for the sunflower HAHB1 protein or a variant or functional part thereof. Preferably, the HAHB1 protein comprises or substantially consists of a sequence as defined in SEQ ID NO. 5, a functional part or functional variant thereof. Thus, in one embodiment, the transgenic plant expresses or overexpresses a gene encoding for the HAHB1 protein as defined in SEQ ID NO. 5. For example, the plant may be transformed with a gene construct comprising a nucleic acid sequence of SEQ ID No. 6 or 7, a functional part or functional variant thereof. The nucleic acid comprising a sequence of SEQ ID No. 6 or 7 may be under the control of a promoter as defined above. In one embodiment, the nucleic acid comprising a sequence of SEQ ID No. 6 or 7 is under the control of the HAHB1 promoter comprising a nucleic acid sequence of SEQ ID No. 1, a functional part or functional variant thereof. In another embodiment, the nucleic acid comprising a sequence of SEQ ID No. 6 or 7 is under the control of the CaMV35S promoter. As explained below, the gene sequence may also be a gene sequence that encodes a homolog of HAHB1 (with high homology in the HD, LZ and CICII domains), such as ATHB13, or a chimeric construct that comprises the C-terminal domain of HAHB1. The plant is characterised in that it is more tolerant to stress conditions compared to its wild type counterpart, specifically to a stress selected from freezing, low temperature, chilling, drought, high salinity and/or invasion of pathogens. In one embodiment, the stress is freezing.

We also describe, a method for producing a stress tolerant plant or enhancing stress tolerance of a plant comprising transforming a plant with a nucleic acid sequence encoding for *ATHB13* comprising a sequence as shown in SEQ ID NO. 64 or a functional variant or part thereof. Thus, the uses and methods as described in respect of SEQ ID NO. 2, 6 and 7 also apply to SEQ ID NO. 64. Another example that can be used is *ATHB23,* a gene that shares high homology with *HAHB1.*

The transgenic plant thus obtained is characterised in that it shows enhanced stress tolerance compared to a control plant. The control plant is preferably a wild type plant.

In another aspect, we describe a method for producing a stress tolerant plant comprising transforming a plant with a nucleic acid sequence as described herein or a vector as described herein. We also disclose a method for increasing stress tolerance in a plant comprising transforming a plant with a nucleic acid sequence as described herein or a vector as described herein.

In one embodiment of these methods, the transgene directs the expression of the HAHB1 protein comprising a sequence of SEQ ID No. 5. For example, transformation may be using a nucleic acid comprising a sequence of SEQ ID No. 6 or 7. This sequence may be under the control of the *HAHB1* gene promoter comprising a nucleic acid sequence of SEQ ID No. 1, a functional part or functional variant thereof or under the control of the CaMV35S promoter. The transgene may also direct expression of a part of the HAHB1 protein (for example the C-terminus fused to the N-terminus of another HD-Zip protein) or of a homolog of *HAHB1,* for example *ATHB13.*

In connection with the foregoing aspects, stress tolerance is selected from tolerance to abiotic or biotic stress. Abiotic stress is selected from freezing, low-temperature, chilling, drought and/or conditions of high salinity. Biotic stress is stress caused by pathogenic organisms, such as bacterial or fungal pathogens (see example 16 and figure 26).

In another aspect, we disclose a plant obtainable or obtained by a method as described herein.

In another aspect, we disclose use of a nucleic acid sequence or use of a vector as defined herein in conferring increasing stress tolerance in a plant. In one embodiment, the sequence used is a nucleic acid comprising a sequence of SEQ ID No. 6 or 7. This sequence may be under the control of the HAHB1 promoter comprising a nucleic acid sequence of SEQ ID No. 1, a functional part or functional variant thereof or under the control of the CaMV35S promoter. Said stress tolerance is selected from tolerance to abiotic or biotic stress. Abiotic stress is selected from freezing, low-temperature, chilling tolerance, drought and/or conditions of high salinity. Biotic stress is invasion by a pathogen, for example Pseudomonas.

As shown herein, the plant into which a vector or sequence as defined herein is introduced may be *Arabidopsis.* The inventors have shown that the *HAHB1* gene sequence from sunflower can direct the expression of the HAHB1 protein in *Arabidopsis.* Moreover, the inventors have shown that transgenic expression of 35S:HABH1 and expression of 35S:ATHB13 respectively in other plants or plant tissue results in the upregulation of the expected target genes, thus providing evidence that both HABH1 and ATHB13 are effective in exogenous plant hosts and that transgenic expression of HABH1 and ATHB13 has universal application in genetically manipulating plants. The skilled person thus would know that the invention is not limited to *Arabidopsis,* soybean or tobacco which are used as non-limiting examples in the experiments herein. The skilled person would know that any monocot or dicot plant can be used. A dicot plant may be selected from the families including, but not limited to *Asteraceae, Brassicaceae (eg Brassica napus), Chenopodiaceae, Cucurbitaceae, Leguminosae (Caesalpiniaceae, Aesalpiniaceae Mimosaceae, Papilionaceae or Fabaceae), Malvaceae, Rosaceae or Solanaceae.* For example, the plant may be selected from lettuce, sunflower, *Arabidopsis,* broccoli, spinach, water melon, squash, cabbage, tomato, potato, capsicum, tobacco, cotton, okra, apple, rose, strawberry, alfalfa, bean, soybean, field (fava) bean, pea, lentil, peanut, chickpea, apricots, pears, peach, grape vine or citrus species. In one embodiment, the plant is oilseed rape.

Also included are biofuel and bioenergy crops such as sugar cane, oilseed rape/canola, linseed, and willow, poplar, poplar hybrids, switchgrass, Miscanthus or gymnosperms, such as loblolly pine. Also included are crops for silage (eg forage maize), grazing or fodder (grasses, clover, sanfoin, alfalfa), fibres (e.g. cotton, flax), building materials (e.g. pine, oak), pulping (e.g. poplar), feeder stocks for the chemical industry (e.g. high erucic acid oil seed rape, linseed) and for amenity purposes (e.g. turf grasses for golf courses), ornamentals for public and private gardens (e.g. snapdragon, petunia, roses, geranium, Nicotiana sp.) and plants and cut flowers for the home (African violets, Begonias, chrysanthemums, geraniums, Coleus spider plants, Dracaena, rubber plant).

A monocot plant may, for example, be selected from the families *Arecaceae, Amaryllidaceae* or *Poaceae.* For example, the plant may be a cereal crop, such as wheat, rice, barley, maize, oat, sorghum, rye, onion, leek, millet, buckwheat, turf grass, Italian rye grass, switchgrass, Miscanthus, sugarcane or Festuca species.

Preferably, the plant into which a sequence or vector of the invention is introduced is a crop plant. By crop plant is meant any plant which is grown on a commercial scale for human or animal consumption or use or other non-food/feed use.

Preferred plants are maize, tobacco, wheat, rice, oilseed rape, sorghum, soybean, potato, tomato, barley, pea, bean, field bean, cotton, lettuce, broccoli or other vegetable brassicas or poplar.

A sequence or vector described herein encoding for the HAHB1 protein is introduced as a transgene into the plant. This can be carried out by various methods as known in the field of plant genetic engineering, for example using transformation with *Agrobacterium* or particle bombardment.

The gene may be an exogenous gene, such as sunflower *HAHB1,* expressed in a different plant species. Alternatively, the invention also relates to using an endogenous gene expressing a *HAHB1* homologue, i.e. a gene encoding for a homologue of *HAHB1* that is endogenous to the plant in which it is introduced and expressed or overexpressed. As explained elsewhere herein, the homologous gene shows high sequence similarity in the HD and LZ domains, but also in the C-terminal domains (CI and CII) as the C-terminal domains appear crucial in conferring HAHB1 function.

We describe measurement of several select parameters in plants transformed with a construct bearing the *HAHB1* gene under the control of a constitutive promoter, such as the 35S promoter, operatively linked to the coding sequence of the HAHB1 protein, or a suitable variant, analogue, homologue or orthologue thereof, referred to generally herein as HAHB1, to produce a construct entitled *35S:HAHB1,* or under the control of the promoter of the HAHB1 gene, a suitable variant, analogue, homologue or orthologue thereof, generally referred to herein as *promHAHB1:HAHB1.* The comparison, using those parameters, of these plants with plants transformed with a control construct, e.g. pBI 121, is described in further detail in the Experimental section below, and referred to herein as "WT". The comparison confirms that the increase in freezing tolerance observed in the plants comprising the HAHB1 construct, under control of either promoter, is due to the action of the induced AFPs, and that this ultimately helps to conserve membrane stability and chlorophyll content as well as to inhibit ice crystal formation and damage in the recombinant plants.

It has been reported previously that it is common to observe developmental and morphological penalties in transformed plants bearing transcription factors controlled by constitutive promoters such the CaMV 35S as transgenes (Arce et *al.,* 2008 and references therein). These penalties are probably caused by the metabolic costs generated by the activation of specific protein biosynthesis, unnecessary in normal growth conditions. In this sense, *HAHB1* seems to delay very slightly the developmental rate in the earlier developmental stages of plants transformed with the 35S:HAHB1 construct, but this characteristic almost completely disappears later in the transformed plant's life cycle. No morphological or developmental negative characteristics were observed in transgenic plants transformed with the construct *promHAHB1:HAHB1,* indicating that in these plants the gene is expressed at a very low level in the absence of environmental stress. Apparently these transcript levels are not enough to produce the high metabolic costs but sufficient to achieve the desired freezing tolerance response.

It is important to distinguish the difference between chilling and freezing conditions because the consequences and the responses triggered by the plants are not the same in these two distinct stresses. Prolonged freezing causes tissue death and, ultimately, plant death while prolonged chilling results in plant acclimation and developmental arrest. In Arabidopsis plants, whether WT or transgenic according to this invention, incubated for several months at 4°C, the plants survive when placed in normal conditions. Subsequent exposure of these acclimated plants to freezing conditions demonstrates enhanced survival of the acclimated plants as compared to non-acclimated plants. In spite of not being able to detect a difference in survival in chilling conditions between the acclimated WT and acclimated *HAHB1*-transformed plants we could measure a significant difference, both in chlorophyll content and in membrane stability between genotypes, thus indicating a better tolerance achieved by the transgenic genotype.

On the other hand, freezing treatments detected a greater percentage of survival, both in vegetative and reproductive stages, for the *HAHB1*-transformed plants as compared to WT plants, reinforcing the results obtained during chilling. Together, the observations confirm that transformation with *HAHB1* confers both freezing tolerance and chilling tolerance to plants.

As well as freezing, or chilling, drought or high salt concentrations, for example, also cause stress to plants. However, the tolerance of plants to different types of abiotic, stresses is not necessarily conferred through related mechanisms and indeed occurs via different signal transduction pathways. Thus, it cannot be expected that a gene that confers, when expressed, one type of stress, could also confer a different type of stress. Notably and unexpectedly, we demonstrate herein that *HAHB1* acts to enhance tolerance responses in plants to all three of these different stress factors - freezing, drought and high salt. *HAHB1* transgenic plants are more tolerant to drought and high salinity than the non-transformed controls, indicating that the HD-Zip protein is somehow concomitantly acting in several abiotic stress responses, at least when it is constitutively expressed. Microarray data confirms this, since several genes previously described as involved in abiotic stress tolerance, such DREB, are strongly induced in transgenic plants ectopically and constitutively expressing *HAHB1.*

Moreover, as shown in the examples, *HAHB1* transgenic plants are more tolerant to invasion by pathogens, for example *Pseudomonas.*

Tolerance responses are conserved among species. In winter rye, one of the more characterized species with respect to the freezing response, antifreeze proteins homologous to those detected in the Arabidopsis microarray, are ultimately responsible for this tolerance. On the other hand, although there is limited genomic data available for sunflower, we were able to identify some genes (homologous to those detected in the microarray) showing higher expression levels in transiently transformed sunflower leaves. Acclimation assays indicate a consistent result in that apoplast protein patterns change both in Arabidopsis and sunflower when the plants are placed at 4°C. In the Experimental Procedures section herein, we provide data from recrystallization experiments which show that transgenic plant apoplast extracts are more efficient at preventing ice crystal formation than apoplast extracts from control plants.

We also show that Arabidopsis PR proteins, thought to function in anti-pathogen responses, act in the antifreeze response. Plants overexpressing HAHB1 and separately, PR2, PR3 and glucanase were tested in freezing conditions and these genotypes were more tolerant to freezing than control (wt) plants. Without wishing to be bound by theory, it is possible, on the one hand, that a group of antifreeze proteins may be cooperatively acting to achieve an optimal tolerance and only if *HAHB1* is present are all of them expressed. On the other hand, based on the data presented herein, some of these PR proteins may be able to confer freezing tolerance by themselves if they are highly expressed. As shown in example 10 and figures 27 to 32, overexpression of PR2, PR4 and β-Glucanase, confers tolerance to freezing conditions compared to the wild type. Moreover, tolerance to increased salinity was also observed. Thus, expressing PR2, PR4 and β-glucanase in a transgenic plant can lead to increased stress resistance. Expression may be from the endogenous promoter or any other promoter defined herein, for example a promoter suitable for overexpression.

Thus, in another aspect, we disclose a method for producing a stress tolerant plant or enhancing stress tolerance of a plant comprising transforming a plant with a nucleic acid sequence encoding for PR2. The sequence may comprise SEQ ID NO. 66, a homologue or variant thereof. Said tolerance is preferably freezing tolerance or drought tolerance. A plant obtained by this method is also disclosed. Also disclosed is the use of a nucleic acid sequence encoding for PR2 in increasing/conferring stress tolerance.

In a further aspect, we describe a method for producing a stress tolerant plant or enhancing stress tolerance of a plant comprising transforming a plant with a nucleic acid sequence encoding for glucanase. The sequence may comprise SEQ ID NO. 65, a homologue or variant thereof. Said tolerance is preferably freezing tolerance or drought tolerance. A plant obtained by this method is also described. Also described is the use of a nucleic acid sequence encoding for glucanase in increasing/conferring stress tolerance.

In a further aspect, we describe a method for producing a stress tolerant plant or enhancing stress tolerance of a plant comprising transforming a plant with a nucleic acid sequence encoding for PR4. The sequence may comprise SEQ ID NO. 67, a homologue or variant thereof. Said tolerance is preferably freezing tolerance or drought tolerance. A plant obtained by this method is also described. Also described is the use of a nucleic acid sequence encoding for PR4 in increasing/conferring stress tolerance.

In another aspect, we describe to a method for identifying a nucleic acid sequence which confers stress tolerance when introduced into a plant, which comprises using a nucleic acid sequence comprising a sequence of SEQ. ID. NO: 1, 2, 6, 7, or a part thereof or a sequence encoding for a sequence having homology to the sequence of SEQ. ID. NO. 11, 12 or 14, or a nucleic acid encoding all or a selected part of SEQ. ID. NO: 8 or 13 or a nucleic acid encoding all or a selected part of SEQ. ID. NO: 5, to probe a plant genome or plant genomic clones in a library. The sequences can also be used to probe an electronic library, thus identifying sequences with homology using bioinformatics.

The disclosure also relates to an isolated nucleic acid sequence obtained or obtainable by the method described above.

In another aspect, the disclosure relates to nucleic acid sequence homologous to the *HAHB1* sequence as defined in SEQ ID NO. 6 or 7 wherein said gene sequence is capable of conferring stress tolerance when introduced and expressed in a plant.

Preferably, said homologous nucleic acid sequence shows at least 80%, preferably at least 90%, more preferably at least 95% homology to the *HAHB1* sequence comprising SEQ ID NO. 6 or 7.

The homologous nucleic acid sequence is characterised in that it encodes a protein that has in its N terminal region a homeodomain with homology to the consensus as shown in SEQ ID No. 14.

The sequence also comprises a leucine zipper. The leucine zipper is already defined, i.e., in the CDD database, in which it is identified with this name: PSSM Id: 121415.

Moreover, the homologous nucleic acid sequence is characterised in that it encodes a protein that has a C-terminal sequence with homology to the C-terminus sequence of HAHB1 as shown in SEQ ID NO. 8. Furthermore, the homologous nucleic acid sequence is characterised in that it encodes a protein that has a C-terminal sequence comprising a sequence with homology to the consensus sequence as shown in SEQ ID NO. 11 and/or 12. Preferably, said homology is at least 80%, preferably at least 90%, more preferably at least 95% homology to the HAHB1 sequence comprising SEQ ID NO. 11.

From the information provided herein, a skilled person will appreciate the presence of conserved domains and will know that on the basis of the sequence information provided, primers can also be designed to aid the identification of homologous.

In addition to the protein sequence homology and the biological function of conferring stress tolerance when expressed in a plant by transgene technology, a homologous sequence can also be identified because it shows similarity to *HAHB1* in respect of the expression pattern directed by its promoters and the behaviour in sucrose.

Due to the detailed sequence information available, it is also possible to identify homologous sequences from existing databases using bioinformatics. Once the homologue sequences has been thus identified, it is possible to design oligonucleotides to amplify the respective cDNAs using RNA isolated from the chosen plant in a condition in which the gene is expressed. After amplification, the DNA segment can be cloned in a suitable vector to be checked by sequence determination (multicopy) and subcloned in a vector suitable for plant transformation directed by a selected promoter. If the promoter of the homologue is wanted, it is necessary to amplify it by PCR or alternatively to isolate the whole gene from a genomic library. Isolation from a genomic library is obliged when the whole sequence is unknown and only a partial segment is known to construct a probe. In the same way the cDNA can be isolated from a cDNA library if such library and a suitable probe are available. The gene thus identified can then be used in the method described below.

In another aspect, the disclosure relates to a method for producing a stress tolerant plant or enhancing stress tolerance of a plant comprising transforming a plant with a homologous nucleic acid sequence as described above.

The classification of HD-Zip proteins into four subfamilies is supported by the following four distinguishing characteristics: conservation of the HD-Zip domain determining DNA binding specificities, gene structures, additional conserved motifs and functions.

Members of the HD-Zip family exhibit a LZ motif just downstream from the HD motif. The two motifs are present in transcription factors belonging to other eukaryotic kingdoms, but their association with each other in a single protein is unique to plants. The HD is responsible for the specific binding to DNA while the LZ acts as a dimerization motif. HD-Zip proteins bind to DNA as dimers, and the absence of the LZ absolutely abolishes their binding ability, indicating that the relative orientation of the monomers, driven by this motif, is crucial for an efficient recognition of DNA.

In Arabidopsis, subfamily I is composed of seventeen members *(ATHB1*/*HAT5, 3*/*HAT7, 5, 6, 7, 12, 13, 16, 20, 21, 22, 23, 40, 51, 52, 53, 54).* HD-Zip I subsets of genes (in Arabidopsis) share their intron/exon distribution in accordance with their phylogenetic relationships. The molecular weight of the encoded proteins is about 35 kDa and exhibit a highly conserved HD and a less conserved LZ. No other similarity or the presence of additional conserved motifs has, to our knowledge, been described.

A full *in vitro* description, consisting of PCR-assisted binding site selection and footprinting assays, determined that all the proteins encoded by HD-Zip I genes and tested, recognize, as dimmers, the pseudopalindromic sequence CAAT(A/T)ATTG (Ariel et al., 2007).

Aligning the 15 most homologous Arabidopsis HD-ZIP subfamily I proteins, it is possible to obtain a relatively good consensus for the homeodomain, but not for the leucine zipper. The definition of the latter depends on a conservation of leucines and certain other residues with special attention to relative position, so homology percentages tend to be very low (the conservation is mainly structural defined by the positions of the leucines).

In an alignment of 15 *Arabidopsis thaliana* HD-Zip class I proteins, the percentages are much higher for the homeodomain than for the leucine zipper domain.

Expression of the HD-Zip class I genes that have been characterized indicates that its expression is regulated by external factors like drought, extreme temperatures, osmotic stresses and illumination conditions, and is specific to different tissues and organs of the plant. Their role as transcription factors is related to developmental events in response to such environmental conditions, particularly those in which abiotic factors generate stress but not necessarily conferring tolerance to such stresses. Nor, for those factors which do confer tolerance, can it be predicted, *a priori,* to which stress they will confer resistance.

Different subsets of Arabidopsis HD-Zip I genes that bear a close phylogenetic resemblance exhibit common organ expression patterns and are responsive to the same environmental factors.

*ATHB1,* the first isolated member, acts as a mediator in the leaf cell fate determination, whereas *ATHB16* is involved in blue-light perception signalling. Another group of genes was proposed to be involved in ABA-related and abiotic stress responses. Evidence was obtained from expression studies and transgenic plants, indicating that *ATHB7, 12, 5* and 6 are up or down regulated by water deficit conditions and/or externally applied ABA. Under the effect of these stimuli, HD-Zip I genes behave as developmental and growth regulators. The sunflower *HAHB4* gene, for its part, confers drought tolerance to transgenic Arabidopsis plants when it is expressed under the control of constitutive or drought-inducible promoters, while ATHB7 and ATHB12 do not, even though they are the more related genes in Arabidopsis, except for at the C-terminus. Since the sunflower genome sequence is not available, and considering that *HAHB4* does not behave as do *ATHB7* or *12,* it is most likely that they are not orthologous genes even if they exhibit a high sequence homology and are regulated by the same external factors.

Overexpression of *ATHB3, 13, 20* or *23* suggests that these genes are involved in the regulation of cotyledon and leaf development, even though *ATHB13* and *ATHB23* are the Arabidopsis genes presenting the highest homology with *HAHB1.*

HD-Zip I genes have evolved by a series of gene duplications to a considerable complexity, resulting in subsets of paralogous genes which share intron/exon distribution, amino acid sequences and expression patterns. Ectopic expression of each HD-Zip I subset of genes provokes different phenotypic effects.

The inventors investigated why HD-Zip I proteins, which all bind to the same DNA sequences (a feature determined by helix III of the HD domain) and have similar expression patterns, nevertheless participate in different signal transduction pathways and even exert diverse functions.

We have shown herein that a comparison of each HD-Zip member shows a high degree of conservation in the carboxy-terminus region of proteins belonging to different species (see alignment provided as figure 21 herein).

Subfamily I can be divided into several groups according to the conservation in the carboxy terminus domain. All the members share homology in the HD-Zip domain, but when the alignment with the whole encoded proteins is performed, several groups can be visualized (phylogenetic tree) that differ between them in the carboxy terminus.

Notwithstanding the high degree of conservation presented in this region, no similarity with any other known motif has been detected, despite a deep bioinformatic analysis. HAHB1 shares this similarity with proteins of varied and distant species. Without wishing to be bound by theory, the inventors believe that the C terminal region provides a disordered protein-protein interaction domain, and the interaction of this domain with other diverse proteins determines the function of the protein.

The inventors have shown herein that without the HD-Zip domain, the HAHB1 COO terminus does not function autonomously. They have also shown that the HD-Zip of HAHB4 (with its COO terminus deleted) fused to the COO terminus of HAHB1, appears to behave like HAHB1. All the HD-Zip I members characterized up to now bind the same DNA sequence. Therefore, it is believed that the HAHB1 COO terminus fused to the HD-Zip region of any subfamily I HD-Zip domain acts as a functional HAHB1.

From the experiments shown herein, it can be seen that it is the C-terminal region (CICII) that confers protein function. Diversity in the C terminus of the members of the HD-Zip I family thus seem to explain the different functions and developmental effects of these proteins. Chimeric constructs which comprise the HAHB1 COO terminus fused to the HD-Zip region of any subfamily I HD-Zip domain acts as a functional HAHB1 can therefore also be used in the methods for conferring increased abiotic and biotic stress tolerance as described herein.

In such a chimeric construct, the N-terminal region is characterised in that it comprises a homeodomain with homology to the consensus as shown in SEQ ID NO. 14 (which was obtained from subfamily I), associated in its C terminus to a leucine zipper. Moreover, this conserved homeodomain is able to bind the palindromic sequence CAAT(A/T)ATTG which is characteristic for this subfamily and differs from the sequences bound by members of other subfamilies.

Therefore, in another aspect, we disclose an isolated nucleic acid sequence comprising or consisting of a sequence as defined in SEQ ID No. 8, 9, 10, 11, 12 or 13. Also disclosed are vectors comprising such sequences.

In another aspect, we disclose a chimeric gene construct comprising a nucleic acid sequence encoding for the N-terminal sequence of an HD Zip protein, a part thereof or a sequence comprising a N-terminal consensus motif of subfamily I, operatively associated with a nucleic acid sequence encoding for a sequence comprising the C-terminus of HAHB1, part thereof or a sequence comprising the HAHB1C-terminal consensus motif.

The disclosure also relates to a polypeptide encoded by this gene construct. The polypeptide is capable of conferring stress tolerance in a plant into which a gene expressing such polypeptide is introduced.

In one embodiment of this aspect of the disclosure, the C-terminal sequence of said polypeptide comprises or consists of SEQ ID NO. 8, 9 and/or 10. In another embodiment, the N-terminal sequence of said polypeptide comprises or consists of SEQ ID NO. 14 or a sequence with homology to the consensus sequence of SEQ ID NO. 14. Said homology is at least 80%, preferably at least 90%, more preferably at least 95% or 98%. In another embodiment, the polypeptide has the N-terminal sequence of an HD-Zip protein of subfamily I or the N-terminal consensus motif operatively associated with a sequence comprising or consisting of a sequence as defined in SEQ ID NO. 11 and/or SEQ ID NO. 12 or a sequence with homology to the C-terminal consensus motif as defined in SEQ ID NO. 11 and/or the C-terminal consensus motif as defined in SEQ ID NO. 12. Said homology is at least 80%, preferably at least 90%, more preferably at least 95% or 98%.

In another embodiment, the N-terminus of said polypeptide is the N-terminus of HAHB4.

The disclosure also relates to a method for conferring stress tolerance as defined herein in a plant which comprises introducing and expressing in a plant a chimeric gene construct above which encodes for a polypeptide or protein as defined above.

### EXAMPLES

Having generally described the invention disclosed herein, including methods by which those skilled in the art could make and use the invention, the following examples are provided to further extend this description, to enable those skilled in the art to practice this invention, including its best mode. However, the specifics of the examples which follow are not limiting. Rather, for an appreciation of the scope of the invention contemplated herein, reference should be had to the appended claims and the equivalents thereof.

### EXPERIMENTAL PROCEDURES

Unless expressly stated otherwise, the following materials and methods were utilized in the non-limiting examples which follow:

### A. Constructs

***35SCaMV:HAHB1:*** The cDNA was isolated from a library constructed in lambda gt10 as previously described (Chan & Gonzalez, 1992, and the sequence was provided to GenBank, see accession no. L22847, and SEQ ID. NO:2: herein for the nucleic acid sequence and SEQ ID. NO:5: herein for the translated protein sequence). This fragment was cloned in the EcoRI site of the pMTL22 vector and restricted with *BamH*I/*Sac*I in order to clone it into the pBI 121 vector previously treated with the same enzymes. In this way, the cDNA expression is controlled by the 35S CaMV promoter.

***HAHB1:GUS:*** The promoter region (SEQ ID. NO:1: herein) was isolated from a BAC genomic library using as probe the first intron (SEQ ID. NO:3: herein) of the gene previously isolated by PCR using two specific oligonucleotides. The approximately 125 kbp BAC insert was restricted with several enzymes, electrophoresed and hybridized in a Southern blot with the same probe. A positive 2000 bp *Hind*III fragment was subcloned in the pUC119 vector and sequenced. The insert presented a partial coding sequence plus the promoter region. The promoter region was amplified by PCR with the oligonucleotides PromHAHB1 (table a) and PR (table a) and cloned in the TOPO vector (Invitrogen). This plasmid was then restricted with *Xba*I and *BamH*I and finally cloned in the pBI101.3 vector. In this way the promoter of *HAHB1* directs the expression of the *GUS* reporter gene. This construct was named *RAHB1:GUS.*

*promHAHB1:HAHB1: HAHB1:GUS* was restricted with *BamH*I/*Sac*I and the *HARB1* cDNA was cloned into the vector, replacing the *GUS* gene. In this way the expression of *HAHB1* is controlled by its own promoter.

These three constructs as well as pBI121 and pBI101.3 (used as controls) were used to transform DH5α *Escherichia coli* cells and then *Agrobacterium tumefaciens.*

Both, Arabidopsis glucanase and PR2 were cloned as follows: Arabidopsis (Col 0) genomic DNA was amplified with oligonucletotides glucanase CDS-R/glucanase CDS-F and PR2 CDS-R/PR2 CDS-F. The sequence of the oligonucleotides used for glucanase is shown in the sequence listing herein as SEQ ID NO. 23 and 24. The sequence of the oligonucleotides used for PR2 is shown in the sequence listing herein as SEQ ID NO. 21 and 22.

Once the DNA segments were obtained, they were cloned in the pBI121 vector previously restricted with *BamH1*/*SacI* replacing the *GUS* encoding gene. As with the other constructs, the cloning was performed in *E. Coli* cells and then *Agrobacteria* cells were transformed. Arabidopsis plants were then transformed following the floral dip method.

### B. Plant material and growth conditions

*Arabidopsis thaliana* Heyhn. ecotype Columbia (Col-0) was purchased from Lehle Seeds (Tucson, AZ). Plants were grown directly in soil in a growth chamber at 22-24°C under long-day photoperiods (16 h of illumination with a mixture of cool-white and GroLux fluorescent lamps) at an intensity of approximately 150 µE m⁻² s⁻¹ in 8 cm diameter x 7 cm height pots during the periods indicated in the figures.

*Helianthus annuus* L. (sunflower cv. contiflor 15, from Zeneca) seeds were grown in soil in a culture room at 28°C for variable periods of time depending on the purpose of the experiment as detailed in the figure legends.

### C. Abiotic stress and hormone treatments

Sunflower seedlings (7-day-old) grown on wet paper were placed in liquid media containing the different hormones (in the concentrations indicated in the figure legends) and incubated for two hours. The plants were then frozen in liquid nitrogen; total RNA was extracted from each sample and analyzed by real time PCR (RT-PCR) as described below.

A similar procedure was followed with 21-day-old plants, but in this case, instead of whole seedlings, 1 cm diameter leaf disks (three disks for each treatment) were placed in the media containing the hormones. For seedling drought assays, the seedlings were placed for 30 minutes on a dry paper.

### D. Arabidopsis transformation

Transformed *Agrobacterium tumefaciens* strain LBA4404 was used to obtain transgenic *Arabidopsis* plants by the floral dip procedure (Clough & Bent, 1998). Transformed plants were selected on the basis of kanamycin resistance and positive PCR which was carried out on genomic DNA with specific oligonucleotides. To assess *HAHB1* expression, RT-PCR was performed on T2 transformants, as described below. Five positive independent lines for each construct (arising from at least two different transformation experiments) were used to select homozygous T3 and T4 in order to analyze phenotypes and the expression levels of *HAHB1.* Plants transformed with pBI101.3 were used as negative controls. For the other constructs, selection was similarly carried out and three to five independent lines chosen for the analysis.

### E. Transient transformation of sunflower leaves

Sunflower leaves (in the R1 developmental stage; Schneiter & Miller, 1981) were infiltrated with 5 ml of *Agrobacterium tumefaciens* strain LBA4404 and then transformed with *35S:HAHB1* or *35S:GUS,* used as control. After infiltration, plants were placed in a growth chamber for an additional 48 hr; 1 cm diameter disks (50 mg each) were excised from the infiltrated leaves and RNA was then extracted with Trizol (see below). For each gene transcript measurement, two disks coming from different plants were analyzed and the experiment was repeated at least twice. In order to test the efficiency of infiltration in these experiments, *GUS* reporter gene expression was measured by histochemical assays as previously described (Dezar et *al.,* 2005b).

### F. Water stress treatments

Early water stress treatment in soil was carried out as follows: sixteen 8 x 7 cm pots, each with 120 g soil and 4 seeds, water-saturated, were placed in a 35 cm plastic square tray and cultured as described above except that further water was not added until severe damage was observed.

Water stress treatment was done on mature 4-week-old plants grown in the same culture conditions. At this age, no water was added again until stress became evident (approximately an additional 17 days). In both cases, photographs were taken two days after watering.

### G. Chilling assays

Arabidopsis plants were grown in the same temperature and photoperiod as for water stress treatments during 14 days (for vegetative stage) or 25 days (reproductive stage). Then, they were placed in a special culture chamber with the same photoperiod and illumination conditions at 4°C and maintained during the periods indicated in the corresponding figures.

### H. Freezing assays

Two different assay types were performed. The first used incubations of 6-8 hours at -8°C of non-acclimated plants grown in normal conditions. After the freezing treatment the plants were placed again in normal conditions and all the parameters measured after 6 days recuperation.

The alternative treatment used incubation of 14-day-old plants over 7 or 14 days at 4 °C and only after this acclimating treatment, were they subjected to - 8°C over 6-8 hrs. After that, the plants were placed at 4 °C for 24 hrs and then in normal growth conditions for 6 days.

### I. Salt stress treatments

To 14-day-old plants grown in normal conditions 1.5 L of 50 mM NaCl was added to the whole tray. Seven days after that, an additional litre of 150 mM NaCl was used to water the plants and finally seven days later another litre of 200 mM NaCl was added. In this way the salt stress occurs during the reproductive stage. Salt stress during the vegetative stage was generated by adding increasing concentrations of NaCl from 50 to 200 mM for watering of 10-day-old plants every 4 days.

### J. Chlorophyll quantification

Extracts from 100 mg of leaves were prepared after freezing with liquid nitrogen. To each sample, 1.5 ml of 80 % acetone was added and the tubes placed in darkness for 30 min. During this incubation the sample solids were decanted and the absorbance at 645 and 663 nm measured in the supernatants with a spectrophotometer. Chlorophyll concentration was quantified according to Whatley et *al.,* (1963).

### K. Membrane stability

Membrane stability was determined by the ion leakage technique. Leaves of each genotype were washed with distilled water before treating them with salt stress or low temperatures according to the experiment. After the corresponding treatment, they were placed in 15 ml distilled water and agitated for 1 hr at 25°C. After that, the conductivity (C₁, proportionally inverse to the stability) was measured in the liquid surrounding media. The final conductance (C₂) was determined in the supernatants after 4 hrs at 65 °C and one additional hour at 25 °C with continuous agitation. The damage index (L) was calculated as the ratio C₁/C₂. The basis for this technique is that the damaged tissues lose electrolytes and these electrolytes spread into the surrounding media and increase the conductivity. Values greater than 0.5 indicate severe damage (Sukumaran & Weiser, 1972).

### L. Hormone and stress treatments to transgenic plants

Transgenic plants (21-days-old) from the *promHAHB1:HAHB1genotype* were placed in 200 µM SA, 200 µM ABA or 20 µM ACC over 2 hrs. After the incubations, they were frozen and total RNA was extracted from each sample. Cold and drought treatments were carried out over 8 and 2 hrs respectively by placing the plants in a cultured chamber at 4°C or on dry paper. Plants transformed with the pBI101.3 plasmid were used as controls.

### M. RT-PCR measurements

RNA for real-time RT-PCR was prepared with Trizol® reagent (Invitrogen™) according to the manufacturer's instructions. RNA (1 µg) was used for the RT-PCR reactions using M-MLV reverse transcriptase (Promega). Quantitative PCRs were carried out using a MJ-Cromos 4 apparatus in a 25 µl final volume containing 1 µl SyBr green (10 x), 10 pmol of each primer, 3 mM MgCl₂, 5 µl of the RT reaction and 0.20 µl Platinum Taq (Invitrogen Inc.). Fluorescence was measured at 80-84°C during 40 cycles. Sunflower RNA was also prepared with the Trizol (Invitrogen Inc.) technique. Specific oligonucleotides for each gene were designed using publicly available sequences (www.arabidopsis.org). The designed sequences are as specified in Table a.

### N. Apoplast protein extraction and chromatographic purification

Apoplastic protein extraction was carried out essentially as described by Mauch and Staehelin (1989). Plant leaves (7 g) grown over 25 days in normal conditions and then alternatively acclimated over 10 days at 4°C or placed for 3h at 8 °C as indicated in the figures legend and treated as follows. After the acclimation period, they were infiltrated under vacuum for 20 min. in a solution containing 5 mM EDTA, 10 mM ascorbic acid, 10 mM β-mercaptoethanol, 1 mM PMSF, 2 mM caproic acid and 2 mM benzamidine. After infiltration, the leaves were dried over paper and placed in a 20 ml syringe. The syringe with the dried leaves inside was placed in a 50 ml tube and centrifuged for 20 min at 830 g. After centrifugation, the apoplastic extracts are recovered from the bottom of the tubes when they are not contaminated with chlorophyll. In such cases (chlorophyll contamination) the extracts were discarded. Protein concentration was determined by the Bradford technique. After that, the extracts were concentrated through Millipore Centricon Ultracel YM-10 columns equilibrated with 50 mM NH₄HCO₃. Aliquots of each sample (1 mg protein each) were purified through Sephadex G-200 columns previously equilibrated with 50 mM NH₄HCO₃ and the elution was performed with the same buffer and followed spectrophotometrically at 280 mn.

The proteins were analyzed in 12% SDS-PAGE carried out according to the Laemmli technique and visualized with *Coomassie Brilliant Blue* R-250 (Sigma). An Amersham Biosciences LMW calibration kit was used as a molecular weight marker. This kit allows the visualization of the following bands: 97, 66, 45, 30, 20 and 14.4 kDa.

### O. Recrystallization of ice

Recrystallization assays were carried out essentially as described by Griffith (2005). To the apoplastic extracts (10 µl) obtained as described above from previously acclimated plants, 10 µl 26% (w/w) sucrose was added. Each mix, corresponding to the different genotypes, was treated as follows: 3 minutes at -80°C, 10 minutes at -20°C, 15 minutes at -8°C, 30 seconds at 4°C and finally 1 hour at - 8°C. A control was performed using buffer (10 µl) instead of apoplast extract. The samples were observed and photographed with a NIKON optical microscope.

### EXAMPLE 1 HAHB1 expression pattern

*HARB1* cDNA was isolated from a sunflower stem library (Chan & Gonzalez, 1994) but its function was not characterized. This cDNA encodes a 365 amino acid protein and seems to be a non divergent member of the HD-Zip I subfamily. In order to characterize expression of this gene, total RNAs were isolated from 7-day-old organs and these were analyzed by qRT-PCR as described in the Experimental section. Figure 1a shows that at this stage of development the TF is mainly expressed in hypocotyls and apical meristems while the expression in cotyledons and roots is lower but detectable. This expression pattern changes in 21-day-old plants (Figure 1b) showing primary expression in petioles and leaves and lower expression levels in roots, cotyledons, hypocotyls and stems.

### EXAMPLE 2 HAHB1 expression is up-regulated by ABA, cytokines and abiotic stress effectors

We investigated the effect of some phytohormones in the expression of this gene in 7- and 21-day-old plants. The results, shown in Figure 2a and 2b, indicate that in seedlings BAP presents the higher effect on *HAHB1* expression while ABA exhibits the major effect in leaves of more developed plants. Other phytohormones tested (IAA; ethylene (ACC), SA, JA and GA) either inhibitor, slightly induce or produce no effects at all on the expression of this TF. Sunflower seedlings (7-day-old) or developing plants (21-day-old) were subjected to different abiotic stress treatments in order to investigate the role of this gene in the response to environmental conditions.

Drought, salinity, oxidative stress caused by H₂O₂, and osmotic stress caused by sucrose all show low induction of expression of this gene in seedlings. Etiolated plants exhibit transcript levels two-fold greater than those observed in normal growth conditions (Figure 3a). On the other hand, low temperatures (4°C) induce the expression of *RAHB1* more than five fold, indicating a significant up-regulation of this gene under this condition. A time course shows a maximal expression of *HAHB1* after 7 hours of cold treatment (Figure 3b). By contrast, in more developed plants (21-days-old), a different behaviour is exhibited. Several abiotic stress effectors such drought, NaCl high concentrations, darkness and cold temperatures induce the expression of the *HAHB1* gene (Figure 3c).

Together, the results indicate that *HAHB1* is induced in mature leaves by various different abiotic stress conditions.

### EXAMPLE 3 Obtaining Arabidopsis transgenic plants ectopically expressing HAHB1

To confirm the function of *HAHB1,* we have used an ectopic expression approach. The coding region of *HAHB1* was fused to the 35S promoter of Cauliflower mosaic virus, and the construct was used to transform *Arabidopsis* plants. Several homozygous lines were recovered. Five independent transgenic lines, named *35S:HAHB1-A,* -B, -C, -D and -E, were selected for more detailed analysis.

Figure 4a shows the difference in the leaf morphology between transgenic and wild type plants. Transformed plants show serrated borders and a differentiated shape in comparison with their non-transformed counterparts (Figure 4a and 4c). Regarding the development rate, transgenic plants present retarded stem elongation in early stages but reach a similar height at the end of the life cycle (Figure 4b). The maximal difference in stem height between genotypes was observed when the plants pass from the vegetative to the reproductive stage. The number of rosette leaves and siliques as well as the seed productivity did not show significant differences between genotypes.

### EXAMPLE 4 Transgenic plants expressing HAHB1 are more tolerant to low temperatures than their wild type counterparts

In view of the known role of some HD-Zip proteins in abiotic stress responses, and our finding that *HAHB1* is up-regulated at the transcriptional level by low temperatures, we investigated the behavior of transgenic plants bearing the construct *35S:HAHB1* when they are subjected to chilling or freezing temperatures at different developmental stages.

In order to test the behavior under freezing temperatures, the plants in vegetative stage were placed at -8°C for 7 hours and then in a culture chamber in normal conditions to allow them to recover. Figure 5 shows the results obtained. Between 57 and 85% (depending on the line) of transgenic plants expressing *HAHB1* survived the treatment as compared with only 20% of the non-transformed plants. The severe stress affected the leaves of all of the genotypes, but the transgenic plants became healthy after a few days of recovery while the WT plants did not.

As mentioned above, low temperatures cause inhibition of photosynthesis and respiration as well as a repression in protein biosynthesis and induction of protein degradation. Carbohydrate transport is inhibited as well. All these effects share, as a common feature, the loss of membrane functionality.

In order to determine the membrane health after freezing treatment, we used the ion leakage technique (see Experimental Procedures) that allows quantification of the damage caused. Electrolyte release to the medium is an indicator of the severity of membrane damage and the conductivity in the surrounding solution provides a quantitative measurement of this damage. After centrifugation the apoplastic extracts are recovered from the bottom of the tubes unless they are contaminated with chlorophyll. In such cases (chlorophyll contamination), the extracts were discarded. Plants subjected for a range of different times to freezing temperature (-8°C) were treated as described in the Experimental Procedures section above, and the conductivity measured. Figure 5a shows that, after two hours of incubation at -8°C the stability of WT plant membranes is lower than that of transgenic plants. At least in part, this explains the differences observed between genotypes in their survival under these conditions.

Regarding the composition of membrane lipids, no differences were detected in unsaturated and saturated fatty acid relationship between genotypes in plants subjected to chilling temperatures (data not shown) indicating that the observed freezing tolerance is not a consequence of a change in membrane lipids. Chilling also causes inhibition of photosynthesis. Chlorophyll concentration was measured in plants subjected to these conditions, both in vegetative and reproductive stages. Figure 6 shows that chlorophyll content remained stable in transgenic plants in both stages while non-transformed plants progressively lost this pigment content indicating that transgenic plants better tolerate the adverse temperatures. It is important to note that in prolonged chilling conditions Arabidopsis plants almost stop their development and it becomes impossible to count survivors versus dead plants as a parameter of tolerance.

### EXAMPLE 5 Transgenic plants expressing HAHB1 are more tolerant to drought and salt stress than their wild type counterparts

Transcript levels of *HAHB1* increase when sunflower plants are watered with high salt concentrations as described above. In order to test if this gene participates in the plant response to this adverse condition, we irrigated the plants with increasing concentrations of NaCl and observed their behavior.

Membrane stability under these conditions was one of the chosen parameters in order to test plants behaviour. The obtained results are shown in Figure 7a. The conductivity of the wild type genotype membranes notably increased during the treatment compared with that of the transformed one, indicating that membrane stability decreased accordingly. The difference between genotypes is more marked the greater the NaCl concentration.

The damage caused to plants is observable in a photograph two days after irrigating them with 400 mM NaCl (Figure 7b).

In the same way, drought tolerance was also tested in transformed and non-transformed plants (Figure 8). The treatment was severe, as described in the Experimental section. *HARB1* transgenic plants better tolerated the drought conditions and this tolerance correlated with the expression level of the *HAHB1* transgene in the different independent lines.

### EXAMPLE 6 Isolation and characterization of the HAHB1 promoter region

A 1060 bp segment corresponding to the promoter region of *HAHB1* was isolated from a BACs genomic library using as probe the 5'-intron of the cDNA, previously isolated by PCR on genomic DNA with two oligonucleotides based on the coding sequence. Within this promoter region two boxes were identified as cold responsive elements by PLACE.

This segment was cloned so as to be operatively associated with the *GUS* reporter gene (i.e. expression of the GUS gene was under the transcriptional control of the *HAHB1* promoter) and, in a separate construct, with the *RAHB1* cDNA, as described in the Experimental Procedures section above. Both constructs were used to transform Arabidopsis plants and once homozygous lines from each were identified, they were analyzed as shown in Figures 9 and 10, which demonstrate the *GUS* expression pattern directed by the *HAHB1* promoter. As can be seen, GUS is expressed in the vascular system of Arabidopsis seedlings and in the meristematic regions of adult plants (25-day-old and 40-day-old) in normal growth conditions.

### EXAMPLE 7 Phenotypic characteristics of plants bearing the construct promHAHB1:HAHB1

Transgenic plants bearing the construct *promHAHB1:HAHB1* were produced, selected and grown in normal conditions and observed. No significant differences in shape, form or color as compared to their wild type counterparts were detectable (i.e. these transgenic plants are morphologically indistinguishable from wild type counterparts), see Figure (11a). Regarding the growing curve, measured as the stem height during the life cycle, no significant differences were detected, indicating that under the control of its own inducible promoter, the gene does not produce a developmental delay in the early stages of plant development, as was observed with the constitutive 35S:HAHB1 construct bearing plants (Figure 11b).

### EXAMPLE 8 Plants transformed with the construct promHAHB1:HAHB1 are more tolerant to freezing conditions

In order to test if transgenic plants expressing *HAHB1* under the control of its own promoter tolerate freezing conditions better than wild type plants, the plants were subjected after acclimation of 10 days at 4°C to 6 hours at -8°C and then for 2 days at 4°C and then in normal temperature, to allow them to recover over a period of 6 days. The % of survivors of the transgenic genotype varied from 67 to 86% while the average rate of survival of the non-tranformed plants was 40% in this assay. The assay was performed with 16 plants of each genotype. Figure 12 illustrates the results achieved in this type of experiment.

### EXAMPLE 9 XAHB1 confers cold tolerance to transgenic plants via the induction of genes encoding antifreeze proteins

The results presented herein above indicate that *HAHB1* confers tolerance to several abiotic stress factors. To elucidate the molecular and physiological mechanisms involved in this response, since this gene encodes a transcription factor and it could therefore be regulating different transduction signal pathways, we performed a microarray analysis comparing the transcriptome of transgenic and wild type plants using RNAs extracted from both genotypes.

A simple analysis of the data obtained allows visualization of the up-regulation of several genes previously associated with abiotic stress in the *HAHB1*-expressing transgenic plants. Among these genes, the most relevant are those related to cell wall synthesis, to the pathogen response genes (i.e. PR encoding genes) and a β-1,3 glucanase gene. Glucanases, chitinases and thaumatin-like proteins have been described as functioning like antifreeze proteins when plants are subjected to low temperatures. Freezing temperatures lead to the formation of growing ice crystals inside and outside the cell, and the crystals are responsible for mechanical damage (inner crystals) and dehydration (outer crystals) of plants, respectively, and can result in plant death. Antifreeze proteins (AFPs) have been found and characterized in organisms of several kingdoms, including in fish, some insects, land arthropods, bacteria, fungi and plants. They have been purified from the apoplast region of plants and they function by locating themselves on the forming ice crystals. In this way, ice crystals adopt a bi-pyramidal shape that is unable to grow. A second way in which these proteins perform an antifreeze function is by preventing recrystalization (the formation of large crystals from small ones). Additionally, Tomczak et *al.* (2003) demonstrated that antifreeze proteins are able to directly interact with membranes, thereby inhibiting leakage. Accordingly, and without wishing to be bound by mechanistic considerations, the increased membrane stability observed in *HAHB1* transgenic plants could, at least in part, be explained by the action of these proteins.

Accordingly, we isolated the apoplast proteins with the aim of analyzing the potential differences between genotypes. We did not observe very great differences between transgenic and wild type plants grown in normal conditions (Figure 13A) or acclimated during 16 hours (Figure 13B) or 10 days (Figure 12C), in SDS-PAGE but we were able to identify at least five bands secreted to the cellular apoplast in Arabidopsis not that, to the best of our knowledge, have not been previously described (Figure 13 C). These polypeptides were sequenced and three of them match the sequences of PR2, PR5 and an unknown protein respectively. Unfortunately, the two others did not yield clear sequences and they appear to be less concentrated in transgenic plants than in wild type ones. We can speculate that these unidentified bands may be ice-nucleating proteins, the expression of which is repressed in *HAHB1* transgenic plants. Surprisingly, when the plants, both transgenic and wild type, were subjected to freezing conditions at -8 °C, the protein pattern observed was clearly different between genotypes. Transgenic plants bearing the *35S:HAHB1* construct exhibit a higher concentration of apoplast proteins (25 µg protein/g tissue in WT extracts versus 50 µg protein/g tissue in transgenic plants) as well as an additional band with an approximate molecular weight of 23 kDa, indicating a faster response in front of the adverse condition compared with their non transformed counterparts (Figure 13 D). Such faster response could be, at least in part, responsible for the tolerance observed in this genotype.

Chromatographic eluates were spectrophotometrically measured at 230 and 280 nm, since it is known that antifreeze activity in winter rye apoplast can be measured in eluates at 230 nm (Griffith et al., 1992; DeVries et *al.,* 1986). As can be appreciated from Figure 14, the profiles corresponding to the transgenic genotypes differ from those of the wild type profiles.

According to Griffith and Yaish (2004), low temperatures and drought trigger a transduction signal pathway dependent on ethylene, resulting in the expression of proteins with antifreeze activity, while, if the induction is by exposure instead to cold or drought, is produced by SA, ABA or psycrophylic pathogens like snow mold, the induced signal transduction pathway results in the expression of proteins with antipathogen activity. Taking these reports in the literature into account, we analyzed the transcript levels of three genes encoding putative antifreeze proteins from the PR family treated or untreated with SA, ABA, drought, low temperature or ethylene in transgenic plants bearing the construct *promHAHB1:HAHB1* in comparison with non transformed plants.

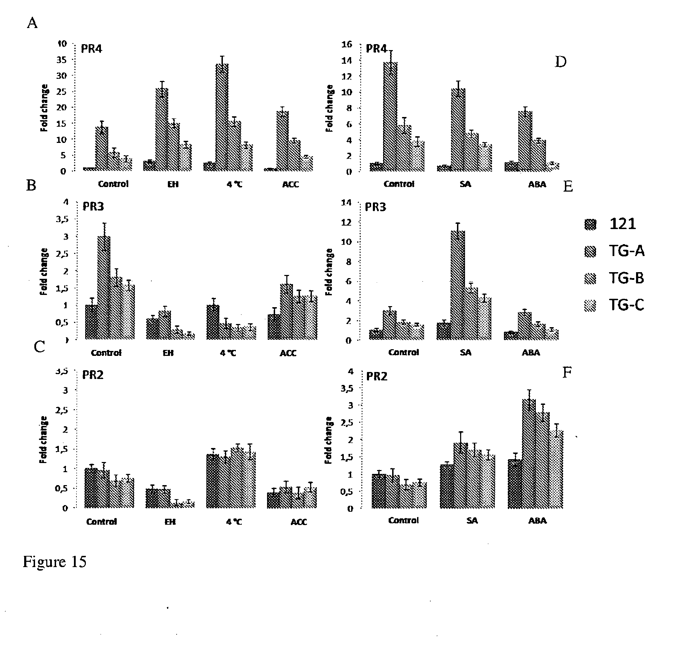

Figure 15 illustrates this experiment in which the transcript levels of the genes encoding Arabidopsis PR4 (At3g04720), chitinase (PR3, At3g12500) and PR2, were quantified under various stress or non-stressed conditions. As it can be appreciated from the figure, PR4 is expressed in transgenic plants in control conditions between 4 to 15 fold more than in wild type plants. After exposure to drought, low temperatures or ACC, these levels increased in all the genotypes, while when the plants were treated with SA or ABA, the basal levels slightly decreased (Figure 15).

PR3, also called chitinase-B, is more highly expressed in the transgenic genotypes than in controls, and it is significantly induced in the presence of SA. When ABA is applied, no changes were observed as compared to control conditions, while the other treatments (drought, ACC and 4°C) produce a decrease in the transcript levels of this gene. On the other hand, PR2 is not induced in transgenic plants compared with wild type plants under normal conditions, but is induced in the presence of ABA or SA or in low temperatures, while it is repressed by treatments with ACC or exposure to drought. Together, these results could indicate that this gene may have a double function, both in cold tolerance and in response to pathogens.

### EXAMPLE 10 Apoplast antifreeze proteins present in transgenic plants expressing HAHB1 are responsible for the observed freezing tolerance

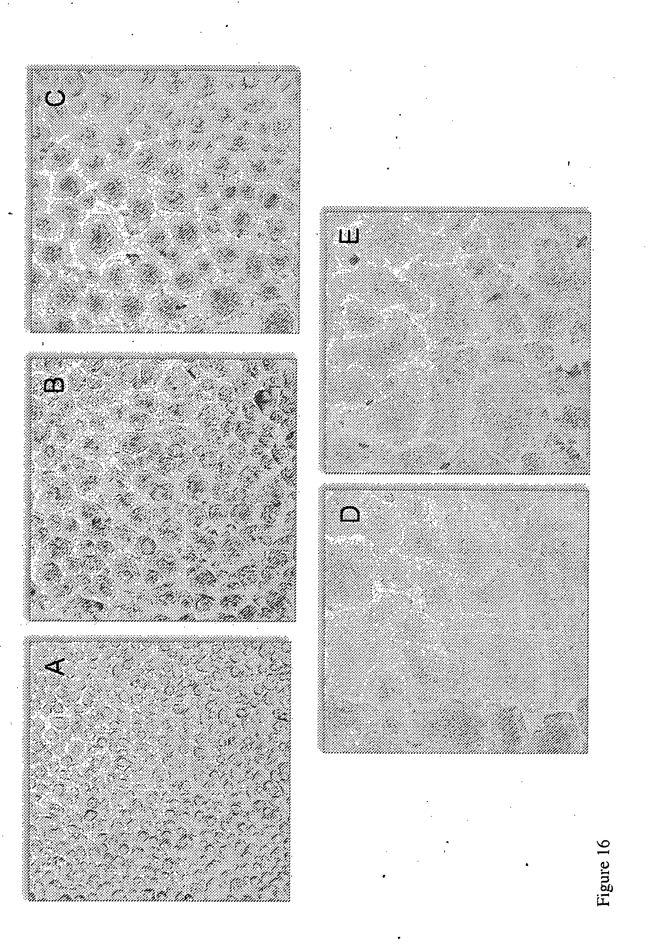

Antifreeze mechanisms are not well studied either in Arabidopsis or in sunflower. In view of the results obtained herein indicating that transcript levels of some putative antifreeze proteins increased in transgenic plants expressing *HAHB1* and that the apoplast protein pattern is different in acclimated plants compared to non-acclimated plants, we investigated the antifreeze activity of these proteins in transgenic and wild type plants. A recrystallization assay with apoplast proteins was performed following the technique described in the Experimental section. As can be appreciated from Figure 16, transgenic acclimated plant apoplast proteins inhibit the arrangement of small ice crystals into larger ones while wild type extracts did not exhibit this inhibitory effect. Among the transgenic lines, line A seems to be the most effective in achieving this function.

On the other hand, some of the genes identified in the microarray as presenting a putative antifreeze activity were chosen in order to obtain transgenic plants and subsequently evaluate their behavior under freezing conditions. Two of these selected genes, encoding PR2 and a β-1,3-glucanase, were analyzed. The genes were isolated by PCR with specific oligonucleotides using Arabidopsis genomic DNA. They were cloned into pBI 121.3 and were used to transform Arabidopsis. Transgenic plants were selected by kanamycin resistance and by the insertion of the genes after being checked by PCR. Five independent F2 lines (non homozygous) were analyzed in normal growth conditions as well as when the plants were subjected to freezing. Figures 17 and 18 show that neither PR2 nor glucanase expressing plants exhibit a differential phenotype in normal growth conditions, while both genes seem to confer freezing tolerance to transgenic plants. However, while glucanase expressing plants exhibit a 50 % survival rate under severe freezing conditions (as compared with only 8 % survival for WT plants; Figure 17), PR2 plants show a lower extent (17 % vs. 8 %, Figure 18) indicating that it is probable that both genes play a role in the freezing tolerance conferred by *HAHB1* but not in the same proportion. In addition to experiments with heterozygous lines, we also carried out experiments with homozygous lines.

In order to corroborate the hypothesis stating that the induced PR like proteins are responsible for the antifreeze activity presented by *HAHB1* expressing plants, we obtained transgenic plants expressing each of these genes independently. The selected three genes, encoding PR2, PR4 and a β-1,3-glucanase were isolated by PCR with specific oligonucleotides using Arabidopsis genomic DNA and cloned into pBI 121.3. The constructs were used to transform Arabidopsis and the correct insertion of the genes was confirmed by PCR. Five independent homozygous lines were analyzed in normal growth conditions as well as when the plants were subjected to freezing temperatures. We show that that neither PR2 nor *GLUC* nor PR4 expressing plants exhibit a differential phenotype in normal growth conditions, but all these genes seem to confer freezing tolerance to transgenic plants in freezing conditions. However, while *GLUC* expressing plants exhibit a 62% survival rate under severe freezing conditions (as compared with only 14% survival for WT plants), PR2 plants show a lower extent (42% vs. 14%) indicating that it is probable that both genes participate in the freezing tolerance conferred by *HAHB1* but not in the same proportion (see figures 32). The transgenic plants also showed increased drought resistance (see figures 31 to 32). Membrane stability, measured as the conductivity of the treated extracts was higher in the transgenic genotypes than in controls, thus indicating that this physiological mechanism is at least in part, responsible for the conferred tolerance.

### EXAMPLE 11 Arabidopsis and sunflower exhibit conserved mechanisms

In view of both the microarray data and the freezing tolerance conferred by *HAHB1,* we wondered if similar events as those described for winter rye involving AFPs occur in these plants. With the aim of answering this question we isolated apoplast proteins from acclimated and non acclimated sunflower plants and analyzed them by SDS-PAGE. Figure 19 shows a time course of the protein pattern as a function of the acclimation days. As can be seen, under normal conditions (time 0 of the acclimation experiment) some bands appear in the sunflower apoplast. This was a surprising result since no proteins were detected under the same conditions in Arabidopsis. During the acclimation process, new protein bands appear whereas some of the original ones diminished or disappeared. We did not confirm to which protein each band corresponds, and this is not trivial in light of the fact that the sunflower genome sequence is not yet available. However, the experiment indicates that a mechanism of induction of AFPs and repression of ice-nucleating proteins is occurring.

On the other hand, using known ESTs and gene sequences, we have been able to identify some sequences that could be homologous to those identified in the Arabidopsis microarray induced by *HAHB1.* According to this identification we designed specific oligonucleotides in order to quantify these genes in transiently transformed sunflower leaves (see Experimental section). Four genes encoding one putative chitinase (Accession N° TC18434), two putative transcription factors homologous to ZAT10 and SAG21 from Arabidopsis (Accession N° of the sunflower genes TC16546 and TC19654 respectively) and one DREB-like (Accession N° TC23839) transcription factor were analyzed. All of these genes exhibited higher transcript levels in sunflower discs overexpressing *HAHB1* than in controls. Since the Arabidopsis homologous genes were also induced in transgenic plants, this result indicates that there is a conserved mechanism of action in both species controlled by the HD-Zip transcription factor, HAHB1.

### EXAMPLE 12 Functions of the protein domains of members of subfamily HD-Zip I

We analysed the structure of the C and N-terminus of HD-Zip I family members using standard techniques and algorithms and found consensus motifs.

For example, we analyzed the sequence logo of N -terminus to find consensus sequences in the HD-Zip and LZ domains. The ids of the *Arabidopsis thaliana* sequences used for the alignment were: AT5G53980.1, AT3G01470.1, AT4G40060.1, AT2G22430.1, AT5G65310.1, AT1G69780.1, AT1G26960.1, AT5G15150.1, AT3G01220.1, AT4G36740.1, AT2G18550.1, AT5G66700.1, AT5G03790.1, AT3G61890.1, AT2G46680.1. Consensus sequences were thus identified.

We also analyzed the Sequence of C-terminus to find consensus sequences using standard techniques and bioinformatics. The ids of the proteins used to produce the sequence logo are: BAA05625.1, BAA05623.1, XP_002276889.1, CAN62385.1, CAO48425.1, EEF42166.1, XP_002311597.1, XP_002315797.1, AAT40488.1, AAT40518.2, AF011556_1, ABL63116.1, AAD14502.1 (all previous by gene id), HAHB-1 (from sunflower) and ATHB13, ATHB23 (from *Arabidopsis thaliana).*

The alignment of the C-terminus from these 15 proteins (most homologous to HAHB1 C- terminus) belonging to different species plus HAHB1 was used to obtain 2 consensus sequences for the 2 best conserved regions, one at the beginning (N end), and the other located at the C end. In the whole carboxy terminus region, there are two conserved domains, one located 5' and the other one in the 3' of the region. The amino acids between these two motifs are not conserved.

We also constructed chimeric proteins fusing the HD-Zip domain of one protein with the carboxy-terminal domain of another. The constructs we produced are shown schematically in figure 22. With these constructs, we transformed Arabidopsis plants, obtained homozygous lines and analyzed the phenotypes, especially regarding characteristics conferred by wt proteins and HAHB1, as compared, for example, to the known characteristics and effects of HAHB4 (see Dezar et al, 2005a and b, Manavella et al 2006, Manavella et al 2008a, b and c. Cabello et al 2007; and WO2004/099365).

The plants transformed with the construct H4CI (comprising the HD-Zip of HAHB4 plus the CI of HAHB1) exhibit the leaf and inflorescence morphological phenotype conferred by HAHB4 (compact), while the plants transformed with H4CICII (comprising the HD-Zip of HAHB4 plus the whole carboxy terminus of HAHB1) exhibit the leaf morphological phenotype conferred by HAHB1 (serrated leaves and equal rosette leaves number).

Regarding ethylene sensitivity in etiolated seedlings, H1WCT (HAHB1 without its whole carboxy terminus) and H1C1 (HAHB1 without the CII) plants behaved like HAHB4 plants (they did not present the triple response, Manavella et *al.,* 2006). Ethylene sensitivity assay in adult plants showed that H4CII (comprising the HD-Zip of HAHB4 plus the CII of HAHB1) and HAHB4 plants behaved similarly (low senescence induction) while HAHB1, WT and H4-H1 (H4CICII) quickly entered in the senescence stage (see figure 33).

HAHB1 transformants, like Arabidopsis ATHB13 transformants, show rounded cotyledons when grown in 4 % sucrose. In this kind of assay H4CI, HAHB4 and WT did not change their morphology while H4-H1 (H4CICII) and HAHB1 exhibited the described phenotype.

H4-H1 (H4CICII) transformed plants exhibit the same morphology as do HAHB1 plants. Both HAHB4 and HAHB1 appear more tolerant to drought than WT. HAHB4 plants are more tolerant than HAHB1 transgenics.

In chilling assays, the H4CI genotype seems to behave like the HAHB1 genotype (tolerant) using HAHB4 plants (non-tolerant) as internal controls.

These results confirm that the carboxy-terminus is responsible for the different functions exerted by HAHB1 as compared with those induced by HAHB4, which belongs to the same subfamily.

### EXAMPLE 14 Overexpression of ATHB13 in Arabidopsis

The cDNA was amplified using RNA isolated from 21-day-old plants by RT-PCR. PCR was carried out with two specifically designed oligonucleotides and cloned in the pBI 121.3 vector previously restricted with BamH1 and XbaI. Once a positive clone was identified, *Agrobacteria* cells were transformed with the construct and these *Agrobacteria* used to transform plants by the floral dip procedure. Transgenic plants were selected by kanamicyn resistance and then the insertion of the gene checked with two oligonucleotides, one matching the 35S (to differentiate the insertion from the endogenous gene) and one matching the cDNA. Once homozygous lines were identified, the transgenic plants were subjected to the same treatments as the HAHB1 plants with the appropriate controls.

### Example 15 Sunflower, soybean and tobacco leaf discs transformed with 35S:HAHB1 over-express genes putatively related to the cold response

In view of both the microarray data and the freezing tolerance conferred by *HAHB1,* we wondered if similar events as those described for winter rye involving AFPs occur in other plant species. With the aim of answering this question, using known ESTs and genes sequences, we have been able to identify some sequences in sunflower, tobacco and soybean, homologous to those identified in the Arabidopsis microarray as induced by *HAHB1.* According to this identification we designed specific oligonucleotides in order to quantify these genes in transiently transformed sunflower, tobacco and soybean leaves both with *35S:HAHB1* and *35S:ATHB13.* Transient transformation of sunflower, soybean and tobacco leaf disks was carried out as described for sunflower tissue (Manavella and Chan, 2009). For each construct, six disks originated from different plants were analyzed and the experiment repeated at least twice. As a control of the infiltration test, *GUS* reporter gene expression in these experiments was measured by histochemical assays.

In sunflower, four genes encoding one putative chitinase (Accession N° TC18434), two putative transcription factors homologous to the Arabidopsis *ZAT10* and *SAG21* (Accession N° of the sunflower genes TC16546 and TC19654 respectively) and one DREB-like (Accession N° TC23839) transcription factor were analyzed.

The expression of three genes homologous to Arabidopis glucanase, PR2 and PR4 was quantified in soybean transiently transformed leaf discs. All of them showed a significant increase in their levels when the transformation was performed with *35S:HAHB1* or *35:ATHB13;* however with the second construct PR2 levels increased to a lesser extent. The experiment performed in tobacco leaf disks showed similar results but the inductions in the transformed disks were lower.

Since the Arabidopsis homologous genes were also induced in transgenic plants, this result indicates that there is a conserved mechanism of action in these species controlled by HD-Zip transcription factors, HAHB1 and its homologues.

The antifreeze mechanism mediated by *HAHB1*/*ATHB13* seems to be conserved between other plant species like tobacco and soybean. The transient transformation of leaves from these plants induced the expression of *HAHB1*-target homologues, indicating that a homologue gene in these species is exerting the same type of regulation, probably conferring a similar tolerant phenotype.

### Example 16 Expression of HAHB1 in Arabidopsis confers increased tolerance to Pseudomonas infection

### Infection with Pseudomonas

An isolated colony of *Pseudomonas syringae* spp. was inoculated in LB medium supplemented with ryfampicine and cultured overnight at 28°C. The culture (2 ml) was centrifuged for 5 min at 4500 rpm and the cellular pellet washed three times with sterile water and finally suspended (1/50) in 10 mM MgCl₂, 15 µl/l Triton X-100. The diluted bacterial suspension was sprayed on 4-week-old Arabidopsis plants from different genotypes as indicated in the Figure legend. The tray was covered with nylon and the plants were photographed 2 days after the infection.

Staining with Evans blue was carried out essentially as described by Kato et al., 2007. Excised leaves were vacuum infiltrated with 0.1 % Evans blue twice for 5 min. After that the leaves were washed three times with distilled water applying vacuum (10 min each time) until they were fully decolorized and then visualized on a microscope and photographed.

### References

Arce A.L., Cabello J.V., Chan R.L. 2008. Patents on Plant Transcription Factors. Recent Patents on Biotechnology, 2: 209-217
Ariel F.D., Manavella P.A., Dezar C.A., Chan R.L. 2007. The true story of the HD-Zip family. Trends Plant Sci. 12, 419-426.
Cabello J.V., Dezar C.A., Manavella P.A., Chan R.L. 2007. The intron of the Arabidopsis thaliana COX5c gene is able to improve the drought tolerance conferred by the sunflower Hahb-4 transcription factor. Planta 226, 1143-1154.
Carabelli M., Sessa G., Baima S., Morelli G., Ruberti I. 1993. The Arabidopsis Athb-2 and -4 genes are strongly induced by far-red-rich light. Plant J. 4: 469-479.
Chan R.L., Gago G.M., Palena C.M., Gonzalez D.H. 1998. Homeoboxes in plant development. Biochim. Biophys. Acta 1442: 1-19.
Chan R.L., Gonzalez D.H. 1994. A cDNA encoding an HD-Zip protein from sunflower. Plant Physiol. 106: 1687-1688.
Chinnusamy V., Zhu J., Zhu J.K. 2007. Cold stress regulation of gene expression in plants. Trends Plant Sci. 12: 444-451.
Clough S.J., Bent A.F. 1998. Floral dip: a simplified method for Agrobacterium-mediated transformation of Arabidopsis thaliana. Plant J. 16, 735-743.
DeVries A.L. 1986. Antifreeze Glycopeptides and Peptides: Interactions with Ice and Water, Methods In Enzymology, vol. 127, 293-303
Dezar C.A., Gago G.M., González D.H., Chan R.L. 2005. Hahb-4, a sunflower homeobox-leucine zipper gene, confers drought tolerance to Arabidopsis thaliana plants. Transgenic Res., 14, 429-440.
Dezar C.A., Fedrigo G.V., Chan R.L. 2005. The promoter of the sunflower HD-Zip protein gene Hahb4 directs tissue-specific expression and is inducible by water stress, high salt concentrations and ABA. Plant Sci. 169: 447-459.
Gago G.M., Almoguera C., Jordano J., González D.H., Chan R.L. 2002. Hahb-4, a homeobox-leucine zipper gene potentially involved in ABA-dependent responses to water stress in sunflower. Plant Cell Environ. 25: 633-640.
Gonzalez D.H., Valle E.M., Gago G.M., Chan R.L. 1997. Interaction between proteins containing homeodomains associated to leucine zippers from sunflower. Biochim. Biophys. Acta 1351: 137-149.
Griffith M., Yaish M.W.F. 2004. Antifreeze proteins in overwintering plants: a tale of two activities Trends Plant Sci. 9: 399-405.
Griffith M., Ala P., Yang D.S., Hon W.C., Moffatt B.A. 1992. Antifreeze Protein Produced Endogenously in Winter Rye Leaves. Plant Physiol. 100: 593-596.
Griffith, 2005. Antifreeze proteins modify the freezing process in planta. Plant Physiol. 138:330-340
Guy C.L., Huber J.L.A., Huber S.C. 1992. Sucrose phosphate synthase and sucrose accumulation at low temperature. Plant Physiol. 100: 502-508
Henriksson E., Olsson A.S., Johannesson H., Johansson H., Hanson J., Engstrom P., Soderman E. 2005. Homeodomain leucine zipper class I genes in Arabidopsis. Expression patterns and phylogenetic relationships. Plant Physiol. 139: 509-518.
Lee Y.H., Chun J.Y. 1998. A new homeodomain-leucine zipper gene from Arabidopsis thaliana induced by water stress and abscisic acid treatment. Plant Mol. Biol. 37: 377-384. Levitt J. 1980. Responses of plants to environmental stresses. Academic Press. Vol I and II.
Manavella P.A., Arce A.L., Dezar C.A., Bitton F., Renou J.P., Crespi M., Chan R.L. 2006. Cross-talk between ethylene and drought signaling pathways is mediated by the sunflower Hahb-4 transcription factor. Plant J. 48: 125-137.
Manavella P.A., Dezar C.A., Bonaventure G., Baldwin I.T., Chan R.L. 2008 HAHB4, a sunflower HD-Zip protein, integrates signals from the jasmonic acid and ethylene pathways during wounding and biotic stress responses, Plant J. 56:376-388.
Mauch F., Staehelin L.A. 1989. Functional implications of the subcellular localization of ethylene-induced chitinase and [beta]-1,3-glucanase in bean leaves. Plant Cell. 1: 447-457.
Nanjo T., Kobayashi M., Yoshiba Y., Kakubari Y., Yamaguchi-Shinozaki K., Shinozaki K. 1999. Antisense suppression of proline degradation improves tolerance to freezing and salinity in Arabidopsis thaliana. FEBS Lett. 461:205-10.
Palena C.M., Gonzalez D.H., Chan R.L. 1999. A monomerdimer equilibrium modulates the interaction of the sunflower homeodomain leucine-zipper protein Hahb-4 with DNA. Biochem. J. 341: 81-87.
Schena M., Davis R.W. 1992. HD-Zip protein members of Arabidopsis homeodomain protein superfamily. Proc. Natl Acad. Sci. USA 89: 3894-3898.
Schena M., Davis R.W. 1994. Structure of homeobox-leucine zipper genes suggests a model for the evolution of gene families. Proc. Natl. Acad. Sci. USA 91: 8393-8397.
Schena M., Lloyd A.M., Davis R.W. 1993. The HAT4 gene of Arabidopsis encodes a developmental regulator. Genes Dev. 7: 367-379.
Schneiter A.A., Miller J.F. 1981. Description of sunflower growth stages. Crop Science 21: 901-903.
Sessa G., Ruberti I., Morelli G. 1997. DNA-binding specificity of the homeodomain-leucine zipper domain. J. Mol. Biol. 5:303-309.
Sessa G., Carbelli M., Ruberti, I. 1994. Identification of distinct families of HD-Zip proteins in Arabidopsis thaliana. In: Molecular-Genetic Analysis of Plant Development and Metabolism. G. Coruzzi and P. Puigdomenech (Eds.). Springer Verlag, Berlin.
Sessa G., Morelli G., Ruberti I. 1993. The Athb-1 and -2 HD-Zip domains homodimerize forming complexes of different DNA binding specificities. EMBO J. 12: 3507-3517.
Söderman E., Hjellstrom M., Fahleson J., Engstrom P. 1999. The HD-Zip gene ATHB6 in Arabidopsis is expressed in developing leaves, roots and carpels and up-regulated by water deficit conditions. Plant Mol. Biol. 40: 1073-1083.
Söderman E., Hjellström M., Fahleson J., Engström P. 1999. The HD-Zip gene ATHB6 in Arabidopsis is expressed in developing leaves, roots and carpels and up-regulated by water deficit conditions. Plant Mol. Biol. 40: 1073-1083.
Söderman E., Mattsson J., Engström P. 1996. The Arabidopsis homeobox gene ATHB-7 is induced by water deficit and by abscisic acid. Plant J. 10: 375-381.
Söderman E., Mattsson J., Svenson M., Borkird C., Engström P. 1994. Expression patterns of novel genes encoding homeodomain leucine-zipper proteins in Arabidopsis thaliana. Plant Mol Biol. 26: 145-154.
Sukumaran N.P., Weiser C.J. 1972. Freezing Injury in Potato Leaves, Plant Physiol. 50: 564-567
Thomashow M.E. 1999. Plant cold acclimation: freezing tolerance genes and regulatory mechanisms. Annu. Rev. Plant Biol., 50: 571-599.
Tomczak M.M., Marshall C.B., Gilbert J.A., Davies P.L. 2003. A facile method for determining ice recrystallization inhibition by antifreeze proteins. Biochem Biophys Res Commun. 311: 1041-1046.
Waterhouse A.M., Procter J.B., Martin D.M.A Clamp M., Barton G. J. 2009. Jalview Version 2 - a multiple sequence alignment editor and analysis workbench. Bioinformatics. 25:1189-1191
Whatley F.R., Tagawa K., Arnon D.I. 1963. Separation of the Light and Dark Reactions in Electron Transfer during Photosynthesis. Proceedings of the National Academy of Sciences USA 49: 266-270.

### Sequence listing information

### SEQUENCE LISTING

### HAHB1 promoter - SEQ ID.1

### HAHB1 mRNA - SEQ. ID.2

Coding sequence: in upper case
5' and 3' untranslated regions: in lower case
Flanking nucleotides from first and second introns: in bold and underlined

### Intron 1 - SEQ ID.3

### Intron 2 - SEQ ID.4

### HAHB1 protein - SEQ ID.5

### HAHB1 cDNA- SEQ ID.6

Coding sequence: in upper case
5' and 3' untranslated regions: in lower case
Flanking nucleotides from first and second introns: in bold and underlined

### HAHB1 gene sequence - SEQ ID.7

Coding sequence: in upper case
Promoter region: lower case
5' and 3' untranslated regions: in underlined lower case First and second introns: in bold lower case

### C-terminus of HAHB1 SEQ ID NO. 8

### HAHB1 C-terminus motif I (CI) SEQ ID NO. 9

LINLNIKETEGSCSNRSENSSEIKLDISRTPATDS

### HAHB1 C-terminus motif II (CII) SEQ ID NO. 10

IKEECFSTMFVGMDDQSGFWPWLEQPQFN

### Consensus sequence for the conserved region adjacent to the leucine zipper (CI, 34 aminoacids) SEQ ID NO. 11

SINLNKETEGSCSNRSENSSDIKLDISRTPAIDS

### Consensus sequence for the second conserved region located at the C-terminal end (CII, 29 aminoacids) SEQ ID NO. 12

VKEESLSNMFCGIDDQSGFWPWLEQQHFN

### N-terminus of HAHB1 SEQ ID NO. 13

### N-terminal homeodomain consensus sequence SEQ ID NO. 14

KKRRLTDEQVKALEKSFELENKLEPERKVQLARELGLQPRQVAVWFQNRRARWKTKQ

| Primer name | Primer sequence | SEQ ID No. | Sequence information | Used for |
|---|---|---|---|---|
| PrH1R | | 15 | 2 | Cloning HAHB1 promoter |
| PR-TOPO | | 16 | | Cloning HAHB1 promoter |
| H1qF | | 17 | 2 | Real Time PCR |
| H1qR | | 18 | 2 | Real Time PCR |
| PR4 CDS-F | | 19 | | Cloning At3g04720 |
| PR4 CDS-R | | 20 | | Cloning At3g04720 |
| PR2 CDS-F | | 21 | | Cloning At3g57260 |
| PR2 CDS-R | | 22 | | Cloning At3g57260 |
| | | | | |
| Glucanase CDS-F | | 23 | | Cloning At4g16260 |
| Glucanase CDS-R | | 24 | | Cloning At4g16260 |
| tc23839-F | | 25 | Sunflower gene similar to DREB 1A and 1B from Arabidopsis | Real Time PCR |
| tc23839-R | | 26 | | Real Time PCR |
| tc19654-F | | 27 | Sunflower gene similar to SAG21 from Arabidopsis | Real Time PCR |
| tc19654-R | | 28 | | Real Time PCR |
| tc16546-F | | 29 | Sunflower gene similar to ZAT10 from Arabidopsis | Real Time PCR |
| tc16546-R | | 30 | | Real Time PCR |
| tc18434-F | | 31 | Sunflower gene similar to Quitinase from Arabidopsis | Real Time PCR |
| tc18434-R | | 32 | | Real Time PCR |
| At3g12500 -F | | 33 | PR3 from Arabidopsis | Real Time PCR |
| At3g12500 -R | | 34 | | Real Time PCR |
| At3g57260 -F | | 35 | β-1,3 Glucanase from Arabidopsis | Real Time PCR |
| At3g57260 -R | | 36 | | Real Time PCR |
| At3g04720 -F | | 37 | PR4 from Arabidopsis | Real Time PCR |
| At3g04720 -R | | 38 | | Real Time PCR |

**Used for micro-array validation**

| Primer name | Primer sequence | SEQ ID No | Sequence ID | Used for |
|---|---|---|---|---|
| At1g62440-F | 5'ACCAACACCACCTTCTCTGC 3' | 39 | LRX1 | Real Time PCR |
| At1g62440-R | 5'TTGATGGTGGAGGAGGAGAC 3' | 40 | LRX1 | Real Time PCR |
| At2g43050-F | 5'GGTTAAATGGAGTGGGTGTCAT 3' | 41 | Cell wall modificator | Real Time PCR |
| At2g43050-R | 5'CAAGTCCTGGGTCGAAACTAAC 3' | 42 | | Real Time PCR |
| At4g16260-F | | 43 | Glucanase | Real Time PCR |
| At4g16260-R | 5'AATGTGATCGGAAATTTTGGTTGT 3' | 44 | Glucanase | Real Time PCR |
| At4g25480-F | | 45 | DREB1b | Real Time PCR |
| At4g25480-R | | 46 | DREB1b | Real Time PCR |
| At4g02380-F | 5'ATGCTATCTTCCGACGTGGTTATG 3' | 47 | SAG21 | Real Time PCR |
| At4g02380-R | 5'CTTCCACTCCCTTCTrCTrCATCA 3' | 48 | SAG21 | Real Time PCR |
| At4g25490-F | 5'CGTTGGCTTTTCAAGATGAGAC 3' | 49 | DREB1a | Real Time PCR |
| At4g25490-R | 5'CGCTCTGTTCCGGTGTATAAATAG 3' | 50 | DREB1a | Real Time PCR |
| At1g27730-F | 5'GTCCACTAGCCACGTTAGCAGTA 3' | 51 | ZAT10 | Real Time PCR |
| At1g27730-R | 5'AGTTGAAGTTTGACCGGAAAGTC 3' | 52 | ZAT10 | Real Time PCR |
| At3g04720-F | | 53 | PR4 | Real Time PCR |
| At3g12500-F | | 54 | PR3 | Real Time PCR |
| At3g12500-R | | 55 | PR3 | Real Time PCR |
| At3g52130-F | | 56 | LBP | Real Time PCR |
| At3g52130-R | 5'GCTAATGACTGAGATTTTGATTCG 3' | 57 | LBP | Real Time PCR |
| At3g07450-F | 5'GACACTTGGTCAACCTTGTTTATG 3' | 58 | LTP | Real Time PCR |
| At3g07450-R | | 59 | LTP | Real Time PCR |
| At5g44420-F | | 60 | PDF1.2 | Real Time PCR |
| At5g44420-R | 5'CTTCAAGGTTAATGCACTGATTCT 3' | 61 | PDF1.2 | Real Time PCR |
| At3g57260-F | | 62 | PR2 | Real Time PCR |
| At3g57260-R | | 63 | PR2 | Real Time PCR |

### SEQ ID NO. 64: ATHB13

This sequence corresponds to the immature mRNA (including introns and untranslated 3' and 5') as defined in the TAIR database (www.Arabidopsis.org).

### SEQ ID NO. 65 glucanase

ATG signaled in bold and capital letters
Introns in bold
Oligonucleotides used for cloning, underlined

### SEQ ID NO. 66 PR2

ATG signaled in bold and capital letters
Introns in bold
Oligonucleotides used for cloning, underlined

### SEQ ID No. 67

### Genomic sequence of PR4 (At3g04720) encoding gene. Underlined are the sequences of the oligonucleotides used to amplified this gene from genomic DNA

Oligonucleotides used to quantify putative targets in Nicotiana and soybean
GMPR2 gi|210143170|dbj|AK285952.1| Glycine max cDNA, clone:
GMFL01-19-B17
GmPR2qF
   5' CCTTCTTCTGGTGGAACTGC 3' SEQ ID No. 68
GmPR2qR
   5' ATAGGAGAAAAGAGCCCCCA 3' SEQ ID No. 69
NTPR2 gi|194719370|gb|EU867448.1| Nicotiana tabacum basic beta-1,3-glucanase gene, complete cds
NtPR2qF
5' CTGGTTTGGGAAACAACATCAA 3' SEQ ID No. 70
NtPR2qR
5' AATCTGGCCTGGATTACCAGAA 3' SEQ ID No. 71
GMPR4 gi|210142121|dbj|AK246040.1| Glycine max cDNA, clone: GMFL01-49-I17
GmPR4qF
5' ACAGGAACAGGAGCAAACACAA 3' SEQ ID No. 72
GmPR4qR
5' CATTCCCACAATCCACAAACTG 3' SEQ ID No. 73
NTPR4 gi|632733|gb|S72452.1| Nicotiana tabacum pathogen- and wound-inducible antifungal protein CBP20 (CBP20) mRNA, complete cds
NtPR4qF
5' TTTGGCATGGAGGAGGAAGTAT 3' SEQ ID No. 74
NtPR4qR
5' TCCACGATTCTCACTGTGGTCT 3' SEQ ID No. 75
GM-glucanase gi|38640794|gb|AY461847.1| Glycine max endo-1,3-beta-glucanase mRNA, complete cds
GmGlucqF
5' GAGAAAGTAGGGGCACCAAATG 3' SEQ ID No. 76
GmGlucqR
5' TTCTGGTTTCCATCAAACATGG 3' SEQ ID No. 77
GM elongation factor (used as internal control)
GmEFlaqF
5' TGAAACAGATGATTTGCTGCTGTA 3' SEQ ID No. 78
GmEFlaqR
5' CAATCATGTTGTCTCCCTCAAAAC 3' SEQ ID No. 79
Nt ACTIN (used as internal control)
NtActqF
5' CTGATGGACAGGTTATCACCATTG 3' SEQ ID No. 80
NtActqR
5' TAATGCGGTAATTTCCTTGCTCAT 3' SEQ ID No. 81

## Claims

1. A method for increasing tolerance to all of drought, high salinity and freezing comprising transforming a plant with a nucleic acid comprising a sequence of SEQ ID No. 6 or 7 or a sequence with at least 90% or at least 95% homology to a sequence comprising SEQ ID No. 6 or 7.

2. A method according to claim 1 wherein the nucleic acid sequence is under the control of the HAHB1 promoter comprising a nucleic acid of SEQ ID No. 1.

3. An isolated chimeric nucleic acid construct comprising a nucleic acid sequence encoding for the N-terminal sequence of another HD Zip protein of subfamily I operatively associated with a nucleic acid sequence encoding for a sequence comprising the C-terminus of HAHB1 as defined in SEQ ID NO. 8.

4. A polypeptide encoded by a gene construct of claim 3.

5. A polypeptide of claim 4 wherein the N-terminus is the N-terminus of HAHB4.

6. A method for conferring tolerance in a plant to all of drought, high salinity and freezing which comprises introducing and expressing in a plant a gene construct as defined in claim 3 or a polypeptide as defined in any of claims 4 or 5.

7. Use of a nucleic acid sequence of SEQ ID No. 6 or 7, or a sequence with at least 90% or at least 95% homology to SEQ ID No. 6 or 7 or use of a vector comprising a nucleic acid sequence of SEQ ID No. 6 or 7 or a sequence with at least 90% or at least 95% homology to SEQ ID No. 6 or 7 in conferring increased tolerance to all of drought, high salinity and freezing.

## Patentansprüche

1. Verfahren zur Erhöhung der Toleranz gegenüber sowohl Trockenheit als auch hoher Salzhaltigkeit als auch Frost, umfassend Transformieren einer Pflanze mit einer Nukleinsäure, die eine Sequenz gemäß SEQ ID Nr.: 6 oder 7 oder eine Sequenz mit mindestens 90% oder mindestens 95% Homologie zu einer Sequenz umfasst, die SEQ ID Nr.: 6 oder 7 umfasst.

2. Verfahren nach Anspruch 1, wobei die Nukleinsäuresequenz unter der Kontrolle des HAHB1-Promoters steht, der eine Nukleinsäure gemäß SEQ ID Nr.: 1 umfasst.

3. Isoliertes chimäres Nukleinsäurekonstrukt, umfassend eine für die N-terminale Sequenz eines anderen HD-Zip-Proteins aus Unterfamilie I codierende Nukleinsäuresequenz, die funktionell verbunden ist mit einer Nukleinsäuresequenz, die für eine Sequenz codiert, die den C-Terminus von HAHB1 gemäß Definition in SEQ ID Nr.: 8 umfasst.

4. Polypeptid, das durch ein Genkonstrukt nach Anspruch 3 codiert ist.

5. Polypeptid nach Anspruch 4, wobei der N-Terminus der N-Terminus von HAHB4 ist.

6. Verfahren zur Übertragung von Toleranz gegenüber sowohl Trockenheit als auch hoher Salzhaltigkeit als auch Frost in eine Pflanze, das umfasst, dass ein Genkonstrukt gemäß Definition in Anspruch 3 oder ein Polypeptid gemäß Definition in einem der Ansprüche 4 oder 5 in eine Pflanze eingebracht und darin exprimiert wird.

7. Verwendung einer Nukleinsäuresequenz gemäß SEQ ID Nr.: 6 oder 7, oder einer Sequenz mit mindestens 90% oder mindestens 95% Homologie zu SEQ ID Nr.: 6 oder 7, oder Verwendung eines Vektors, der eine Nukleinsäuresequenz gemäß SEQ ID Nr.: 6 oder 7 oder eine Sequenz mit mindestens 90% oder mindestens 95% Homologie zu SEQ ID Nr.: 6 oder 7 umfasst, bei Übertragung erhöhter Toleranz gegenüber sowohl Trockenheit als auch hoher Salzhaltigkeit als auch Frost.

## Revendications

1. Procédé pour accroître la tolérance à la fois à la sécheresse, à une haute salinité et au gel, comprenant la transformation d'une plante avec un acide nucléique comprenant une séquence de la SEQ ID No. 6 ou 7 ou une séquence ayant au moins 90% ou au moins 95% d'homologie avec une séquence comprenant la SEQ ID No. 6 ou 7.

2. Procédé selon la revendication 1, dans lequel la séquence d'acide nucléique est sous la régulation du promoteur HAHB1 comprenant un acide nucléique de la SEQ ID No. 1.

3. Construction d'acide nucléique chimérique isolée comprenant une séquence d'acide nucléique codant la séquence N-terminale d'une autre protéine HD Zip de la sous-famille I associée opérationnellement avec une séquence d'acide nucléique codant une séquence comprenant l'extrémité C-terminale de HAHB1 telle que définie dans la SEQ ID No. 8.

4. Polypeptide codé par une construction génique selon la revendication 3.

5. Polypeptide selon la revendication 4, dans lequel l'extrémité N-terminale est l'extrémité N-terminale de HAHB4.

6. Procédé pour conférer à une plante de la tolérance à la fois à la sécheresse, à une haute salinité et au gel, qui comprend l'introduction et l'expression, chez une plante, d'une construction génique telle que définie dans la revendication 3 ou d'un polypeptide tel que défini dans l'une quelconque des revendications 4 ou 5.

7. Utilisation d'une séquence d'acide nucléique de la SEQ ID No. 6 ou 7, ou d'une séquence ayant au moins 90% ou au moins 95% d'homologie avec la SEQ ID No. 6 ou 7, ou utilisation d'un vecteur comprenant une séquence d'acide nucléique de la SEQ ID No. 6 ou 7 ou une séquence ayant au moins 90% ou au moins 95% d'homologie avec la SEQ ID No. 6 ou 7, pour conférer une tolérance accrue à la fois à la sécheresse, à une haute salinité et au gel.
